# EUROPEAN PATENT SPECIFICATION

(11) **EP 2 516 386 B1**
(45) Date of publication and mention of the grant of the patent: **22.04.2015**
(21) Application number: 10840112.6
(22) Date of filing: 22.12.2010
(51) Int. Cl.: C07C 247/08, C07C 233/09, G01N 33/68, G01N 33/50, G01N 33/53, C07C 247/04

(54) **PROTEIN MODIFICATION FROM THE OXIDATION OF CLICKABLE POLYUNSATURATED FATTY ACID ANALOGS**
PROTEINMODIFIZIERUNG AUS DER OXIDIERUNG VON CLICKBAREN POLYUNGESÄTTIGTEN FETTSÄUREANALOGA
MODIFICATION DE PROTÉINE POUR L'OXYDATION D'ANALOGUES D'ACIDES GRAS POLYINSATURÉS APTES À LA CHIMIE CLIC

(30) Priority: 23.12.2009 US 289815 P; 03.02.2010 US 301166 P; 08.03.2010 US 311732 P; 17.03.2010 US 314931 P
(43) Date of publication of application: 31.10.2012
(73) Proprietor: Life Technologies Corporation, Minneapolis, Minnesota 55402 (US)
(72) Inventor: AGNEW, Brian, Eugene Oregon 97405 (US); PICKENS, Chad, Tigard Oregon 97223 (US); SINGH, Upinder, Eugene Oregon 97401 (US)
(74) Representative: Selby, David Sean
(86) International application number: PCT/US2010/061768
(87) International publication number: WO 2011/079188

(56) References cited:
- WO-A1-00/32200
- WANG Y ET AL: "Identification of intracellular modified proteins by the lipid peroxidation aldehyde DODE", ABSTRACTS OF PAPERS AMERICAN CHEMICAL SOCIETY, vol. 238, 23-TOXI, 22 June 2009 (2009-06-22), XP008159966, ISSN: 0065-7727
- GERHARD SPITELLER: "Linoleic acid peroxidation-the dominant lipid peroxidation process in low density lipoprotein-and its relationship to chronic diseases", CHEMISTRY AND PHYSICS OF LIPIDS, vol. 95, no. 2, 1 October 1998 (1998-10-01), pages 105-162, XP055052808, ISSN: 0009-3084, DOI: 10.1016/S0009-3084(98)00091-7
- OSBURN W O ET AL: "Nrf2 regulates an adaptive response protecting against oxidative damage following diquat-mediated formation of superoxide anion", ARCHIVES OF BIOCHEMISTRY AND BIOPHYSICS, vol. 454, no. 1, 1 October 2006 (2006-10-01), pages 7-15, XP024943041, ACADEMIC PRESS, US ISSN: 0003-9861, DOI: 10.1016/J.ABB.2006.08.005 [retrieved on 2006-10-01]
- STEPHEN B MILNE ET AL: "Capture and release of alkyne-derivatized glycerophospholipids using cobalt chemistry", NATURE CHEMICAL BIOLOGY, vol. 6, no. 3, 24 January 2010 (2010-01-24), pages 205-207, XP055052500, ISSN: 1552-4450, DOI: 10.1038/nchembio.311
- BHASKAR S MANDAVILLI ET AL: "Cell-Based Analysis of Oxidative Stress, Lipid Peroxidation and Lipid Peroxidation-Derived Protein Modifications using Fluorescence Microscopy", FREE RADICAL BIOLOGY & MEDICINE, vol. 53, 1 November 2012 (2012-11-01), page S136, XP055052546, ISSN: 0891-5849, DOI: 10.1016/j.freeradbiomed.2012.10.371
- Anonymous: "Click-iT Lipid Peroxidation Detection with Linoleamide Alkylene (LAA). Catalog nos. C10446, C10447", Life Technologies , 30 May 2012 (2012-05-30), pages 1-6, XP002691909, Retrieved from the Internet: URL:http://tools.invitrogen.com/content/sf s/manuals/mp10446.pdf [retrieved on 2013-02-07]
- LIN, SONYUAN ET AL.: 'Novel Analogues of Arachidonylethanolamide (Anandamide) : Affinities for the CB1 and CB2 Cannabinoid Receptors and Metabolic Stability' J. MED. CHEM. vol. 41, 12 December 1998, pages 5353 - 5361, XP002550957
- PATE, DAVID W. ET AL.: 'Effects of Topical Anandamides on Intraocular Pressure in Normotensive Rabbits' LIFE SCIENCE vol. 58, 19 April 1996, pages 1849 - 1860, XP002963823

## Description

### FIELD OF THE INVENTION

This invention relates to the field oxidative stress-induced protein fatty acid acylation.

### BACKGROUND OF THE INVENTION

Protein-carbonyls formed from the oxidation of unsaturated fatty acids are potential markers for oxidative stress and inflammation. Reactive oxygen species (ROS) generated under conditions of oxidative stress can result in membrane lipid peroxidation and decomposition to multiple α,β-unsaturated aldehydes which readily covalently modify proteins. For example, the oxidative decomposition of a phospholipid containing linoleate would yield several electrophilic phospholipid products. 4-hydroxynonenal (HNE) and 4-oxononenal (4ONE) are formed from the ω-end of linoleate ester, but other reactive electrophiles contain the carboxy-end of the linoleate ester. Due to sample complexity and low protein abundance, the cellular identification of lipid-derived protein modifications is challenging.

Previously, polyunsaturated fatty acid probes have involved the placement of a fluorophore or biotin molecule on the carboxyl end of the molecule. As a result, upon lipid peroxidation and breakdown of the fatty acid, only the reactive aldehydes species generated from the carboxylate end can be traced after covalent attachment to a protein. Additionally, the large, bulky, hydrophobic characteristics of fluorophores and biotin can reduce the ability of these molecules to be efficiently taken up into the cell and to incorporate in a physiologically correct orientation.

WANG Y ET AL: "Identification of intracellular modified proteins by the lipid peroxidation aldehyde DODE", ABSTRACTS OF PAPERS AMERICAN CHEMICAL SOCIETY, vol. 238, 23-TOXI, 22 June 2009 (2009-06-22), disclose a method of detecting intracellular modified proteins generated in response to oxidative stress.

### SUMMARY OF THE INVENTION

In one aspect, the present invention provides compounds of the formula: 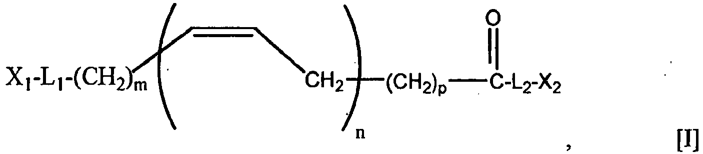 wherein m is 1-4; n is 2-3 or 5-6; p is 1-12; at least one of X₁ or X₂ is selected from the group consisting of alkyne reactive moiety and azide reactive moiety, and the other is selected from the group consisting of H, alkyl, alkenyl, cycloalkyl, cycloalkenyl, alkoxy, aryl, aralkyl, heteroaryl, and heteroaralkyl; and L₁ and L₂ are independently selected from the group consisting of O, NH, alkyl linker group comprising 1-10 carbon atoms, and alkyl linker group comprising 1-10 carbon atoms any of which may be substituted with one or more heteroatoms independently selected from the group consisting of O, N and S;
wherein the compound is not:

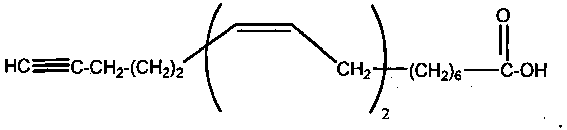

In some embodiments, at least one of X₁ or X₂ is selected from the group consisting of alkyne reactive moiety and azide reactive moiety, and the other is selected from the group consisting of H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atom.

In some embodiments, X₁ is an alkyne reactive moiety; and X₂ is selected from the group consisting of an H, alkyl, alkenyl, cycloalkyl, cycloalkenyl, alkoxy, aryl, aralkyl, heteroaryl, and heteroaralkyl. In some of these, the alkyne reactive moiety is an azido group.

In some embodiments, X₁ is azide reactive moiety; and X₂ is selected from the group consisting of an H, alkyl, alkenyl, cycloalkyl, cycloalkenyl, alkoxy, aryl, aralkyl, heteroaryl, and heteroaralkyl. In some of these, the azide reactive moiety is a terminal alkyne, a cyclooctyne or a phosphine. In some of these, the azide reactive moiety is a terminal alkyne. In some of these, the alkyne is -C=CH.

In some embodiments, X₂ is an alkyne reactive moiety; and X₁ is selected from the group consisting of H, alkyl, alkenyl, cycloalkyl, cycloalkenyl, alkoxy, aryl, aralkyl, heteroaryl, and heteroaralkyl. In some of these, the alkyne reactive moiety is an azido group.

In some embodiments, X₂ is an azide reactive moiety; and X₁ selected from the group consisting of H, alkyl, alkenyl, cycloalkyl, cycloalkenyl, alkoxy, aryl, aralkyl, heteroaryl, and heteroaralkyl. In some of these, the azide reactive moiety is a terminal alkyne, a cyclooctyne or a phosphine. In some of these, the azide reactive moiety is a terminal alkyne. In some of these, the terminal alkyne is -C≡CH.

In some embodiments, X₁ and X₂ are independently selected from the group consisting of an alkyne reactive moiety, and azide reactive moiety. In some of these, the alkyne reactive moiety is an azido group and the azide reactive moiety is a terminal alkyne, a cyclooctyne, or a phosphine. In some of these, the azide reactive moiety is a terminal alkyne. In some of these, the terminal alkyne is -C≡CH.

In some embodiments, the compound is selected from the group consisting of:

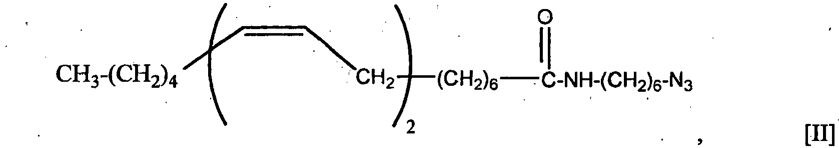

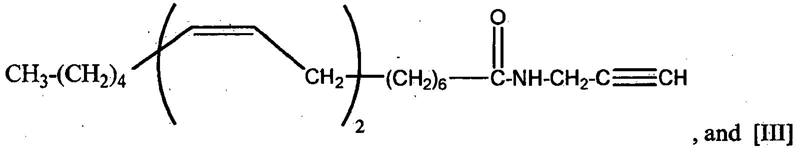

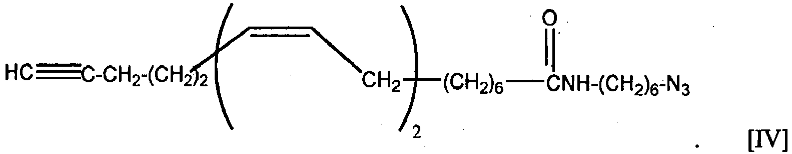

The compounds disclosed above may be used in any of the following methods, including the various embodiments as to X₁, X₂, L₁, L₂, m, n, p, and the excluded compounds.

In another aspect, the present invention provides methods of detecting in a cell a modified protein generated in response to oxidative cellular conditions, comprising the steps of: (a) contacting a cell in an aqueous solution with a compound having the formula: 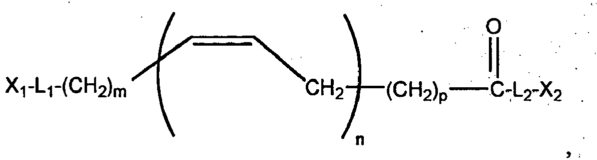 wherein m is 1-4; n is 2-6; p is 1-12;
at least one of X₁ or X₂ is selected from the group consisting of alkyne reactive moiety and azide reactive moiety, and the other is selected from the group consisting of H, alkyl, alkenyl, cycloalkyl, cycloalkenyl, alkoxy, aryl, aralkyl, heteroaryl, and heteroaralkyl; and

L₁ and L₂ are independently selected from the group consisting of O, NH, alkyl linker group comprising 1-10 carbon atoms, and alkyl linker group comprising 1-10 carbon atoms any of which may be substituted with one or more heteroatoms independently selected from the group consisting of O, N and S; (b) contacting the cell in the aqueous solution with a reporter molecule comprising a chemical handle capable of reacting with the alkyne reactive group or azide reactive moiety of the compound; and (c) detecting the presence of the modified protein in the cell.

In some embodiments, at least one of X₁ or X₂ is selected from the group consisting of alkyne reactive moiety and azide reactive moiety, and the other is selected from the group consisting of H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, X₁ is an alkyne reactive moiety; and X₂ is selected from the group consisting of an H, alkyl, alkenyl, cycloalkyl, cycloalkenyl, alkoxy, aryl, aralkyl, heteroaryl, and heteroaralkyl. In some of these, the alkyne reactive moiety is an azido group.

In some embodiments, X₁ is azide reactive moiety; and X₂ is selected from the group consisting of an H, alkyl, alkenyl, cycloalkyl, cycloalkenyl, alkoxy, aryl, aralkyl, heteroaryl, and heteroaralkyl. In some of these, the azide reactive moiety is a terminal alkyne, a cyclooctyne or a phosphine. In some of these, the azide reactive moiety is a terminal alkyne. In some of these, the terminal alkyne is -C=CH.

In some embodiments, X₂ is an alkyne reactive moiety; and X₁ selected from the group consisting of H, alkyl, alkenyl, cycloalkyl, cycloalkenyl, alkoxy, aryl, aralkyl, heteroaryl, and heteroaralkyl. In some of these, the alkyne reactive moiety is an azido group.

In some embodiments, X₂ is an azide reactive moiety; and X₁ selected from the group consisting of H, alkyl, alkenyl, cycloalkyl, cycloalkenyl, alkoxy, aryl, aralkyl, heteroaryl, and heteroaralkyl. In some of these, the azide reactive moiety is a terminal alkyne, a cyclooctyne or a phosphine. In some of these, the azide reactive moiety is a terminal alkyne. In some of these, the terminal alkyne is -C=CH.

In some embodiments, X₁ and X₂ are independently selected from the group consisting of alkyne reactive moiety, and azide reactive moiety. In some of these, the alkyne reactive moiety is an azido group and the azide reactive moiety is a terminal alkyne, a cyclooctyne or a phosphine. In some of these, the azide reactive moiety is a terminal alkyne. In some of these, the terminal alkyne is -C≡CH.

In some embodiments, the compound is selected from the group consisting of the compound having the formula [II], the compound having the formula [III], and the compound having the formula [IV].

In some embodiments, the reporter molecule comprises a chromophore, fluorophore, fluorescent protein, phosphorescent dye, tandem dye, particle, hapten, enzyme, or radioisotope. In some of these, the fluorophore is a xanthene, coumarin, cyanine, pyrene, oxazine, borapolyazaindacene, or carbopyranine. In some of these, the enzyme is horseradish peroxidase, alkaline phosphatase, beta-galactosidase, or beta-lactamase. In some of these, the particle is a semiconductor nanocrystal.

In some embodiments, the chemical handle of the reporter molecule is an azido group, where X₁, X₂, or both X₁ and X₂ of the compound is an azide reactive moiety. In some of these, the azide reactive moiety is a terminal alkyne, a cyclooctyne, or a phosphine. In some of these, the azide reactive moiety is a terminal alkyne. In some of these, the terminal alkyne is -C≡CH.

In some embodiments, the chemical handle of the reporter molecule is an azide reactive moiety, where X₁, X₂, or both X₁ and X₂ group of the compound is azido group. In some of these, the azide reactive moiety is a terminal alkyne, a cyclooctyne or a phosphine. In some of these, the azide reactive moiety is a terminal alkyne. In some of these, the terminal alkyne is -C≡CH.

In some embodiments, the aqueous solution of step (b) further comprises Cu(I) ions; Cu(I) ions and a copper chelator; Cu(II) ions; Cu(II) ions and at least one reducing agent; or, Cu(II) ions, at least one reducing agent and a copper chelator. In some of these, the at least one reducing agent is ascorbate, Tris(2-Carboxyethyl) Phosphine (TCEP), NADH, NADPH, thiosulfate, 2-mercaptoethanol, dithiothreotol, glutathione, cysteine, metallic copper, hydroquinone, vitamin K₁, Fe²⁺, Co²⁺, or an applied electric potential. In some of these, the at least one reducing agent is ascorbate. In some of these, the copper chelator is a copper(I) chelator. In some of these, the copper chelator is N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), 1,10 phenanthroline, or a derivative thereof, trientine, glutathione, histadine, polyhistadine, or tetra-ethylenepolyamine (TEPA). In some of these, the copper chelator is 1,10 phenanthroline, bathophenanthroline disulfonic acid (4,7-diphenyl-1,10-phenanthroline disulfonic acid), bathocuproine disulfonic acid (2,9-dimethyl-4,7-diphenyl-1,10-phenanthroline disulfonate), or THPTA.

In some embodiments, the aqueous solution of step (b) further comprises Cu(I) ions.

In some embodiments, the aqueous solution of step (b) further comprises Cu(II) ions.

In another aspect, the present invention provides methods of detecting in solution a modified protein generated in response to oxidative cellular conditions, comprising the steps of: (a) contacting a cell in an aqueous solution with a compound having the formula: 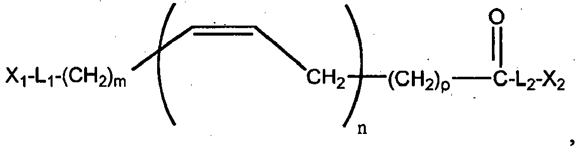 wherein m is 1-4; n is 2-6; p is 1-12;
at least one of X₁ or X₂ is selected from the group consisting of alkyne reactive moiety and azide reactive moiety, and the other is selected from the group consisting of H, alkyl, alkenyl, cycloalkyl, cycloalkenyl, alkoxy, aryl, aralkyl, heteroaryl, and heteroaralkyl; and

L₁ and L₂ are independently selected from the group consisting of O, NH, alkyl linker group comprising 1-10 carbon atoms, and alkyl linker group comprising 1-10 carbon atoms any of which may be substituted with one or more heteroatoms independently selected from the group consisting of O, N and S; (b) preparing an isolate of the cell; (c) contacting the isolate with a reporter molecule, carrier molecule or solid support comprising a chemical handle capable of reacting with the alkyne reactive moiety or azide reactive moiety of the compound; and (d) detecting the presence of the modified protein.

In some embodiments, at least one of X₁ or X₂ is selected from the group consisting of alkyne reactive moiety and azide reactive moiety, and the other is selected from the group consisting of H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, X₁ is an alkyne reactive moiety; and X₂ is selected from the group consisting of an H, alkyl, alkenyl, cycloalkyl, cycloalkenyl, alkoxy, aryl, aralkyl, heteroaryl, and heteroaralkyl. In some of these, the alkyne reactive moiety is an azido group.

In some embodiments, X₁ is azide reactive moiety; and X₂ is selected from the group consisting of an H, alkyl, alkenyl, cycloalkyl, cycloalkenyl, alkoxy, aryl, aralkyl, heteroaryl, and heteroaralkyl. In some of these, the azide reactive moiety is a terminal alkyne, a cyclooctyne, or a phosphine. In some of these, the azide reactive moiety is a terminal alkyne. In some of these, the terminal alkyne is -C≡CH.

In some embodiments, X₂ is an alkyne reactive moiety; and X₁ selected from the group consisting of H, alkyl, alkenyl, cycloalkyl, cycloalkenyl, alkoxy, aryl, aralkyl, heteroaryl, and heteroaralkyl. In some of these, the alkyne reactive moiety is an azido group.

In some embodiments, X₂ is an azide reactive moiety; and X₁ selected from the group consisting of H, alkyl, alkenyl, cycloalkyl, cycloalkenyl, alkoxy, aryl, aralkyl, heteroaryl, and heteroaralkyl. In some embodiments, the azide reactive moiety is a terminal alkyne, a cyclooctyne, or a phosphine. In some of these, the azide reactive moiety is a terminal alkyne. In some of these, the terminal alkyne is -C≡CH.

In some embodiments, X₁ and X₂ are independently selected from the group consisting of alkyne reactive moiety, and azide reactive moiety. In some of these, the alkyne reactive moiety is an azido group and the azide reactive moiety is a terminal alkyne, a cyclooctyne, or a phosphine. In some of these, the azide reactive moiety is a terminal alkyne. In some of these, the terminal alkyne is -C=CH.

In some embodiments, the compound is selected from the group consisting of the compound having the formula [II], the compound having the formula [III], and the compound having the formula [IV].

In some embodiments, the reporter molecule comprises a chromophore, fluorophore, fluorescent protein, phosphorescent dye, tandem dye, particle, hapten, enzyme, or radioisotope. In some of these, the fluorophore is a xanthene, coumarin, cyanine, pyrene, oxazine, borapolyazaindacene, or carbopyranine. In some of these, the enzyme is horseradish peroxidase, alkaline phosphatase, beta-galactosidase, or beta-lactamase. In some of these, the particle is a semiconductor nanocrystal.

In some embodiments, the chemical handle of the reporter molecule is an azido group, where X₁, X₂, or both X₁ and X₂ of the compound is an azide reactive moiety. In some of these, the azide reactive moiety is a terminal alkyne, a cyclootyne, or a phosphine. In some of these, the azide reactive moiety is a terminal alkyne. In some of these, the terminal alkyne is -C=CH.

In some embodiments, the chemical handle of the reporter molecule is an azide reactive moiety, where X₁, X₂, or both X₁ and X₂ group of the compound is azido group. In some of these, azide reactive moiety is a terminal alkyne, a cyclooctyne or a phosphine. In some of these, the azide reactive moiety is a terminal alkyne. In some of these, the terminal alkyne is -C=CH.

In some embodiments, the isolate of step (c) further comprises Cu(I) ions; Cu(I) ions and a copper chelator; Cu(II) ions; Cu(II) ions and at least one reducing agent; or, Cu(II) ions, at least one reducing agent and a copper chelator. In some of these, the at least one reducing agent is ascorbate, Tris(2-Carboxyethyl) Phosphine (TCEP), NADH, NADPH, thiosulfate, 2-mercaptoethanol, dithiothreotol, glutathione, cysteine, metallic copper, hydroquinone, vitamin K₁, Fe²⁺, Co²⁺, or an applied electric potential. In some of these, the at least one reducing agent is ascorbate. In some of these, the copper chelator is a copper(I) chelator. In some of these, the copper chelator is N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), 1,10 phenanthroline, or a derivative thereof, trientine, glutathione, histadine, polyhistadine, or tetra-ethylenepolyamine (TEPA). In some of these, the copper chelator is 1,10 phenanthroline, bathophenanthroline disulfonic acid (4,7-diphenyl-1,10-phenanthroline disulfonic acid), bathocuproine disulfonic acid (2,9-dimethyl-4,7-diphenyl-1,10-phenanthroline disulfonate), or THPTA.

In some embodiments, the isolate of step (c) further comprises Cu(I) ions.

In some embodiments, the isolate of step (c) further comprises Cu(II) ions.

In another aspect, the present invention provides kits comprising the compound of the formula [I]; and further comprising at least one of: (a) an aqueous solution comprising Cu(I) ions; Cu(I) ions and a copper chelator; Cu(II) ions; at least one reducing agent; a copper chelator; at least one reducing agent and a copper chelator; Cu(II) ions and at least one reducing agent; Cu(II) ions and a copper chelator; or, Cu(II) ions, at least one reducing agent and a copper chelator; or (b) a reporter molecule, carrier molecule, or solid support comprising a chemical handle capable of reacting with the alkyne reactive moiety or azide reactive moiety of the compound.

In some embodiments, at least one of X₁ or X₂ is selected from the group consisting of alkyne reactive moiety and azide reactive moiety, and the other is selected from the group consisting of H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, X₁ is an alkyne reactive moiety; and X₂ is selected from the group consisting of an H, alkyl, alkenyl, cycloalkyl, cycloalkenyl, alkoxy, aryl, aralkyl, heteroaryl, and heteroaralkyl. In some of these, the alkyne reactive moiety is an azido group.

In some embodiments, X₁ is azide reactive moiety; and X₂ is selected from the group consisting of an H, alkyl, alkenyl, cycloalkyl, cycloalkenyl, alkoxy, aryl, aralkyl, heteroaryl, and heteroaralkyl. In some of these, the azide reactive moiety is a terminal alkyne, a cyclooctyne or a phosphine. In some of these, the azide reactive moiety is a terminal alkyne. In some of these, the terminal alkyne is -C≡CH.

In some embodiments, X₂ is an alkyne reactive moiety; and X₁ selected from the group consisting of H, alkyl, alkenyl, cycloalkyl, cycloalkenyl, alkoxy, aryl, aralkyl, heteroaryl, and heteroaralkyl. In some of these, the alkyne reactive moiety is an azido group.

In some embodiments, X₂ is an azido reactive moiety; and X₁ selected from the group consisting of H, alkyl, alkenyl, cycloalkyl, cycloalkenyl, alkoxy, aryl, aralkyl, heteroaryl, and heteroaralkyl. In some of these, the azide reactive moiety is a terminal alkyne, a cyclooctyne, or a phosphine. In some of these, the azido reactive moiety is a terminal alkyne. In some of these, the terminal alkyne is -C=CH.

In some embodiments, X₁ and X₂ are independently selected from the group consisting of alkyne reactive moiety, and azide reactive moiety. In some of these, the alkyne reactive moiety is an azido group and the azide reactive moiety is a terminal alkyne, a cyclooctyne or a phosphine. In some of these, the azide reactive moiety is a terminal alkyne. In some of these, the terminal alkyne is -C=CH.

In some embodiments, the compound is selected from the group consisting of the compound having the formula [II], the compound having the formula [III], and the compound having the formula [IV].

In another aspect, the present invention provides methods of detecting in a cell modified proteins generated in response to oxidative cellular conditions, comprising the steps of: (a) contacting a cell in an aqueous solution with a first and second compound having the formula [I], wherein the first compound has X₁ that is an alkyne reactive moiety and X₂ that is not an alkyne reactive moiety or an azide reactive moiety, and the second compound has an X₂ that is an azide reactive moiety and X₁ that is not an azide reactive moiety or an alkyne reactive moiety; (b) contacting the cell in the aqueous solution with a first reporter molecule comprising a chemical handle capable of reacting with the alkyne reactive moiety of the first compound; (c) contacting the cell in the aqueous solution with a second reporter molecule comprising a chemical handle capable of reacting with the azide reactive moiety of the second compound; and (d) detecting the presence of the modified proteins in the cell.

In some embodiments, X₁ and X₂, when they are not an alkyne reactive moiety or an azide reactive moiety, are independently selected from the group consisting of H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, the alkyne reactive moiety is an azido group and the azide reactive moiety is a terminal alkyne, a cyclooctyne or a phosphine. In some of these, the azide reactive moiety is a terminal alkyne. In some of these, the terminal alkyne is -C≡CH.

In some embodiments, the compound is selected from the group consisting of the compound having the formula [II], the compound having the formula [III], and the compound having the formula [IV].

In some embodiments, the aqueous solution of steps (b) and (c) further comprise Cu(I) ions; Cu(I) ions and a copper chelator; Cu(II) ions; Cu(II) ions and at least one reducing agent; or, Cu(II) ions, at least one reducing agent and a copper chelator. In some of these, the at least one reducing agent is ascorbate, Tris(2-Carboxyethyl) Phosphine (TCEP), NADH, NADPH, thiosulfate, 2-mercaptoethanol, dithiothreotol, glutathione, cysteine, metallic copper, hydroquinone, vitamin K₁, Fe²⁺, Co²⁺, or an applied electric potential. In some of these, the at least one reducing agent is ascorbate. In some of these, the copper chelator is a copper(I) chelator. In some of these, the copper chelator is N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), 1,10 phenanthroline, or a derivative thereof, trientine, glutathione, histadine, polyhistadine, or tetra-ethylenepolyamine (TEPA). In some of these, the copper chelator is 1,10 phenanthroline, bathophenanthroline disulfonic acid (4,7-diphenyl-1,10-phenanthroline disulfonic acid), bathocuproine disulfonic acid (2,9-dimethyl-4,7-diphenyl-1,10-phenanthroline disulfonate), or THPTA.

In some embodiments, the aqueous solution of steps (b) and (c) further comprise Cu(I) ions.

In some embodiments, the aqueous solution of steps (b) and (c) further comprise Cu(II) ions.

In another aspect, the present invention provides methods of detecting in a cell modified proteins generated in response to oxidative cellular conditions, comprising the steps of: (a) contacting a cell in an aqueous solution with a first and second compound having the formula [I], wherein the first compound has X₁ that is an azide reactive moiety and X₂ that is not an azide reactive reactive moiety or an alkyne reactive moiety, and the second compound has an X₂ that is an alkyne reactive moiety and X₁ that is not an alkyne reactive moiety or an azide reactive moiety; (b) contacting the cell in the aqueous solution with a first reporter molecule comprising a chemical handle capable of reacting with the azide reactive moiety of the first compound; (c) contacting the cell in the aqueous solution with a second reporter molecule comprising a chemical handle capable of reacting with the alkyne reactive moiety of the second compound; and (d) detecting the presence of the modified proteins in the cell.

In some embodiments, X₁ and X₂, when they are not an alkyne reactive moiety or an azide reactive moiety, are independently selected from the group consisting of hydrogen, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, the alkyne reactive moiety is an azido group and the azide reactive moiety is a terminal alkyne, a cyclooctyne or a phosphine. In some of these, the azide reactive moiety is a terminal alkyne. In some of these, the terminal alkyne is -C≡CH.

In some embodiments, the compound is selected from the group consisting of the compound having the formula [II], the compound having the formula [III], and the compound having the formula [IV].

In some embodiments, the aqueous solution of steps (b) and (c) further comprise Cu(I) ions; Cu(I) ions and a copper chelator; Cu(II) ions; Cu(II) ions and at least one reducing agent; or, Cu(II) ions, at least one reducing agent and a copper chelator. In some of these, the at least one reducing agent is ascorbate, Tris(2-Carboxyethyl) Phosphine (TCEP), NADH, NADPH, thiosulfate, 2-mercaptoethanol, dithiothreotol, glutathione, cysteine, metallic copper, hydroquinone, vitamin K₁, Fe²⁺, Co²⁺, or an applied electric potential. In some of these, the at least one reducing agent is ascorbate. In some of these, the copper chelator is a copper(I) chelator. In some of these, the copper chelator is N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), 1,10 phenanthroline, or a derivative thereof, trientine, glutathione, histadine, polyhistadine, or tetra-ethylenepolyamine (TEPA). In some of these, the copper chelator is 1,10 phenanthroline, bathophenanthroline disulfonic acid (4,7-diphenyl-1,10-phenanthroline disulfonic acid), bathocuproine disulfonic acid (2,9-dimethyl-4,7-diphenyl-1,10-phenanthroline disulfonate), or THPTA.

In some embodiments, the aqueous solution of steps (b) and (c) further comprise Cu(I) ions.

In some embodiments, the aqueous solution of steps (c) and (d) further comprise Cu(II) ions.

In another aspect, the present invention provides methods of detecting in solution modified proteins generated in response to oxidative cellular conditions, comprising the steps of: (a) contacting a cell in an aqueous solution with a first compound and second compound having the formula [I], wherein the first compound has X₁ that is an alkyne reactive moiety and X₂ that is not an alkyne reactive moiety or an azide reactive moiety, and the second compound has an X₂ that is an azide reactive moiety and X₁ that is not an azide reactive moiety or an alkyne reactive moiety; (b) preparing an isolate of the cell; (c) contacting the isolate with a first reporter molecule comprising a chemical handle capable of reacting with the alkyne reactive moiety of the first compound; (d) contacting the isolate with a second reporter molecule comprising a chemical handle capable of reacting with the azide reactive moiety of the second compound; and (e) detecting the presence of the modified proteins.

In some embodiments, X₁ and X₂, when they are not an alkyne reactive moiety or an azide reactive moiety, are independently selected from the group consisting of hydrogen, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some of embodiments, the alkyne reactive moiety is an azido group and the azide reactive moiety is a terminal alkyne, a cyclooctyne or a phosphine. In some of these the azide reactive moiety is a terminal alkyne. In some of these, the terminal alkyne is -C≡CH.

In some embodiments, the compound is selected from the group consisting of the compound having the formula [II], the compound having the formula [III], and the compound having the formula [IV].

In some embodiments, the isolate of steps (c) and (d) further comprise Cu(I) ions; Cu(I) ions and a copper chelator; Cu(II) ions; Cu(II) ions and at least one reducing agent; or, Cu(II) ions, at least one reducing agent and a copper chelator. In some of these, the at least one reducing agent is ascorbate, Tris(2-Carboxyethyl) Phosphine (TCEP), NADH, NADPH, thiosulfate, 2-mercaptoethanol, dithiothreotol, glutathione, cysteine, metallic copper, hydroquinone, vitamin K₁, Fe²⁺, Co²⁺, or an applied electric potential. In some of these, the at least one reducing agent is ascorbate. In some of these, the copper chelator is a copper(I) chelator. In some of these, the copper chelator is N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), 1,10 phenanthroline, or a derivative thereof, trientine, glutathione, histadine, polyhistadine, or tetra-ethylenepolyamine (TEPA). In some of these, the copper chelator is 1,10 phenanthroline, bathophenanthroline disulfonic acid (4,7-diphenyl-1,10-phenanthroline disulfonic acid), bathocuproine disulfonic acid (2,9-dimethyl-4,7-diphenyl-1,10-phenanthroline disulfonate), or THPTA.

In some embodiments, the isolate of steps (c) and (d) further comprise Cu(I) ions.

In some embodiments, the isolate of steps (c) and (d) further comprise Cu(II) ions.

In another aspect, the present invention provides methods of detecting in solution modified proetins generated in response to oxidative cellular conditions, comprising the steps of: (a) contacting a cell in an aqueous solution with a first compound and second compound having the formula [I], wherein the first compound has X₁ that is an azide reactive moiety and X₂ that is not an azide reactive moiety or an alkyne reactive moiety, and the second compound has an X₂ that is an alkyne reactive moiety and X₁ that is not an alkyne reactive moiety or an azide reactive moiety; (b) preparing an isolate of the cell; (c) contacting the isolate with a first reporter molecule comprising a chemical handle capable of reacting with the azide reactive moiety of the first compound; (d) contacting the isolate with a second reporter molecule comprising a chemical handle capable of reacting with the alkyne reactive moiety of the second compound; and (e) detecting the presence of the modified proteins.

In some embodiments, X₁ and X₂, when they are not an alkyne reactive moiety or an azide reactive moiety, are independently selected from the group consisting of hydrogen, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, the alkyne reactive moiety is an azido group and the azide reactive moiety is a terminal alkyne, a cyclooctyne or a phosphine. In some of these, the azide reactive moiety is a terminal alkyne. In some of these, the terminal alkyne is -C≡CH.

In some embodiments, the compound is selected from the group consisting of the compound having the formula [II], the compound having the formula [III], and the compound having the formula [IV].

In some embodiments, the isolate of steps (c) and (d) further comprise Cu(I) ions; Cu(I) ions and a copper chelator; Cu(II) ions; Cu(II) ions and at least one reducing agent; or, Cu(II) ions, at least one reducing agent and a copper chelator. In some of these, the at least one reducing agent is ascorbate, Tris(2-Carboxyethyl) Phosphine (TCEP), NADH, NADPH, thiosulfate, 2-mercaptoethanol, dithiothreotol, glutathione, cysteine, metallic copper, hydroquinone, vitamin Ki, Fe²⁺, Co²⁺, or an applied electric potential. In some of these, the at least one reducing agent is ascorbate. In some of these, the copper chelator is a copper(I) chelator. In some of these, the copper chelator is N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), 1,10 phenanthroline, or a derivative thereof, trientine, glutathione, histadine, polyhistadine, or tetra-ethylenepolyamine (TEPA). In some of these, the copper chelator is 1,10 phenanthroline, bathophenanthroline disulfonic acid (4,7-diphenyl-1,10-phenanthroline disulfonic acid), bathocuproine disulfonic acid (2,9-dimethyl-4,7-diphenyl-1,10-phenanthroline disulfonate), or THPTA.

In some embodiments, the isolate of steps (c) and (d) further comprise Cu(I) ions.

In some embodiments, the isolate of steps (c) and (d) further comprise Cu(II) ions.

In another aspect, the present invention provides methods of conjugating a modified protein generated in response to oxidative.cellular conditions to a solid support comprising the steps of: (a) contacting a cell in an aqueous solution with a compound having the formula [I]; (b) preparing an isolate of the cell; (c) contacting the isolate with a solid support comprising at least one alkyne reactive moiety to form a contacted modified protein; and (d) incubating the contacted modified protein for a sufficient amount of time to form a modified protein-solid support conjugate.

In some embodiments, when X₁ is an azide reactive moiety, X₂ is selected from the group consisting of azide reactive moiety, H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, when X₂ is an azide reactive moiety, X₁ is selected from the group consisting of azide reactive moiety H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, the alkyne reactive moiety is an azido group and the azide reactive moiety is a terminal alkyne, a cyclooctyne or a phosphine. In some of these, the azide reactive moiety is a terminal alkyne. In some of these, the terminal alkyne is -C≡CH.

In some embodiments, the compound is selected from the group consisting of the compound having the formula [II], the compound having the formula [III], and the compound having the formula [IV].

In some embodiments the isolate of step (c) further comprises Cu(I) ions; Cu(I) ions and a copper chelator; Cu(II) ions; Cu(II) ions and at least one reducing agent; or, Cu(II) ions, at least one reducing agent and a copper chelator. In some of these, the at least one reducing agent is ascorbate, Tris(2-Carboxyethyl) Phosphine (TCEP), NADH, NADPH, thiosulfate, 2-mercaptoethanol, dithiothreotol, glutathione, cysteine, metallic copper, hydroquinone, vitamin K₁, Fe²⁺, Co²⁺, or an applied electric potential. In some of these, the at least one reducing agent is ascorbate. In some of these, the copper chelator is a copper(I) chelator. In some of these, the copper chelator is N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), 1,10 phenanthroline, or a derivative thereof, trientine, glutathione, histadine, polyhistadine, or tetra-ethylenepolyamine (TEPA). In some of these, the copper chelator is 1,10 phenanthroline, bathophenanthroline disulfonic acid (4,7-diphenyl-1,10-phenanthroline disulfonic acid), bathocuproine disulfonic acid (2,9-dimethyl-4,7-diphenyl-1,10-phenanthroline disulfonate), or THPTA.

In some embodiments, the isolate of step (c) further comprises Cu(I) ions.

In some embodiments, the isolate of step (c) further comprises Cu(II) ions.

In another aspect, the present invention provides methods of conjugating a modified protein generated in response to oxidative cellular conditions to a solid support comprising the steps of: (a) contacting a cell in an aqueous solution with a compound having the formula [I]; (b) preparing an isolate of the cell; (c) contacting the isolate with a solid support comprising at least one azide reactive moiety to form a contacted modified protein; and (d) incubating the contacted modified biomolecule for a sufficient amount of time to form a modified protein-solid support conjugate.

In some embodiments, when X₁ is an alkyne reactive moiety, X₂ is selected from the group consisting of alkyne reactive moiety, H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, when X₂ is an alkyne reactive moiety, X₁ is selected from the group consisting of alkyne reactive moiety, H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atoms.

In some embodiments, the alkyne reactive moiety is an azido group and the azide reactive moiety is a terminal alkyne, a cyclooctyne or a phosphine. In some of these, the azide reactive moiety is a terminal alkyne. In some of these, the terminal alkyne is -C≡CH.

In some embodiments, the compound is selected from the group consisting of the compound haying the formula [II], the compound having the formula [III], and the compound having the formula [IV].

In some embodiments, the isolate of step (c) further comprises Cu(I) ions; Cu(I) ions and a copper chelator; Cu(II) ions; Cu(II) ions and at least one reducing agent; or, Cu(II) ions, at least one reducing agent and a copper chelator. In some of these, the at least one reducing agent is ascorbate, Tris(2-Carboxyethyl) Phosphine (TCEP), NADH, NADPH, thiosulfate, 2-mercaptoethanol, dithiothreotol, glutathione, cysteine, metallic copper, hydroquinone, vitamin K₁, Fe²⁺, Co²⁺, or an applied electric potential. In some of these, the at least one reducing agent is ascorbate. In some embodiments, the copper chelator is a copper(I) chelator. In some embodiments, the copper chelator is N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), 1,10 phenanthroline, or a derivative thereof, trientine, glutathione, histadine, polyhistadine, or tetra-ethylenepolyamine (TEPA). In some of these, the copper chelator is 1,10 phenanthroline, bathophenanthroline disulfonic acid (4,7-diphenyl-1,10-phenanthroline disulfonic acid), bathocuproine disulfonic acid (2,9-dimethyl-4,7-diphenyl-1,10-phenanthroline disulfonate), or THPTA.

In some embodiments, the isolate of step (c) further comprises Cu(I) ions.

In some embodiments, the isolate of step (c) further comprises Cu(II) ions.

### DESCRIPTION OF THE DRAWINGS

FIG. 1 shows the reaction scheme for the synthesis of a linoleic acid analog **3** containing an azido group and a linoleic acid analog **4** containing an terminal acetylene group.
FIG. 2 shows the reaction scheme for the synthesis of a linoleic acid analogs **11, 12,** and **14.**
FIG. 3 shows the image of BPAE cells which were treated with the linoleic acid azide analog **3,** then were treated with and without menadione to induce oxidative stress. The left image in each panel shows nuclear staining of the cells, the middle image shows the resulting fluorescence after the labeling of the cells with Alexa Fluor® 594, and the right image shows the combined image of the first and second image.
FIG. 4 shows the image of BPAE cells which were not treated with the linoleic acid azide analog **3,** but were treated with and without menadione to induce oxidative stress. The left image in each panel shows nuclear staining of the cells, the middle image shows the resulting fluorescence after the labeling of the cells with Alexa Fluor® 594, and the right image shows the combined image of the first and second image.
FIG. 5 shows the image of BPAE cells which were treated with the linoleic acid alkyne analog **4,** then were treated with and without menadione to induce oxidative stress. The left image in each panel shows nuclear staining of the cells, the middle image shows the resulting fluorescence after the labeling of the cells with Alexa Fluor® 594, and the right image shows the combined image of the first and second image.
FIG. 6 shows the image of BPAE cells which were not treated with the linoleic acid alkyne analog **4,** but were treated with and without menadione to induce oxidative stress. The left image in each panel shows nuclear staining of the cells, the middle image shows the resulting fluorescence after the labeling of the cells with Alexa Fluor® 594, and the right image shows the combined image of the first and second image.
FIG. 7 shows the image of RAW 264.7 cells after treatment with linoleic acid alkyne analog **4** using hemin to induce oxidative stress.
FIG. 8A shows the dramatic increase in signal observed of the hemin treated linoleic acid alkyne analog **4** containing sample, compared to the linoleic acid alkyne analog **4** only and the DMSO sample.
FIG. 8B quantifies the signal observed in FIG. 8A.
FIG. 9 shows in a Venn diagram, that the majority of the proteins identified by mass spectrometry were from cells treated with linoleic acid azide analog 3 while under hemin induced oxidative stress.
FIG. 10 shows the image of BPAE cells after treatment with linoleic acid alkyne analog **4** treated with hemin to induce oxidative stress.
FIG. 11 shows the image of U-2 OS cells after treatment with linoleic acid alkyne analog **4** treated with hemin to induce oxidative stress.

### DETAILED DESCRIPTION OF THE INVENTION

The present invention has utility in the study of oxidative stress and inflammation. The present invention provides compositions, methods, and kits for the labeling, detecting, isolating and/or analysis of proteins modified by attachment of the products of lipid peroxidation in a cell of the modified polyunsaturated fatty acid analogs of the present invention.

### Definitions and Abbreviations

Before describing the present invention in detail, it is to be understood that this invention is not limited to specific compositions or process steps, as such may vary. It must be noted that, as used in this specification and the appended claims, the singular form "a", "an" and "the" include plural referents unless the context clearly dictates otherwise. Thus, for example, reference to "a ligand" includes a plurality of ligands and reference to "an antibody" includes a plurality of antibodies and the like.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the racemates, diastereomers, geometric isomers and individual isomers are encompassed within the scope of the present invention.

The compounds described herein may be prepared as a single isomer (e.g., enantiomer, *cis-trans,* positional, diastereomer) or as a mixture of isomers. In a preferred embodiment, the compounds are prepared as substantially a single isomer. Methods of preparing substantially isomerically pure compounds are known in the art. For example, enantiomerically enriched mixtures and pure enantiomeric compounds can be prepared by using synthetic intermediates that are enantiomerically pure in combination with reactions that either leave the stereochemistry at a chiral center unchanged or result in its complete inversion. Alternatively, the final product or intermediates along the synthetic route can be resolved into a single stereoisomer. Techniques for inverting or leaving unchanged a particular stereocenter, and those for resolving mixtures of stereoisomers are well known in the art and it is well within the ability of one of skill in the art to choose, and appropriate method for a particular situation. *See,* generally, Furniss et al. (eds.),VOGEL'S ENCYCLOPEDIA OF PRACTICAL ORGANIC CHEMISTRY 5TH ED., Longman Scientific and Technical Ltd., Essex, 1991, pp. 809-816; and Heller, Acc. Chem. Res. 23: 128 (1990).

The compounds disclosed herein may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are intended to be encompassed within the scope of the present invention.

Where a disclosed compound includes a conjugated ring system, resonance stabilization may permit a formal electronic charge to be distributed over the entire molecule. While a particular charge may be depicted as localized on a particular ring system, or a particular heteroatom, it is commonly understood that a comparable resonance structure can be drawn in which the charge may be formally localized on an alternative portion of the compound.

Selected compounds having a formal electronic charge may be shown without an appropriate biologically compatible counterion. Such a counterion serves to balance the positive or negative charge present on the compound. As used herein, a substance that is biologically compatible is not toxic as used, and does not have a substantially deleterious effect on biomolecules. Examples of negatively charged counterions include, among others, chloride, bromide, iodide, sulfate, alkanesulfonate, arylsulfonate, phosphate, perchlorate, tetrafluoroborate, tetraarylboride, nitrate and anions of aromatic or aliphatic carboxylic acids. Preferred counterions may include chloride, iodide, perchlorate and various sulfonates. Examples of positively charged counterions include, among others, alkali metal, or alkaline earth metal ions, ammonium, or alkylammonium ions.

Where substituent groups are specified by their conventional chemical formulae, written from left to right, they equally encompass the chemically identical substituents, which would result from writing the structure from right to left, e.g., -CH₂O- is intended to also recite -OCH₂-.

The term "acyl" or "alkanoyl" by itself or in combination with another term, means, unless otherwise stated, a stable straight or branched chain, or cyclic hydrocarbon radical, or combinations thereof, consisting of the stated number of carbon atoms and an acyl radical on at least one terminus of the alkane radical. The "acyl radical" is the group derived from a carboxylic acid by removing the -OH moiety therefrom.

The term "alkyl," by itself or as part of another substituent means, unless otherwise stated, a straight or branched chain, or cyclic hydrocarbon radical, or combination thereof, which may be fully saturated, mono- or polyunsaturated and can include divalent ("alkylene") and multivalent radicals, having the number of carbon atoms designated (*i.e.* C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl," unless otherwise noted, is also meant to include those derivatives of alkyl defined in more detail below, such as "heteroalkyl." Alkyl groups that are limited to hydrocarbon groups are termed "homoalkyl".

Exemplary alkyl groups of use in the present invention contain between about one and about twenty five carbon atoms (e.g. methyl, ethyl and the like). Straight, branched or cyclic hydrocarbon chains having eight or fewer carbon atoms will also be referred to herein as "lower alkyl". In addition, the term "alkyl" as used herein further includes one or more substitutions at one or more carbon atoms of the hydrocarbon chain fragment.

The term "amino" or "amine group" refers to the group NR'R" (or NRR'R") where R, R' and R" are independently selected from the group consisting of hydrogen, alkyl, substituted alkyl, aryl, substituted aryl, arylalkyl, substituted arylalkyl, heteroaryl, and substituted heteroaryl. A substituted amine being an amine group wherein R' or R" is other than hydrogen. In a primary amino group, both R' and R" are hydrogen, whereas in a secondary amino group, either, but not both, R' or R" is hydrogen. In addition, the terms "amine" and "amino" can include protonated and quaternized versions of nitrogen, comprising the group NRR'R" and its biologically compatible anionic counterions.

The term "aryl" as used herein refers to cyclic aromatic carbon chain having twenty or fewer carbon atoms, e.g., phenyl, naphthyl, biphenyl, and anthracenyl. One or more carbon atoms of the aryl group may also be substituted with, e.g., alkyl; aryl; heteroaryl; a halogen; nitro; cyano; hydroxyl, alkoxyl or aryloxyl; thio or mercapto, alkyl-, or arylthio; amino, alkylamino, arylamino, dialkyl-, diaryl-, or arylalkylamino; aminocarbonyl, alkylaminocarbonyl, arylaminocarbonyl, dialkylaminocarbonyl, diarylaminocarbonyl, or arylalkylaminocarbonyl; carboxyl, or alkyl- or aryloxycarbonyl; aldehyde; aryl- or alkylcarbonyl; iminyl, or aryl- or alkyliminyl; sulfo; alkyl- or alkylcarbonyl; sulfo; alkyl- or arylsufonyl; hydroximinyl, or aryl- or alkoximinyl. In addition, two or more alkyl or heteroalkyl substituents of an aryl group may be combined to form fused aryl-alkyl or aryl-heteroalkyl ring systems (e.g., tetrahydronaphthyl). Substituents including heterocyclic groups (e.g., heteroaryloxy, and heteroaralkylthio) are defined by analogy to the above-described terms.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a straight or branched chain, or cyclic carbon-containing radical, or combinations thereof, consisting of the stated number of carbon atoms and at least one heteroatom selected from the group consisting of O, N, Si, P, S, and Se and wherein the nitrogen, phosphorous, sulfur, and selenium atoms are optionally oxidized, and the nitrogen heteroatom is optionally be quaternized. The heteroatom(s) O, N, P, S, Si, and Se may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃, -CH₂-CH₂-N(CH₃)-CH₃,-CH₂-S-CH₂-CH₃, -CH₂-CH₂,-S(O)-CH₃, -CH₂-CH₂-S(O)₂-CH₃, -CH=CH-O-CH₃,-Si(CH₃)₃, -CH₂-CH=N-OCH₃, and -CH=CH-N(CH₃)-CH₃. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH2-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula-C(O)₂R'- represents both -C(O)₂R'- and -R'C(O)₂-.

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1 -(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1 piperazinyl, 2-piperazinyl, and the like.

The term "aryl" means, unless otherwise stated, a polyunsaturated, aromatic moiety that can be a single ring or multiple rings (preferably from 1 to 3 rings), which are fused together or linked covalently. The term "heteroaryl" refers to aryl groups (or rings) that contain from one to four heteroatoms selected from N, O, S, and Se, wherein the nitrogen, sulfur, and selenium atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, tetrazolyl, benzo[b]furanyl, benzo[b]thienyl, 2,3-dihydrobenzo[1,4]dioxin-6-yl, benzo[1,3]dioxol-5-yl and 6-quinolyl. Substituents for each of the above noted aryl and heteroaryl ring systems are selected from the group of acceptable substituents described below.

For brevity, the term "aryl" when used in combination with other terms (*e.g.,* aryloxy, arylthioxy, arylalkyl) includes both aryl and heteroaryl rings as defined above. Thus, the term "arylalkyl" is meant to include those radicals in which an aryl group is attached to an alkyl group (*e.g.,* benzyl, phenethyl, pyridylmethyl and the like) including those alkyl groups in which a carbon atom (*e.g.,* a methylene group) has been replaced by, for example, an oxygen atom (*e.g.,* phenoxymethyl, 2-pyridyloxymethyl, 3-(1-naphthyloxy)propyl, and the like).

Each of the above terms (*e.g.,* "alkyl," "heteroalkyl," "aryl" and "heteroaryl") includes both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are generically referred to as "alkyl group substituents," and they can be one or more of a variety of groups selected from, but not limited to: -OR', =O, NR', =N-OR', -NR'R", -SR', -halogen, -SiR'R"R"', -OC(O)R', -C(O)R', -CO₂R', -CONR'R", =OC(O)NR'R", -NR"C(O)R', -NR'-C(O)NR"R"', -NR"C(O)₂R', -NR-C(NR'R"R"')=NR"", -NR-C(NR'R")=NR"',-S(O)R', -S(O)₂R', -S(O)₂NR'R", -NRSO₂R', -CN and -NO₂ in a number ranging from zero to (2m'+1), where m' is the total number of carbon atoms in such radical. R', R", R'" and R"" each preferably independently refer to hydrogen, substituted or unsubstituted heteroalkyl, substituted or unsubstituted aryl, e.g., aryl substituted with 1-3 halogens, substituted or unsubstituted alkyl, alkoxy or thioalkoxy groups, or arylalkyl groups. When a compound includes more than one R group, for example, each of the R groups is independently selected as are each R', R", R"' and R"" groups when more than one of these groups is present. When R' and R" are attached to the same nitrogen atom, they can be combined with the nitrogen atom to form a 5-, 6-, or 7-membered ring. For example,-NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl *(e.g.,* -CF₃ and -CH₂CF₃) and acyl *(e.g.,* -C(O)CH₃,-C(O)CF₃, -C(O)CH₂OCH₃, and the like).

Similar to the substituents described for the alkyl radical, substituents for the aryl and heteroaryl groups are generically referred to as "aryl group substituents." The substituents are selected from, for example: halogen, -OR', =O, =NR', N-OR', -NR'R", -SR', -halogen, -SiR'R"R"', -OC(O)R', -C(O)R', -CO₂R', -CONR'R", -OC(O)NR'R",-NR"C(O)R', -NR'-C(O)NR"R"', -NR"C(O)₂R', -NR-C(NR'R"R'")=NR"", -NR-C(NR'R")=NR'", -S(O)R', -S(O)₂R', -S(O)₂NR'R", -NRSO₂R', -CN and -NO₂,-R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R'" and R"" are preferably independently selected from hydrogen, substituted or unsubstituted alkyl, substituted or unsubstituted heteroalkyl, substituted or unsubstituted aryl and substituted or unsubstituted heteroaryl. When a compound includes more than one R group, for example, each of the R groups is independently selected as are each R', R", R'" and R"" groups when more than one of these groups is present. In the schemes that follow, the symbol X represents "R" as described above.

Two of the substituents on adjacent atoms of the aryl or heteroaryl ring may optionally be replaced with a substituent of the formula T-C(O)-(CRR')_{q}-U-, wherein T and U are independently NR-, -O-, -CRR'- or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula -(CRR')ₛ-X-(CR"R"')_{d}-, where s and d are independently integers of from 0 to 3, and X is -0-, -NR'-, -S-, -S(O)-, -S(O)₂-, or-S(O)₂NR'-. The substituents R, R', R" and R'" are preferably independently selected from hydrogen or substituted or unsubstituted (C₁-C₆)alkyl.

As used herein, the term "heteroatom" includes oxygen (O), nitrogen (N), sulfur (S), phosphorus (P), silicon (Si), and selenium (Se).

The term "amino" or "amine group" refers to the group -NR'R" (or N⁺RR'R") where R, R' and R" are independently selected from the group consisting of hydrogen, alkyl, substituted alkyl, aryl, substituted aryl, aryl alkyl, substituted aryl alkyl, heteroaryl, and substituted heteroaryl. A substituted amine being an amine group wherein R' or R" is other than hydrogen. In a primary amino group, both R' and R" are hydrogen, whereas in a secondary amino group, either, but not both, R' or R" is hydrogen. In addition, the terms "amine" and "amino" can include protonated and quaternized versions of nitrogen, comprising the group -N⁺RR'R" and its biologically compatible anionic counterions.

The term "carboxyalkyl" as used herein refers to a group having the general formula -(CH₂)ₙCOOH wherein n is 1-18.

The term "activated alkyne," as used herein, refers to a chemical moiety that selectively reacts with an azide reactive group on another molecule to form a covalent chemical bond between the activated alkyne group and the alkyne reactive group. Activated alkynes include, but are not limited to the cyclooctynes and difluorocyclooctynes described by Agard et al., J. Am. Chem. Soc., 2004, 126 (46):15046-15047; the dibenzocyclooctynes described by Boon et al., WO2009/067663 A1 (2009); and the aza-dibenzocyclooctynes described by Debets et al., Chem. Comm., 2010, 46:97-99. These dibenzocyclooctynes (including the aza-dibenzocyclooctynes) described above are collectively referred to herein as cyclooctyne groups.

The term "affinity," as used herein, refers to the strength of the binding interaction of two molecules, such as an antibody and an antigen or a positively charged moiety and a negatively charged moiety. For bivalent molecules such as antibodies, affinity is typically defined as the binding strength of one binding domain for the antigen, e.g. one Fab fragment for the antigen. The binding strength of both binding domains together for the antigen is referred to as "avidity". As used herein "high affinity" refers to a ligand that binds to an antibody having an affinity constant (Kₐ) greater than 10⁴ M⁻¹, typically 10⁵-10¹¹ M⁻¹; as determined by inhibition ELISA or an equivalent affinity determined by comparable techniques such as, for example, Scatchard plots or using K_{d}/dissociation constant, which is the reciprocal of the Kₐ.

The term "alkyne reactive," as used herein, refers to a chemical moiety that selectively reacts with an alkyne modified group on another molecule to form a covalent chemical bond between the alkyne modified group and the alkyne reactive group. Examples of alkyne-reactive groups include, but are not limited to, azides. "Alkyne-reactive" can also refer to a molecule that contains a chemical moiety that selectively reacts with an alkyne group.

The term "antibody," as used herein, refers to a protein of the immunoglobulin (Ig) superfamily that binds noncovalently to certain substances (e.g. antigens and immunogens) to form an antibody-antigen complex. Antibodies can be endogenous, or polyclonal wherein an animal is immunized to elicit a polyclonal antibody response or by recombinant methods resulting in monoclonal antibodies produced from hybridoma cells or other cell lines. It is understood that the term "antibody" as used herein includes within its scope any of the various classes or sub-classes of immunoglobulin derived from any of the animals conventionally used.

The term "antibody fragments," as used herein, refers to fragments of antibodies that retain the principal selective binding characteristics of the whole antibody. Particular fragments are well-known in the art, for example, Fab, Fab', and F(ab')₂, which are obtained by digestion with various proteases, pepsin or papain, and which lack the Fc fragment of an intact antibody or the so-called "half-molecule" fragments obtained by reductive cleavage of the disulfide bonds connecting the heavy chain components in the intact antibody. Such fragments also include isolated fragments consisting of the light-chain-variable region, "Fv" fragments consisting of the variable regions of the heavy and light chains, and recombinant single chain polypeptide molecules in which light and heavy variable regions are connected by a peptide linker. Other examples of binding fragments include (i) the Fd fragment, consisting of the VH and CH1 domains; (ii) the dAb fragment (Ward, et al., Nature 341, 544 (1989)), which consists of a VH domain; (iii) isolated CDR regions; and (iv) single-chain Fv molecules (scFv) described above. In addition, arbitrary fragments can be made using recombinant technology that retains antigen-recognition characteristics.

The term "antigen," as used herein, refers to a molecule that induces, or is capable of inducing, the formation of an antibody or to which an antibody binds selectively, including but not limited to a biological material. Antigen also refers to "immunogen". The target-binding antibodies selectively bind an antigen, as such the term can be used herein interchangeably with the term "target".

The term "anti-region antibody," as used herein, refers to an antibody that was produced by immunizing an animal with a select region that is a fragment of a foreign antibody wherein only the fragment is used as the immunogen. Regions of antibodies include the Fc region, hinge region, Fab region, etc. Anti-region antibodies include monoclonal and polyclonal antibodies. The term "anti-region fragment" as used herein refers to a monovalent fragment that was generated from an anti-region antibody of the present invention by enzymatic cleavage.

The term "aqueous solution," as used herein, refers to a solution that is predominantly water and retains the solution characteristics of water. Where the aqueous solution contains solvents in addition to water, water is typically the predominant solvent.

The term "azide reactive," as used herein, refers to a chemical moiety that selectively reacts with an azido modified group on another molecule to form a covalent chemical bond between the azido modified group and the azide reactive group. Examples of azide-reactive groups include, but are not limited to, alkyne, including, but not limited to terminal alkynes; phosphines, including, but not limited to, triarylphosphines; and cyclooctynes and difluorocyclooctynes as described by Agard et al., J. Am. Chem. Soc., 2004, 126 (46):15046-15047, dibenzocyclooctynes as described by Boon et al., WO2009/067663 A1 (2009), and aza-dibenzocyclooctynes as described by Debets et al., Chem. Comm., 2010, 46:97-99. The various dibenzocyclooctynes described above are collectively referred to herein as cyclooctyne groups. "Azide-reactive" can also refer to a molecule that contains a chemical moiety that selectively reacts with an azido group.

The term "biomolecule," as used herein, refers to proteins, peptides, amino acids, glycoproteins, nucleic acids, nucleotides, nucleosides,oligonucleotides, sugars, oligosaccharides, lipids, hormones, proteoglycans, carbohydrates, polypeptides, polynucleotides, polysaccharides, which having characteristics typical of molecules found in living organisms and may be naturally occurring or may be artificial (not found in nature and not identical to a molecule found in nature).

The term "buffer," as used herein, refers to a system that acts to minimize the change in acidity or basicity of the solution against addition or depletion of chemical substances.

The term "carrier molecule," as used herein, refers to a biological or a non-biological component that is covalently bonded to compound of the present invention. Such components include, but are not limited to, an amino acid, a peptide, a protein, a polysaccharide, a nucleoside, a nucleotide, an oligonucleotide, a nucleic acid, a hapten, a psoralen, a drug, a hormone, a lipid, a lipid assembly, a synthetic polymer, a polymeric microparticle, a biological cell, a virus and combinations thereof.

The term, "chemical handle" as used herein refers to a specific functional group, such as an azide; alkyne, including, but not limited to, a terminal alkyne; activated alkyne; phosphite; phosphine, including, but not limited to a triarylphosphine; and the like. The chemical handle is distinct from the reactive group, defined below, in that the chemical handle are moieties that are rarely found in naturally-occurring biomolecules and are chemically inert towards biomolecules (e.g, native cellular components), but when reacted with an azide-reactive or alkyne-reactive group the reaction can take place efficiently under biologically relevant conditions (e.g., cell culture conditions, such as in the absence of excess heat or harsh reactants).

The term "click chemistry," as used herein, refers to the copper-catalyzed Huisgen cycloaddition or the 1,3- dipolar cycloaddition between an azide and a terminal alkyne to form a 1,2,4-triazole. Such chemical reactions can use, but are not limited to, simple heteroatomic organic reactants and are reliable, selective, stereospecific, and exothermic.

The term "cycloaddition" as used herein refers to a chemical reaction in which two or more π (pi)-electron systems (e.g., unsaturated molecules or unsaturated parts of the same molecule) combine to form a cyclic product in which there is a net reduction of the bond multiplicity. In a cycloaddition, the π (pi) electrons are used to form new π (pi) bonds. The product of a cycloaddition is called an "adduct" or "cycloadduct". Different types of cycloadditions are known in the art including, but not limited to, [3+2] cycloadditions and Diels-Alder reactions. [3+2] cycloadditions, which are also called 1,3-dipolar cycloadditions, occur between a 1,3-dipole and a dipolarophile and are typically used for the construction of five-membered heterocyclic rings. The term "[3+2] cycloaddition" also encompasses "copperless" [3+2] cycloadditions between azides and cyclooctynes and difluorocyclooctynes described by Agard et al., J. Am. Chem. Soc., 2004, 126 (46):15046-15047, the dibenzocyclooctynes described by Boon et al., WO2009/067663 A1 (2009), and the aza-dibenzocyclooctynes described by Debets et al., Chem. Comm., 2010,46:97-99.

The term "detectable response" as used herein refers to an occurrence of, or a change in, a signal that is directly or indirectly detectable either by observation or by instrumentation. Typically, the detectable response is an occurrence of a signal wherein the fluorophore is inherently fluorescent and does not produce a change in signal upon binding to a metal ion or biological compound. Alternatively, the detectable response is an optical response resulting in a change in the wavelength distribution patterns or intensity of absorbance or fluorescence or a change in light scatter, fluorescence lifetime, fluorescence polarization, or a combination of the above parameters. Other detectable responses include, for example, chemiluminescence, phosphorescence, radiation from radioisotopes, magnetic attraction, and electron density.

The term "detectably distinct" as used herein refers to a signal that is distinguishable or separable by a physical property either by observation or by instrumentation. For example, a fluorophore is readily distinguishable either by spectral characteristics or by fluorescence intensity, lifetime, polarization or photo-bleaching rate from another fluorophore in the sample, as well as from additional materials that are optionally present.

The term "directly detectable" as used herein refers to the presence of a material or the signal generated from the material is immediately detectable by observation, instrumentation, or film without requiring chemical modifications or additional substances.

The term "polyunsaturated fatty acid" as used herein refers to naturally occurring fatty acids having a hydrocarbon chain, usually between 18 to 24 carbons in length, two or more *cis*-double bonds between the carbon atoms of the chain, and would include, but is not limited to, the naturally occurring linoleic acid, alpha-linolenic acid, arachidonic acids, eicosapentaenoic acid, and docosahexaenoic acid.

The term "fluorophore" as used herein refers to a composition that is inherently fluorescent or demonstrates a change in fluorescence upon binding to a biological compound or metal ion, i.e., fluorogenic. Fluorophores may contain substitutents that alter the solubility, spectral properties or physical properties of the fluorophore. Numerous fluorophores are known to those skilled in the art and include, but are not limited to coumarin, cyanine, benzofuran, a quinoline, a quinazolinone, an indole, a benzazole, a borapolyazaindacene and xanthenes including fluoroscein, rhodamine and rhodol as well as other fluorophores described in RICHARD P. HAUGLAND, MOLECULAR PROBES HANDBOOK OF FLUORESCENT PROBES AND RESEARCH CHEMICALS (10th edition, CD-ROM, September 2005).

The term "glycoprotein," as used herein, refers to a protein that has been glycosolated and those that have been enzymatically modified, *in vivo* or *in vitro,* to comprise a sugar group.

The term "kit," as used herein, refers to a packaged set of related components, typically one or more compounds or compositions.

The term "label," as used herein, refers to a chemical moiety or protein that is directly or indirectly detectable (e.g. due to its spectral properties, conformation or activity) when attached to a target or compound and used in the present methods, including reporter molecules, solid supports and carrier molecules. The label can be directly detectable (fluorophore) or indirectly detectable (hapten or enzyme). Such labels include, but are not limited to, radiolabels that can be measured with radiation-counting devices; pigments, dyes or other chromogens that can be visually observed or measured with a spectrophotometer; spin labels that can be measured with a spin label analyzer; and fluorescent labels (fluorophores), where the output signal is generated by the excitation of a suitable molecular adduct and that can be visualized by excitation with light that is absorbed by the dye or can be measured with standard fluorometers or imaging systems, for example. The label can be a chemiluminescent substance, where the output signal is generated by chemical modification of the signal compound; a metal-containing substance; or an enzyme, where there occurs an enzyme-dependent secondary generation of signal, such as the formation of a colored product from a colorless substrate. The term label can also refer to a "tag" or hapten that can bind selectively to a conjugated molecule such that the conjugated molecule, when added subsequently along with a substrate, is used to generate a detectable signal. For example, one can use biotin as a tag and then use an avidin or streptavidin conjugate of horseradish peroxidate (HRP) to bind to the tag, and then use a colorimetric substrate (e.g., tetramethylbenzidine (TMB)) or a fluorogenic substrate such as Amplex Red reagent (Molecular Probes, Inc.) to detect the presence of HRP. Numerous labels are know by those of skill in the art and include, but are not limited to, particles, fluorophores, haptens, enzymes and their colorimetric, fluorogenic and chemiluminescent substrates and other labels that are described in RICHARD P. HAUGLAND, MOLECULAR PROBES HANDBOOK OF FLUORESCENT PROBES AND RESEARCH PRODUCTS (9th edition, CD-ROM, September 2002), *supra.*

The term "linker" or "L", as used herein, refers to a single covalent bond or a series of stable covalent bonds incorporating 1-30 nonhydrogen atoms selected from the group consisting of C, N, O, S and P. Exemplary linking members include a moiety that includes -C(O)NH-, -C(O)O-, -NH-, -S-, -O-, and the like. A "cleavable linker" is a linker that has one or more cleavable groups that may be broken by the result of a reaction or condition. The term "cleavable group" refers to a moiety that allows for release of a portion, e.g., a reporter molecule, carrier molecule or solid support, of a conjugate from the remainder of the conjugate by cleaving a bond linking the released moiety to the remainder of the conjugate. Such cleavage is either chemical in nature, or enzymatically mediated. Exemplary enzymatically cleavable groups include natural amino acids or peptide sequences that end with a natural amino acid. In addition to enzymatically cleavable groups, it is within the scope of the present invention to include one or more sites that are cleaved by the action of an agent other than an enzyme. Exemplary non-enzymatic cleavage agents include, but are not limited to, acids, bases, light (e.g., nitrobenzyl derivatives, phenacyl groups, benzoin esters), and heat. Many cleaveable groups are known in the art. *See,* for example, Jung et al., Biochem. Biophys. Acta, 761: 152-162 (1983); Joshi et al., J. Biol. Chem., 265: 14518-14525 (1990); Zarling et al., J. Immunol., 124: 913-920 (1980); Bouizar et al., Eur. J. Biochem., 155: 141-147 (1986); Park et al., J. Biol. Chem., 261: 205-210 (1986); Browning et al., J. Immunol., 143: 1859-1867 (1989). Moreover a broad range of cleavable, bifunctional (both homo-and hetero-bifunctional) spacer arms are commercially available. An exemplary cleavable group, an ester, is cleavable group that may be cleaved by a reagent, e.g. sodium hydroxide, resulting in a carboxylate-containing fragment and a hydroxyl-containing product.

The term "lipid" as used herein refers fatty acids, their conjugates and derivatives. The fatty acids are made up of a hydrocarbon chain that terminates in a carboxy acid group, with the hydrocarbon chain usually between 4 to 24 carbons in length, which may be saturated or unsaturated, and attached to functional groups containing oxygen, halogens, nitrogen and sulfur. Where a double bond exists, there is the possibility of either a *cis* or *trans* geometric isomerism.

The term "modified biomolecule" as used herein refers to a biomolecule which has been modified by covalent attachment of the products of lipid peroxidation in a cell of the polyunsaturated fatty acid analogs of the present invention.

The term "phosphine reactive" as used herein refers to a chemical moiety that selectively reacts via Staudinger ligation with a phosphine group, including but not limited to a triarylphosphine group, on another molecule to form a covalent chemical bond between the triarylphosphine group and the phosphine reactive group. Examples of phosphine reactive groups include, but are not limited to, an azido group.

The term "post translational moiety" as used herein refers to any moiety that is attached to a protein by a chemical process occurring in a cell, whether the process is naturally occurring or induced. Examples of such moieties include, but are not limited to, the products of lipid peroxidation of the polyunsaturated fatty acids analogs of the present invention. As used herein, "azido, alkyne or phosphine modified post translational moiety" means any post translational moiety that comprises an azido group; an alkyne group, including, but not limited to, a terminal alkyne group or an activated alkyne group; or a phosphine group, including, but not limited to, a triarylphosphine group; which groups are rarely round in naturally occurring biological systems.

The terms "protein" and "polypeptide" are used herein in a generic sense to include polymers of amino acid residues of any length. The term "peptide" is used herein to refer to polypeptides having less than 100 amino acid residues, typically less than 10 amino acid residues. The terms apply to amino acid polymers in which one or more amino acid residues are an artificial chemical analogue of a corresponding naturally occurring amino acid, as well as to naturally occurring amino acid polymers.

The term "purified" as used herein refers to a preparation of a protein that is essentially free from contaminating proteins that normally would be present in association with the protein, e.g., in a cellular mixture or milieu in which the protein or complex is found endogenously such as serum proteins or cellular lysate.

The term "reactive group" as used herein refers to a group that is capable of reacting with another chemical group to form a covalent bond, *i. e.* is covalently reactive under suitable reaction conditions, and generally represents a point of attachment for another substance. As used herein, reactive groups refer to chemical moieties generally found in biological systems and that react under normal biological conditions, these are herein distinguished from the chemical handle, defined above, the azido, terminal alkyne, activated alkyne and triarylphosphine moieties of the present invention. As refered to herein the reactive group is a moiety, such as carboxylic acid or succinimidyl ester, on the compounds of the present invention that is capable of chemically reacting with a functional group on a different compound to form a covalent linkage. Reactive groups generally include nucleophiles, electrophiles and photoactivatable groups.

The term "reporter molecule" refers to any moiety capable of being attached to a post translationally modified protein of the present invention, and detected either directly or indirectly. Reporter molecules include, without limitation, a chromophore, a fluorophore, a fluorescent protein, a phosphorescent dye, a tandem dye, a particle, a hapten, an enzyme and a radioisotope. Reporter molecules include, but are not limited to, fluorophores, fluorescent proteins, haptens, and enzymes.

The term "sample" as used herein refers to any material that may contain an analyte for detection or quantification or a post translationally modified protein of the present invention. The analyte may include a reactive group, e.g., a group through which a compound of the invention can be conjugated to the analyte. The sample may also include diluents, buffers, detergents, and contaminating species, debris and the like that are found mixed with the target. Illustrative examples include urine, sera, blood plasma, total blood, saliva, tear fluid, cerebrospinal fluid, secretory fluids from nipples and the like. Also included are solid, gel or sol substances such as mucus, body tissues, cells and the like suspended or dissolved in liquid materials such as buffers, extractants, solvents and the like. Typically, the sample is a live cell, a biological fluid that comprises endogenous host cell proteins, nucleic acid polymers, nucleotides, oligonucleotides, peptides and buffer solutions. The sample may also be a lysate isolated from a cell. The sample may be in an aqueous solution, a viable cell culture or immobilized on a solid or semi-solid surface such as a polyacrylamide gel, membrane blot or on a microarray.

The term "solid support," as used herein, refers to a material that is substantially insoluble in a selected solvent system, or which can be readily separated (e.g., by precipitation) from a selected solvent system in which it is soluble. Solid supports useful in practicing the present invention can include groups that are activated or capable of activation to allow selected one or more compounds described herein to be bound to the solid support.

The term "Staudinger ligation" as used herein refers to a chemical reaction developed by Saxon and Bertozzi (E. Saxon and C. Bertozzi, Science, 2000, 287: 2007-2010) that is a modification of the classical Staudinger reaction. The classical Staudinger reaction is a chemical reaction in which the combination of an azide with a phosphine or phosphite produces an aza-ylide intermediate, which upon hydrolysis yields a phosphine oxide and an amine. A Staudinger reaction is a mild method of reducing an azide to an amine; and triphenylphosphine is commonly used as the reducing agent. In a Staudinger ligation, an electrophilic trap (usually a methyl ester) is appropriately placed on the aryl group of a triarylphosphine (usually ortho to the phosphorus atom) and reacted with the azide, to yield an aza-ylide intermediate, which rearranges in aqueous media to produce a compound with amide group and a phosphine oxide function. The Staudinger ligation is so named because it ligates (attaches/covalently links) the two starting molecules together, whereas in the classical Staudinger reaction, the two products are not covalently linked after hydrolysis.

The terms "structural integrity of the [biomolecule] is not reduced" or "preservation of the structural integrity of the [biomolecule]", as used herein, means that either: 1) when analyzed by gel electrophoresis and detection (such as staining), a band or spot arising from the labeled biomolecule is not reduced in intensity by more than 20%, and preferably not reduced by more than 10%, with respect to the corresponding band or spot arising from the same amount of the electrophoresed unlabeled biomolecule, arising from the labeled biomolecule analyzed; or 2) when analyzed by gel electrophoresis, a band or spot arising from the labeled biomolecule is not observed to be significantly less sharp than the corresponding band or spot arising from the same amount of the electrophoresed unlabeled biomolecule, where " significantly less sharp" (synonymous with "significantly more diffuse") means the detectable band or spot takes up at least 5% more, preferably 10% more, more preferably 20% more area on the gel than the corresponding unlabeled biomolecule. Other reproducible tests for structural integrity of labeled biomolecules include, without limitation detection of released amino acids or peptides, or mass spectrometry.

In general, for ease of understanding, the modification of biomolecules within a cell with post translational moieties (such as the polyunsaturated fatty acid analogs of the present invention) comprising azide moieties; alkyne moieties, including, but not limited to terminal alkyne moieties; activated alkyne moieties; or phosphine moieties, including, but not limited to, triarylphosphine moieties; and the chemical labeling of such moieties with azide reactive moieties, alkyne reactive moieties or phosphine reactive moieties will first be described in detail. This will be followed by some disclosure in which such labeled biomolecules can be detected, isolated and/or analyzed.

Accordingly, provided herein are compounds, compositions, methods, and kits for the labeling, detecting, isolating and/or analysis of biomolecule modified by the lipid peroxidation in a cell of the polyunsaturated fatty acids analogs of the present invention. In particular, presented are compounds which are novel polyunsaturated fatty acid analogs comprising one or more alkyne reactive groups, one or more azide reactive groups; or comprising both an alkyne reactive group and an azide reactive group. Methods are also provided for modifying proteins using these polyunsaturated fatty acid analogs; methods for detecting the modified proteins, methods for isolating the modified proteins, and kits comprising such polyunsaturated fatty acid analogs are also presented. Exemplified methods are then disclosed.

### Polyunsaturated Fatty Acid Analogs

In one aspect, the present invention provides polyunsaturated fatty acid analogs. These analogs are compounds having the formula: 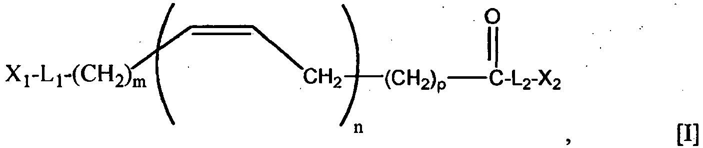 wherein
m may be 1-2, 1-3, 1-4, 2-3, 2-4 or 3-4;
n may be 2-3 or 5-6;
p is least 1, 2, 3, 4, 5, 6, 7, 8, 9, 10, 11 or 12, and at most 1, 2, 3, 4, 5, 6, 7, 8, 9, 10, 11, or 12;
at least one of X₁ or X₂ is selected from the group consisting of alkyne reactive moiety and azide reactive moiety, and the other is selected from the group consisting of H, alkyl, alkenyl, cycloalkyl, cycloalkenyl, alkoxy, aryl, aralkyl, heteroaryl, and heteroaralkyl; and

L₁ and L₂ is independently selected from the group consisting of O, NH, alkyl linker group comprising 1-10 carbon atoms, and alkyl linker group comprising 1-10 carbon atoms any of which may be substituted with one or more heteroatoms independently selected from the group consisting of O, N and S.

The compounds having formula [I] are not the following compounds:

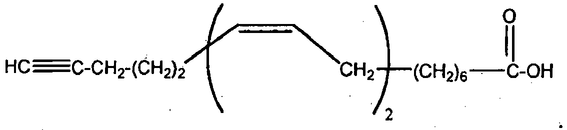

In certain embodiments, at least one of X₁ or X₂ may be selected from the group consisting of alkyne reactive moiety and azide reactive moiety, and the other is selected from the group consisting of H, alkyl comprising 1-10 carbon atoms, alkenyl comprising 1-10 carbon atoms, cycloalkyl comprising 3-10 carbon atoms, cycloalkenyl comprising 5-10 carbon atoms, alkoxy comprising 1-10 carbon atoms, aryl comprising 6-14 carbon atoms, aralkyl comprising 6-20 carbon atoms, heteroaryl comprising 5-14 carbon atoms, and heteroaralkyl comprising 5-20 carbon atom.

In certain embodiments, X₁ is an alkyne reactive moiety; and X₂ may be selected from the group consisting of an H, alkyl, alkenyl, cycloalkyl, cycloalkenyl, alkoxy, aryl, aralkyl, heteroaryl, and heteroaralkyl. In some of these, the alkyne reactive moiety is an azido group.

In certain embodiments, X₁ is azide reactive moiety; and X₂ may be selected from the group consisting of an H, alkyl, alkenyl, cycloalkyl, cycloalkenyl, alkoxy, aryl, aralkyl, heteroaryl, and heteroaralkyl. In some of these, the azide reactive moiety is as terminal alkyne, a cyclooctyne or a phosphine. In some of these, the azide reactive moiety is a terminal alkyne. In some of these, the alkyne is -C≡CH.

In certain embodiments, X₂ is an alkyne reactive moiety; and X₁ is selected from the group consisting of H, alkyl, alkenyl, cycloalkyl, cycloalkenyl, alkoxy, aryl, aralkyl, heteroaryl, and heteroaralkyl. In some of these, the alkyne reactive moiety is an azido group.

In certain embodiments, X₂ is an azide reactive moiety; and X₁ selected from the group consisting of H, alkyl, alkenyl, cycloalkyl, cycloalkenyl, alkoxy, aryl, aralkyl, heteroaryl, and heteroaralkyl. In some of these, the azide reactive moiety is a terminal alkyne, a cyclooctyne or a phosphine. In some of these, the azide reactive moiety is a terminal alkyne. In some of these, the terminal alkyne is -C=CH.

In certain embodiments, X₁ and X₂ are independently selected from the group consisting of an alkyne reactive moiety, and azide reactive moiety. In some of these, the alkyne reactive moiety is an azido group and the azide reactive moiety is a terminal alkyne, a cyclooctyne, or a phosphine. In some of these, the azide reactive moiety is a terminal alkyne. In some of these, the terminal alkyne is -C≡CH.

In certain embodiments, the compounds of the present invention is selected from the group consisting of:

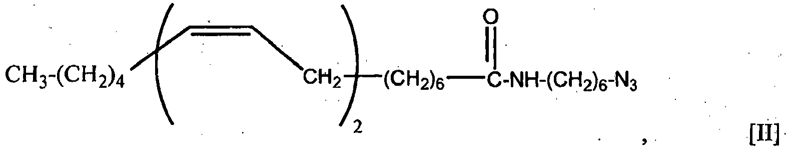

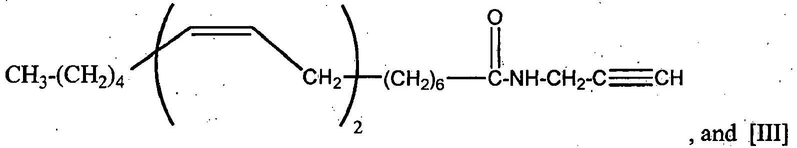

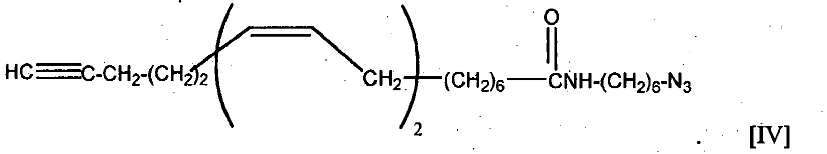

The compounds of the present invention may be made by the reaction schemes shown in FIG. 1 and FIG. 2.

### Modification of Biomolecules

The disclosure provides methods of modifying biomolecules with the compounds of the present invention to provide a modified biomolecule, and provides methods of labeling the modified biomolecule.

The tagging/labeling ofbiomolecules, (proteins in accordance with the methods of the invention), can utilize various post-translational modifications, including, but not limited to, lipid peroxidation, to incorporate a bioorthoganol moiety into a biomolecule followed by chemical attachment of a label (reporter molecule, carrier molecule or solid support). An approach is to incorporate a bioorthoganol moiety into the biomolecule inducing cellular biosynthetic pathways, such as, for example, lipid peroxidation. These bioorthogonol moieties are non-native, non-perturbing chemical handles possessing unique chemical functionality that can be modified through highly selective reactions. Examples of such moieties include, but are not limited to hydrazide and aminooxy derivatives; azides that can be selectively modified with an alkyne, including, but not limited to, terminal alkynes ("click" chemistry); azides that can be selectively modified with activated alkynes, including, but not limited to, cyclooctyne groups; and azides that can be selectively modified with phosphines, including, but not limited to, triarylphosphines (Staudinger ligation).

Post-translational modification is alteration of a primary structure of the protein after the protein has been translated. After translation, the post-translational modification of amino acids extends the range of functions of the protein by attaching to it other biochemical functional groups such as acetate, phosphate, various lipids and carbohydrates. In addition, the range of functions of proteins can be extended by post-translational modifications that change the chemical nature of an amino acid or by making structural changes such as disulfide bridges formation. Other post-translational modifications involve enzymes that remove amino acids from the amino end (N-terminus) of the protein, or cut the protein chain. Post-translational modifications act on individual residues either by cleavage at specific points, deletions, additions or by converting or modifying side chains.

The various post-translational modifications that can be used with the methods and compositions described herein, include, but are not limited to, lipid peroxidation, whether naturally occurring or induced. The post-translation modification of proteins can be performed *in vitro* or in cell culture. The post-translational modifications that involve structural changes which include, but are not limited to, attachment to proteins of the products of lipid peroxidation in a cell of the polyunsaturated fatty acids analogs of the present invention.

### Modified Biomolecules comprising Azide, Alkyne or Phosphine Moieties

Biomolecules that can be chemically modified using the methods decribed herein include, but are not limited to, proteins (including, but not limited to, glycoproteins), peptides, amino acids, protein or peptide hormones, proteoglycans, and polypeptides. Such biomolecules can contain azide moieties; alkyne moieties, including but not limited to, terminal alkyne moieties; activated alkyne moieties, including, but not limited to, cyclooctyne moieties; or phosphine moieties, including, but not limited to, triarylphosphine moieties, that are incorporated into biomolecules using post-translational modifications, such as, for example, lipid peroxidation. These azide moieties, alkyne moieties, activated alkyne moieties, and phosphine moieties are non-native, non-perturbing bioorthogonol chemical moieties that possess unique chemical functionality that can be modified through highly selective reactions. Such reactions are used in the methods described herein, wherein the chemical modification of biomolecules that contain azide moieties or terminal alkyne moieties utilize Copper(I)-catalyzed Azide-Alkyne Cycloaddition, also referred to herein as "click" chemistry; the chemical modification of biomolecules that contain azide moieties or activated-alkyne moieties that utilize a cycloaddition reaction; the chemical modification of biomolecules that contain azide moieties or triarylphosphine moieties utilize Staudinger ligation.

In certain variants, the biomolecules used in the methods and compositions described herein are modified chemically by supplying cells with alkyne-containing, activated alkyne-containing, phosphine-containing, or azido-containing molecular precursors that can be incorporated into biomolecules in the cell through lipid peroxidation. In certain variants, the biomolecules used in the methods and compositions described herein are modified by supplying cells with a terminal alkyne-containing, a cyclooctyne-containing, triarylphosphine-containing, or azido-containing molecular precursors that can be incorporated into biomolecules in the cell through lipid peroxidation. Such methods are described herein. ***"Click" Chemistry**"*

Azides and terminal or internal alkynes can undergo a 1,3-dipolar cycloaddition (Huisgen cycloaddition) reaction to give a 1,2,3-triazole. However, this reaction requires long reaction times and elevated temperatures. Alternatively, azides and terminal alkynes can undergo Copper(I)-catalyzed Azide-Alkyne Cycloaddition (CuAAC) at room temperature. Such copper(I)-catalyzed azide-alkyne cycloadditions, also known as "click" chemistry, is a variant of the Huisgen 1,3-dipolar cycloaddition wherein organic azides and terminal alkynes react to give 1,4-regioisomers of 1,2,3-triazoles. Examples of "click" chemistry reactions are described by Sharpless et al. (U.S. Patent Application Publication No. 20050222427, published October 6, 2005, International Application No. PCT /US03/17311; Lewis W G, et al., Angewandte Chemie-Int'l Ed. 41 (6): 1053; method reviewed in Kolb, H.C., et al., Angew. Chem. Inst. Ed. 2001, 40:2004-2021), which developed reagents that react with each other in high yield and with few side reactions in a heteroatom linkage (as opposed to carbon-carbon bonds) in order to create libraries of chemical compounds. As described herein, "click" chemistry is used in the methods for labeling modified biomolecules.

The copper used as a catalyst for the "click" chemistry reaction used in the methods described herein to conjugate a label to a modified biomolecule is in the Cu (I) reduction state. The sources of copper(I) used in such copper(I)-catalyzed azide-alkyne cycloadditions can be any cuprous salt including, but not limited to, cuprous halides such as cuprous bromide or cuprous iodide. However, this regioselective cycloaddition can also be conducted in the presence of a metal catalyst and a reducing agent. In certain embodiments, copper can be provided in the Cu (II) reduction state (for example, as a salt, such as but not limited to Cu(NO₃)₂ Cu(OAc)₂ or CuSO₄), in the presence of a reducing agent wherein Cu(I) is formed *in situ* by the reduction of Cu(II). Such reducing agents include, but are not limited to, ascorbate, tris(2-carboxyethyl) phosphine (TCEP), NADH, NADPH, thiosulfate, metallic copper, hydroquinone, vitamin K₁, glutathione, cysteine, 2-mercaptoethanol, dithiothreitol, Fe²⁺, Co²⁺, or an applied electric potential. In other embodiments, the reducing agents include metals selected from Al, Be, Co, Cr, Fe, Mg, Mn, Ni, Zn, Au, Ag, Hg, Cd, Zr, Ru, Fe, Co, Pt, Pd, Ni, Rh, and W.

The copper(I)-catalyzed azide-alkyne cycloadditions for labeling modified biomolecules can be performed in water and a variety of solvents, including mixtures of water and a variety of (partially) miscible organic solvents including alcohols, dimethyl sulfoxide (DMSO), dimethyl formamide (DMF), tert-butanol (tBuOH) and acetone.

Without limitation to any particular mechanism, copper in the Cu (I) state is a preferred catalyst for the copper(I)-catalyzed azide-alkyne cycloadditions, or "click" chemistry reactions, used in the methods described herein. Certain metal ions are unstable in aqueous solvents, by way of example, Cu(I), therefore stabilizing ligands/chelators can be used to improve the reaction. In certain embodiments at least one copper chelator is used in the methods described herein, wherein such chelators binds copper in the Cu (I) state. In certain embodiments, at least one copper chelator is used in the methods described herein, wherein such chelators binds copper in the Cu (II) state. In certain embodiments, the copper (I) chelator is a 1,10 phenanthroline-containing copper (I) chelator. Non-limiting examples of such phenanthroline-containing copper (I) chelators include, but are not limited to, bathophenanthroline disulfonic acid (4,7-diphenyl-1,10-phenanthroline disulfonic acid) and bathocuproine disulfonic acid (BCS; 2,9-dimethyl-4,7-diphenyl-1,10-phenanthroline disulfonate). In other embodiments, the copper(I) chelator is THPTA as described in Jentzsch et al., Inorganic Chemistry, 48(2): 9593-9595 (2009). In other embodiments, the copper(I) chelator are those described in Finn et al., U.S. Patent Publication No. US2010/0197871. Other chelators used in such methods include, but are not limited to, N-(2-acetamido)iminodiacetic acid (ADA), pyridine-2,6-dicarboxylic acid (PDA), S-carboxymethyl-L-cysteine (SCMC), trientine, tetra-ethylenepolyamine (TEPA), N,N,N',N'-tetrakis(2-pyridylmethyl)ethylenediamine (TPEN), EDTA, neocuproine, N-(2-acetamido)iminodiacetic acid (ADA), pyridine-2,6-dicarboxylic acid (PDA), S-carboxymethyl-L-cysteine (SCMC), tris-(benzyl-triazolylmethyl)amine (TBTA), or a derivative thereof. Most metal chelators, a wide variety of which are known in the chemical, biochemical, and medical arts, are known to chelate several metals, and thus metal chelators in general can be tested for their function in 1,3 cycloaddition reactions catatlyzed by copper. In certain embodiments, histidine is used as a chelator, while in other embodiments glutathione is used as a chelator and a reducing agent.

The concentration of the reducing agents used in the "click" chemistry reaction described herein can be in the micromolar to millimolar range. In certain embodiments, the concentration of the reducing agent is from about 100 micromolar to about 100 millimolar. In other embodiments, the concentration of the reducing agent is from about 10 micromolar to about 10 millimolar. In other embodiments, the concentration of the reducing agent is from about 1 micromolar to about 1 millimolar.

In certain variants, the methods describe herein for labeling modified biomolecules using "click" chemistry, at least one copper chelator is added after copper(II) used in the reaction has been contacted with a reducing agent. In other variants, at least one copper chelator can be added immediately after contacting copper(II) with a reducing agent. In other variants, the copper chelator(s) is added between about five seconds and about twenty-four hours after copper(II) and a reducing agent have been combined in a reaction mixture. In other variants, at least one copper chelator can be added any time to a reaction mixture that includes copper(II) and a reducing agent, such as, by way of example only, immediately after contacting copper(II) and a reducing agent, or within about five minutes of contacting copper(II) and a reducing agent in the reaction mixture. In some variants, at least one copper chelator can be added between about five seconds and about one hour, between about one minute and about thirty minutes, between about five minutes and about one hour, between about thirty minutes and about two hours, between about one hour and about twenty-four hours, between about one hour and about five hours, between about two hours and about eight hours, after copper(II) and a reducing agent have been combined for use in a reaction mixture.

In other variants, one or more copper chelators can be added more than once to such "click" chemistry reactions. In variants in which more than one copper chelators is added to a reaction, two or more of the copper chelators can bind copper in the Cu (I) state or, one or more of the copper chelators can bind copper in the Cu (I) state and one or more additional chelators can bind copper in the Cu (II) state. In certain variants, one or more copper chelators can be added after the initial addition of a copper chelator to the "click" chemistry reaction. In certain variants, the one or more copper chelators added after the initial addition of a copper chelator to the reaction can be the same or different from a copper chelator added at an earlier time to the reaction.

The concentration of a copper chelator used in the "click" chemistry reaction described herein can be determined and optimized using methods well known in the art, including those disclosed herein using "click" chemistry to label modified biomolecules followed by detecting such labeled biomolecules to determine the efficiency of the labeling reaction and the integrity of the labeled biomolecules. In certain variants, the chelator concentrations used in the methods described herein is in the micromolar to millimolar range, by way of example only, from 1 micromolar to 100 millimolar. In certain variants, the chelator concentration is from about 10 micromolar to about 10 millimolar. In other variants, the chelator concentration is from about 50 micromolar to about 10 millimolar. In other varaints the chelator, can be provided in a solution that includes a water-miscible solvent such as, alcohols, dimethyl sulfoxide (DMSO), dimethyl formamide (DMF), tert-butanol (tBuOH) and acetone. In other variants, the chelator can be provided in a solution that includes a solvent such as, for example, dimethyl sulfoxide (DMSO) or dimethylformamide (DMF).

In certain variants of the methods for labeling modified biomolecules utilising "click" chemistry described herein, the modified biomolecule can possess an azide moiety, whereupon the label possesses an alkyne moiety, whereas in other variants the modified biomolecule can possess an alkyne moiety, and the label possesses an azide moiety.

In certain variants of the methods for labeling modified biomolecules utilizing "click" chemistry described herein, the solution comprising the "click" chemistry reactants will further comprise Cu(I) ions; Cu(I) ions and a copper chelator; Cu(II) ions and at least one reducing agent; or Cu(II) ions, at least one reducing agent, and a copper chelator.

### Activated Alkyne Chemistry

Azides and alkynes can undergo catalyst free [3+2] cycloaddition by a using the reaction of activated alkynes with azides. Such catalyst-free [3+2] cycloaddition can be used in methods described herein to conjugate a label to a modified biomolecule. Alkynes can be activated by ring strain such as, by way of example only, eight membered ring structures, appending electron-withdrawing groups to such alkyne rings, or alkynes can be activated by the addition of a Lewis acid such as, by way of example only, Au(I) or Au(III). Alkynes activated by ring strain have been described. For example, the cyclooctynes and difluorocyclooctynes described by Agard et al., J. Am. Chem. Soc., 2004, 126 (46):15046-15047, the dibenzocyclooctynes described by Boon et al., WO2009/067663 A1 (2009), and the aza-dibenzocyclooctynes described by Debets et al., Chem. Comm., 2010, 46:97-99.

In certain variants of the methods for labeling modified biomolecule utilizing activated alkynes described herein, the biomolecule can possess an azide moiety, whereupon the label possesses an activated alkyne moiety; while in other variants the modified biomolecule can possess an activated alkyne moiety, and the label possesses an azide moiety.

### Staudinger Ligation

The Staudinger reaction, which involves reaction between trivalent phosphorous compounds and organic azides (Staudinger et al. Helv. Chim. Acta 1919, 2, 635), has been used for a multitude of applications. (Gololobov et al. Tetrahedron 1980, 37, 437); (Gololobov et al. Tetrahedron 1992, 48, 1353). There are almost no restrictions on the nature of the two reactants. The Staudinger ligation is a modification of the Staudinger reaction in which an electrophilic trap (usually a methyl ester) is placed on a triaryl phosphine. In the Staudinger ligation, the aza-ylide intermediate rearranges, in aqueous media, to produce an amide linkage and the phosphine oxide, ligating the two molecules together, whereas in the Staudinger reaction the two products are not covalently linked after hydrolysis. Such ligations have been described in U.S. Patent Application No. 20060276658. In certain variants, the phosphine can have a neighboring acyl group such as an ester, thioester or N-acyl imidazole (i.e. a phosphinoester, phosphinothioester, phosphinoimidazole) to trap the aza-ylide intermediate and form a stable amide bond upon hydrolysis. In certain variants, the phosphine can be a di- or triarylphosphine to stabilize the phosphine. The phosphines used in the Staudinger liagation methods described herein to conjugate a label to a modified biomolecule include, but are not limited to, cyclic or acyclic, halogenated, bisphosphorus, or even polymeric. Similarly, the azides can be alkyl, aryl, acyl or phosphoryl. In certain variants, such ligations are carried out under oxygen-free anhydrous conditions. The biomolecules described herein can be modified using a Staudinger ligation.

In certain variants of the methods for labeling modified biomolecules utilizing Staudinger ligation described herein, the modified biomolecule can possess an azide moiety, whereupon the label possesses a phosphine moiety, including, but not limited to, a triarylphosphine moiety; while in other variants the modified biomolecule can possess the phosphine moiety, and the label possesses an azide moiety.

### Chemical modification of Post Translatinally Modified Biomolecules

Protein can be modified using nucleophilic substitution reactions with amines, carboxylates or sulfhydryl groups which are found more commonly on the surface of proteins. However, the methods described herein utilize "click" reactions, cycloaddition reactions, or Staudinger ligation rather than nucleophilic substitution reactions, for selective modifications of biomolecules. Thus, biomolecules described herein can be modified, with the polyunsaturated fatty acid analogs described herein. Such reactions can be carried out at room temperature in aqueous conditions. In the case of "click" chemistry as described herein, excellent regioselectivity is achieved by the addition of catalytic amounts of Gu(I) salts to the reaction mixture. See, e.g., Tomoe, et al., (2002) Org. Chem. 67:3057-3064; and, Rostovtsev, et al., (2002) Angew. Chem. Int. Ed. 41:2596-2599. The resulting five-membered ring resulting from "click" chemistry cycloaddition is not generally reversible in reducing environments and is stable against hydrolysis for extended periods in aqueous environments. Thus, biomolecules attached to a labeling agent, a detection agent, a reporter molecule, a solid support or a carrier molecule via such five-membered ring are stable in reducing environments.

After biomolecules, (proteins in accordance with the methods of the invention), have been modified with either azido moieties, alkyne moieties, including but not limited to, terminal alkyne moieties, such as, for example, a -C=CH moiety; activated alkyne moieties, including, but not limited to a cyclooctyne moiety; or phosphine moieties, including, but not limited to a triarylphosphine moiety; they can be reacted under appropriate conditions to form conjugates with reporter molecules, carrier molecules or solid supports. In certain variants, such biomolecules used for such conjugations may be present as in a cell; as a cell lysate; as an isolated biomolecule; and/or as purified biomolecule, separated by gel electrophoresis or on a solid or semi-solid matrix.

In the methods and compositions described herein, the azide moiety may be used as the alkyne reactive group on the modified biomolecule, and an azide reactive moiety on a reporter molecule, a solid support or a carrier molecule; or the alkyne, activated alkyne or phosphine moiety may be used as the azide reactive group on the modified biomolecule, and an azide moiety may be used as an alkyne reactive moiety on a reporter molecule, a solid support or a carrier molecule. The azide reactive moiety may comprise an alkyne moiety, including, but not limited to, a terminal alkyne group, including, but not limited to, -C≡CH; an activated alkyne moiety, including, but not limited to a cyclooctyne group; or a phosphine moiety, including, but not limited to, a triarylphosphine group. In certain variants, the biomolecules may be modified with one or more alkyne reactive moieties, or one or more azide reactive moieties. In the methods of the invention, such biomolecules are proteins.

In certain embodiments of the methods and compositions described herein, a modified protein comprising at least one azido group can be selectively labeled with a reporter molecule, a solid support and/or a carrier molecule that comprises at least one azide reactive group including, but not limited to, an alkyne group, an activated alkyne group, or a phosphine group, or a combination thereof. In other embodiments, a modified protein comprising at least one alkyne group, including, but not limited to a terminal alkyne group, such as for example, -C=CH; an activated alkyne group, including, but not limited to, a cyclooctyne group; or a phosphine group, including, but not limited to, a triarylphosphine group, can be selectively labeled with a reporter molecule, a solid support and/or a carrier molecule that comprises at least one alkyne reactive group including, but not limited to, an azido group. In other embodiments, a modified protein comprising at least one alkyne group, including, but not limited to a terminal alkyne group, such as for example, -C=CH; at least one activated alkyne group, including, but not limited to a cyclooctyne group; or at least one phosphine group, including, but not limited to a triarylphosphine group, can be selectively labeled with a reporter molecule, a solid support and/or a carrier molecule that comprises at least one alkyne reactive group including, but not limited to, an azido group.

In certain variants, two azide-reactive groups are used to label modified biomolecules: the first may be a terminal alkyne group, such as, for example, such as, for example, -C=CH, used in a "click" chemistry reaction, and the second is a phosphine, such as a triarylphosphine group, used in a Staudinger ligation. In other variants, two azide-reactive groups are used to label modified biomolecules: the first may be a terminal alkyne group, such as, for example, -C≡CH, used in a "click" chemistry reaction, and the second may be an activated alkyne group, such as a cyclooctyne group, used in a cycloaddition reaction.

In certain variants, an alkyne reactive moiety and an azide reactive moiety are used to label modified biomolecules: the first may be an alkyne reactive moiety used in a "click" chemistry reaction, such as, for example, an azido group; and the second may be a terminal alkyne group, such as, for example, -C=CH; an activated alkyne group, such as, for example, a cyclooctyne group, used in a cycloaddition reaction; or a phosphine group, such as, for example, a triarylphosphine group, used in a Staudinger ligation.

In one variant, "click" chemistry is utilized to form a conjugate with a biomolecule comprising an azido group; and a reporter molecule, solid support or carrier molecule, wherein the reporter molecule, solid support and carrier molecule comprises an alkyne group, such as, for example, a terminal alkyne group. In another variant, "click" chemistry is utilized to form a conjugate with a biomolecule comprising an alkyne group, such as, for example, a terminal alkyne group; and a reporter molecule, solid support and/or carrier molecule, wherein the reporter molecule, solid support and carrier molecule comprises an azido group.

In another variant, a cycloaddition reaction is utilized to form a conjugate with a biomolecule comprising an activated alkyne group, such as, for example, a cyclooctyne group; and a reporter molecule, solid support and/or carrier molecule, wherein the reporter molecule, solid support and carrier molecule contains an azido group.

In another variant, a cycloaddition reaction is utilized to form a conjugate with a biomolecule comprising an azido group, and a reporter molecule, solid support and/or carrier molecule, wherein the reporter molecule, solid support and carrier molecule comprises activated alkyne group, such as, for example, a cyclooctyne group.

In another variant, a Staudinger ligation is utilized to form a conjugate with a biomolecule comprising an azido group; and a reporter molecule, solid support and/or carrier molecule, wherein the reporter molecule, solid support and carrier molecule comprises a phosphine group, such as, for example, a triarylphosphine group.

In another variant, a Staudinger ligation is utilized to form a conjugate with a protein comprising a phosphine group, such as, for example, a triaryl phosphine group; and a reporter molecule, solid support and/or carrier molecule, wherein the reporter molecule, solid support and carrier molecule compries an azido group.

The methods described herein are not intended to be limited to these two azide reactive groups, or chemical reactions, but it is envisioned that any chemical reaction utilizing an azide reactive group attached to a reporter molecule, solid support or carrier molecule can be used with the azide modified proteins described herein.

The reporter molecules, solid supports and carrier molecules used in the methods and compositions described herein can comprise at least one alkyne group, including, but not limited to, a terminal alkyne group; at least one activated alkyne group, including, but not limited to, a cyclooctyne group; or at least one phosphine group, including, but not limited to a triarylphosphine group; capable of reacting with an azido group of the modified biomolecule of the present invention. The reporter molecules, solid supports, and carrier molecules used in the methods and compositions described herein, can comprise at least one azide moiety capable of reacting with the alkyne group, activated alkyne group, or a phosphine group of the modified biomolecules of the present disclosure.

In certain variants, the alkyne group of the reporter molecules, solid supports, and carrier molecules described herein is a terminal alkyne group capable of reacting with the modified biomolecule of the present invention. In some variants, the terminal alkyne group is -C=CH.

In certain variants, the activated alkyne group of the reporter molecules, solid supports, and carrier molecules described herein is a terminal alkyne group capable of reacting with the modified biomolecule of the disclosure. In some variants, the activated alkyne group is a cyclooctyne group.

In certain variants, the phosphine group of the reporter molecules, solid supports, and carrier molecules described herein is a phosphine group capable of reacting with the modified biomolecule of the disclosure. In some variants, the phosphine group is a triarylphosphine group.

In certain variants, the reporter molecules used in the methods and compositions described herein can include, but are not limited to labels, while the carrier molecules can include, but are not limited to, affinity tags, nucleotides, oligonucleotides and polymers. The solid supports can include, but are not limited to, solid support resins, microtiter plates and microarray slides.

### Reporter Molecules

In accordance with the methods and compositions described herein, the modified biomolecules can be conjugated to a reporter molecule.

The reporter molecules used in the methods and compositions provided herein include any directly or indirectly detectable reporter molecule known by one skilled in the art that can be covalently attached to a modified biomolecule of the disclosure, (a protein in accordance with methods of the invention). Such modified proteins can be azide modified proteins, alkyne modified proteins, activated alkyne modified proteins, or phosphine modified proteins. In certain variants, the reporter molecules used in the methods and compositions provided herein include any directly or indirectly detectable reporter molecule known by one skilled in the art that can be covalently attached to an azide modified protein, an alkyne modified protein, activated alkyne modified protein or a phosphine modified protein.

Reporter molecules used in the methods and compositions described herein can contain, but are not limited to, a chromophore, a fluorophore, a fluorescent protein, a phosphorescent dye, a tandem dye, a particle, a hapten, an enzyme and a radioisotope. In certain embodiments, such reporter molecules include fluorophores, fluorescent proteins, haptens, and enzymes.

A fluorophore used in a reporter molecule in the methods and compositions described herein, can contain one or more aromatic or heteroaromatic rings, that are optionally substituted one or more times by a variety of substituents, including without limitation, halogen, nitro, cyano, alkyl, perfluoroalkyl, alkoxy, alkenyl, alkynyl, cycloalkyl, arylalkyl, acyl, aryl or heteroaryl ring system, benzo, or other substituents typically present on fluorophores known in the art.

A fluorophore used in a reporter molecule in the methods and compositions described herein, is any chemical moiety that exhibits an absorption maximum at wavelengths greater than 280 nm, and retains its spectral properties when covalently attached to a labeling reagent such as, by way of example only, an azide, and alkyne or a triarylphosphine. Fluorophores used as in reporter molecule in the methods and compositions described herein include, without limitation; a pyrene (including any of the corresponding derivative compounds disclosed in U.S. Patent No. 5,132,432); an anthracene; a naphthalene; an acridine; a stilbene; an indole or benzindole; an oxazole or benzoxazole; a thiazole or benzothiazole; a 4-amino-7-nitrobenz-2-oxa-1,3-diazole (NBD); a cyanine (including any corresponding compounds in U.S. Serial Nos. 09/968,401 and 09/969,853); a carbocyanine (including any corresponding compounds in U.S. Serial Nos. 09/557,275, 09/969,853, and 09/968,401, U.S. Patents Nos. 4,981,977, 5,268,486, 5,569,587, 5,569,766, 5,486,616, 5,627,027, 5,808,044, 5,877,310, 6,002,003, 6,004,536, 6,008,373, 6,043,025, 6,127,134, 6,130,094, 6,133,445, and publications WO 02/26891, WO 97/40104, WO 99/51702, WO 01/21624; EP 1 065 250 A1); a carbostyryl; a porphyrin; a salicylate; an anthranilate; an azulene; a perylene; a pyridine; a quinoline; a borapolyazaindacene (including any corresponding compounds disclosed in U.S. Patent Nos. 4,774,339, 5,187,288, 5,248,782, 5,274,113,and 5,433,896; a xanthene (including any corresponding compounds disclosed in U.S. Patent No. 6,162,931, 6,130,101, 6,229,055, 6,339,392, 5,451,343, and U.S. serial No. 09/922,333); an oxazine (including any corresponding compounds disclosed in U.S. Patent No. 4,714,763) or a benzoxazine; a carbazine (including any corresponding compounds disclosed in U.S. Patent No. 4,810,636); a phenalenone; a coumarin (including an corresponding compounds disclosed in U.S. Patent Nos. 5,696,157; 5,459,276; 5,501,980 and 5,830,912); a benzofuran (including an corresponding compounds disclosed in U.S. Patent Nos. 4,603,209 and 4,849,362); benzphenalenone (including any corresponding compounds disclosed in U.S. Patent No. 4,812,409); a carbopyranine, a semiconductor nanocrystal; and derivatives thereof. As used herein, oxazines include resorufins (including any corresponding compounds disclosed in U.S Patent No. 5,242,805), aminooxazinones, diaminooxazines, and their benzo-substituted analogs.

Xanthene type fluorophores used in reporter molecule in the methods and compositions described herein include, but are not limited to, a fluorescein, a rhodol (including any corresponding compounds disclosed in U.S. Patent Nos. 5,227,487 and 5,442,045), or a rhodamine (including any corresponding compounds in US Patent Nos. 5,798,276; 5,846,737; U.S. Serial no. 09/129,015). As used herein, fluorescein includes benzo- or dibenzofluoresceins, seminaphthofluoresceins, or naphthofluoresceins. Similarly, as used herein rhodol includes seminaphthorhodafluors (including any corresponding compounds disclosed in U.S. Patent No. 4,945,171). In certain variants, the fluorophore is a xanthene that is bound via a linkage that is a single covalent bond at the 9-position of the xanthene. In other variants, the xanthenes include derivatives of 3*H-*xanthen-6-ol-3-one attached at the 9-position, derivatives of 6-amino-3*H-*xanthen-3-one attached at the 9-position, or derivatives of 6-amino-3*H*-xanthen-3-imine attached at the 9-position.

In certain variants, the fluorophores used in reporter molecules in the methods and compositions described herein include xanthene (rhodol, rhodamine, fluorescein and derivatives thereof) coumarin, cyanine, pyrene, oxazine, borapolyazaindacene, carbopyranine, or semiconductor nanocrystal. In other variants, such fluorphores are sulfonated xanthenes, fluorinated xanthenes, sulfonated coumarins, fluorinated coumarins and sulfonated cyanines.

In other variants, the fluorophores used in reporter molecules in the methods and compositions described herein are those that have been modified with a azide moiety, terminal alkyne moiety, activated alkyne moiety or phosphine moiety. When used in "click" chemistry reaction such fluorphores form triazole products which do not requires UV excitation and overcome any quenching effect due to conjugation of azido or terminal alkyne groups to the fluorescent π-system.

The choice of the fluorophore attached to the labeling reagent will determine the absorption and fluorescence emission properties of the labeling reagent, modified biomolecule and immuno-labeled complex. Physical properties of a fluorophore label that can be used for detection of modified biomolecules and an immune-labeled complex include, but are not limited to, spectral characteristics (absorption, emission and stokes shift), fluorescence intensity, lifetime, polarization and photo-bleaching rate, or combination thereof. All of these physical properties can be used to distinguish one fluorophore from another, and thereby allow for multiplexed analysis. In certain embodiments, the fluorophore has an absorption maximum at wavelengths greater than 480 nm. In other embodiments, the fluorophore absorbs at or near 488 nm to 514 nm (particularly suitable for excitation by the output of the argon-ion laser excitation source) or near 546 nm (particularly suitable for excitation by a mercury arc lamp).

Many of fluorophores can also function as chromophores and thus the described fluorophores are also chromophores used in reporter molecules in the methods and compositions described herein.

In addition to fluorophores, enzymes also find use as labels for the detection reagents/reporter molecules used in the methods and compositions described herein. Enzymes are desirable labels because amplification of the detectable signal can be obtained resulting in increased assay sensitivity. The enzyme itself does not produce a detectable response but functions to break down a substrate when it is contacted by an appropriate substrate such that the converted substrate produces a fluorescent, colorimetric or luminescent signal. Enzymes amplify the detectable signal because one enzyme on a labeling reagent can result in multiple substrates being converted to a detectable signal. This is advantageous where there is a low quantity of target present in the sample or a fluorophore does riot exist that will give comparable or stronger signal than the enzyme. However, fluorophores are most preferred because they do not require additional assay steps and thus reduce the overall time required to complete an assay. The enzyme substrate is selected to yield the preferred measurable product, e.g. colorimetric, fluorescent or chemiluminescence. Such substrates are extensively used in the art, many of which are described in the MOLECULAR PROBES HANDBOOK, *supra.*

In certain variants, colorimetric or fluorogenic substrate and enzyme combination use oxidoreductases such as, by way of example only, horseradish peroxidase and a substrate such as, by way of example only, 3,3'-diaminobenzidine (DAB) or 3-amino-9-ethylcarbazole (AEC), which yield a distinguishing color (brown and red, respectively). Other colorimetric oxidoreductase substrates used with the enzymatic reporter molecules described herein include, but are not limited to: 2,2-azino-bis(3-ethyltienzothiazoline-6-sulfonic acid) (ABTS), o-phenylenediamine (OPD), 3,3',5,5'-tetramethylbenzidine (TMB), o-dianisidine, 5-aminosalicylic acid, 4-chloro-1-naphthol. Fluorogenic substrates used with the enzymatic reporter molecules described herein include, but are not limited to, homovanillic acid or 4-hydroxy-3-methoxyphenylacetic acid, reduced phenoxazines and reduced benzothiazines, including Amplex^{®} Red reagent and its variants (U.S. Patent No. 4,384,042), Amplex UltraRed and its variants in (WO05042504) and reduced dihydroxanthenes, including dihydrofluoresceins (U.S. Patent No. 6,162,931) and dihydrorhodamines including dihydrorhodamine 123. Peroxidase substrates can be used with the enzymatic reporter molecules described herein. Such peroxide substrates include, but are not limited to, tyramides (U.S. Patent Nos. 5,196,306; 5,583,001 and 5,731,158) which represent a unique class of peroxidase substrates in that they can be intrinsically detectable before action of the enzyme but are "fixed in place" by the action of a peroxidase in the process described as tyramide signal amplification (TSA). These substrates are extensively utilized to label targets in samples that are cells, tissues or arrays for their subsequent detection by microscopy, flow cytometry, optical scanning and fluorometry.

In other variants the colorimetric (and in some cases fluorogenic) substrates and enzymes combination used in reporter molecules described herein include a phosphatase enzyme such as, by way of example only, an acid phosphatase, an alkaline phosphatase or a recombinant version of such a phosphatase. A colorimetric substrate used in combination with such phosphatases include, but are not limited to, 5-bromo-6-chloro-3-indolyl phosphate (BCIP), 6-chloro-3-indolyl phosphate, 5-bromo-6-chloro-3-indolyl phosphate, *p*-nitrophenyl phosphate, or o-nitrophenyl phosphate or with a fluorogenic substrate such as 4-methylumbelliferyl phosphate, 6,8-difluoro-7-hydroxy-4-methylcoumarinyl phosphate (DiFMUP, U.S. Patent No. 5,830,912), fluorescein diphosphate, 3-*O*-methylfluorescein phosphate, resorufin phosphate, 9*H-*(1,3-dichloro-9,9-dimethylacridin-2-one-7-yl) phosphate (DDAO phosphate), or ELF 97, ELF 39 or related phosphates (U.S. Patent Nos. 5,316,906 and 5,443,986).

Other enzymes used in reporter molecules described herein include glycosidases, including, but not limited to, beta-galactosidase, beta-glucuronidase and beta-glucosidase. The colorimetric substrates used with such enzymes include, but are not limited to, 5-bromo-4-chloro-3-indolyl beta-D-galactopyranoside (X-gal) and similar indolyl galactosides, glucosides, and glucuronides, *o*-nitrophenyl beta-D-galactopyranoside (ONPG) and p-nitrophenyl beta-D-galactopyranoside. Preferred fluorogenic substrates include resorufin beta-D-galactopyranoside, fluorescein digalactoside (FDG), fluorescein diglucuronide and their structural variants (U.S. Patent Nos. 5,208,148; 5,242,805; 5,362,628; 5,576,424 and 5,773,236), 4-methylumbelliferyl beta-D-galactopyranoside, carboxyumbelliferyl beta-D-galactopyranoside and fluorinated coumarin beta-D-galactopyranosides (U.S. Patent No. 5,830,912).

Additional enzymes used in reporter molecules described herein include include, but are not limited to, hydrolases such as cholinesterases and peptidases, oxidases such as glucose oxidase and cytochrome oxidases, and reductases for which suitable substrates are known.

Enzymes and their appropriate substrates that produce chemiluminescence can also be used in reporter molecules described herein. Such enzymes include, but are not limited to, natural and recombinant forms of luciferases and aequorins. In addition, the chemiluminescence-producing substrates for phosphatases, glycosidases and oxidases such as those containing stable dioxetanes, luminol, isoluminol and acridinium esters an also be used in reporter molecules described herein.

In addition to enzymes, haptens can be used in label/reporter molecules described herein. In certain embodiments, such haptens include hormones, naturally occurring and synthetic drugs, pollutants, allergens, affector molecules, growth factors, chemokines, cytokines, lymphokines, amino acids, peptides, chemical intermediates, nucleotides, biotin and the like. Biotin is useful because it can function in an enzyme system to further amplify the detectable signal, and it can function as a tag to be used in affinity chromatography for isolation purposes. For detection purposes, an enzyme conjugate that has affinity for biotin is used, such as, by way of example only, avidin-Horse Radish Peroxidase (HRP). Subsequently a peroxidase substrate as described herein can be added to produce a detectable signal.

Fluorescent proteins can also be used in label/reporter molecules described herein for use in the methods, compositions and labeling reagents described herein. Non-limiting examples of such fluorescent proteins include green fluorescent protein (GFP) and the phycobiliproteins and the derivatives thereof. The fluorescent proteins, especially phycobiliprotein, are particularly useful for creating tandem dye labeled labeling reagents. These tandem dyes comprise a fluorescent protein and a fluorophore for the purposes of obtaining a larger stokes shift wherein the emission spectra is farther shifted from the wavelength of the fluorescent protein's absorption spectra. This is particularly advantageous for detecting a low quantity of a target in a sample wherein the emitted fluorescent light is maximally optimized, in other words little to none of the emitted light is reabsorbed by the fluorescent protein. The fluorescent protein and fluorophore function as an energy transfer pair wherein the fluorescent protein emits at the wavelength that the fluorophore absorbs and the fluorophore then emits at a wavelength farther from the fluorescent proteins emission wavelength than could have been obtained with only the fluorescent protein. A particularly useful combination is the phycobiliproteins disclosed in U.S. Patent Nos. 4,520,110; 4,859,582; 5,055,556 and the sulforhodamine fluorophores disclosed in U.S. Patent No. 5,798,276, or the sulfonated cyanine fluorophores disclosed in U.S. Serial Nos. 09/968/401 and 09/969/853; or the sulfonated xanthene derivatives disclosed in U.S. Patent No. 6,130,101 and those combinations disclosed in U.S. Patent No. 4,542,104. Alternatively, the fluorophore functions as the energy donor and the fluorescent protein is the energy acceptor.

### Carrier Molecules: Azide reactive, Alkyne reactive and Phosphine reactive

In the methods and compositions described herein, the modified biomolecules can be conjugated to a carrier molecule.

In certain variants provided herein the modified biomolecules are covalently conjugated to a carrier molecule. This includes, but is not limited to, any azide modified, alkyne modified, activated alkyne modified, and any phosphine modified biomolecule disclosed herein and any carrier disclosed herein.

In certain variants, the modified biomolecules are modified proteins that comprise at least one azido group and are capable of reacting with a carrier molecule comprising at least one alkyne group, including but not limited to a terminal alkyne group; at least one activated alkyne group, including, but not limited to, a cyclooctyne group; or at least one phosphine group, including, but not limited to, a triarylphosphine group. In some variants, the terminal group is -C≡CH.

In certain embodiments, the modified biomolecule are modified proteins that comprise at least one alkyne group, including but not limited to a terminal alkyne group; at least one activated alkyne group, including, but not limited to, a cyclooctyne group; or at least one phosphine group, including but not limited to a triarylphosphine group; capable of reacting with a carrier molecule comprising at least azido group. In some embodiments, the terminal alkyne group is -C≡CH.

A variety of carrier molecules can be used in the methods and compositions described herein, including, but not limited to, antigens, steroids, vitamins, drugs, haptens, metabolites, toxins, environmental pollutants, amino acids, peptides, proteins, nucleic acids, nucleic acid polymers, carbohydrates, lipids, and polymers. In certain variants, the carrier molecule contain an amino acid, a peptide; a protein, a polysaccharide, a nucleoside, a nucleotide, an oligonucleotide, a nucleic acid, a hapten, a psoralen, a drug, a hormone, a lipid, a lipid assembly, a synthetic polymer, a polymeric microparticle, a biological cell, a virus or combinations thereof.

In other variants, the carrier molecule is selected from a hapten, a nucleotide, an oligonucleotide, a nucleic acid polymer, a protein, a peptide or a polysaccharide. In still other varinats, the carrier molecule is an amino acid, a peptide, a protein, a polysaccharide, a nucleoside, a nucleotide, an oligonucleotide, a nucleic acid, a hapten, a psoralen, a drug, a hormone, a lipid, a lipid assembly, a tyramine, a synthetic polymer, a polymeric microparticle, a biological cell, cellular components, an ion chelating moiety, an enzymatic substrate or a virus. In further varinats, the carrier molecule is an antibody or fragment thereof, an antigen, an avidin or streptavidin, a biotin, a dextran, an IgG binding protein, a fluorescent protein, agarose, and a non-biological microparticle.

In certain variants wherein the carrier molecule is an enzymatic substrate, the enzymatic substrate is selected from an amino acid, a peptide, a sugar, an alcohol, alkanoic acid, 4-guanidinobenzoic acid, a nucleic acid, a lipid, sulfate, phosphate, - CH₂OCO-alkyl and combinations thereof. In certain embodiments, such enzyme substrates can be cleaved by enzymes selected from peptidases, phosphatases, glycosidases, dealkylases, esterases, guanidinobenzotases, sulfatases, lipases, peroxidases, histone deacetylases, exonucleases, reductases, endoglycoceramidases and endonucleases.

In other variants, the carrier molecule is an amino acid (including those that are protected or are substituted by phosphates, carbohydrates, or C₁ to C₂₂ carboxylic acids), or a polymer of amino acids such as a peptide or protein. In a related variant, the carrier molecule contains at least five amino acids, more preferably 5 to 36 amino acids. Such peptides include, but are not limited to, neuropeptides, cytokines, toxins, protease substrates, and protein kinase substrates. Other peptides may function as organelle localization peptides, that is, peptides that serve to target the conjugated compound for localization within a particular cellular substructure by cellular transport mechanisms, including, but not limited to, nuclear localization signal sequences. In certain embodiments, the protein carrier molecules include enzymes, antibodies, lectins, glycoproteins, histones, albumins, lipoproteins, avidin, streptavidin, protein A, protein G, phycobiliproteins and other fluorescent proteins, hormones, toxins and growth factors. In other variants, the protein carrier molecule is an antibody, an antibody fragment, avidin, streptavidin, a toxin, a lectin, or a growth factor. In further variants, the carrier molecules contain haptens including, but not limited to, biotin, digoxigenin and fluorophores.

The carrier molecules used in the methods and composition described herein can also contain a nucleic acid base, nucleoside, nucleotide or a nucleic acid polymer, optionally containing an additional linker or spacer for attachment of a fluorophore or other ligand, such as an alkynyl linkage (U.S. Patent No. 5,047,519), an aminoallyl linkage (U.S. Patent No. 4,711,955) or other linkage. In other variants, the nucleotide carrier molecule is a nucleoside or a deoxynucleoside or a dideoxynucleoside, while in other embodiments, the carrier molecule contains a peptide nucleic acid (PNA) sequence or a locked nucleic acid (LNA) sequence. In certain variants, the nucleic acid polymer carrier molecules are single- or multi-stranded, natural or synthetic DNA or RNA oligonucleotides, or DNA/RNA hybrids, or incorporating an unusual linker such as morpholine derivatized phosphates (AntiVirals, Inc., Corvallis OR), or peptide nucleic acids such as *N*-(2-aminoethyl)glycine units, where the nucleic acid contains fewer than 50 nucleotides, more typically fewer than 25 nucleotides.

The carrier molecules used in the methods and composition described herein can also contain a carbohydrate or polyol, including a polysaccharide, such as dextran, FICOLL, heparin, glycogen, amylopectin, mannan, inulin, starch, agarose and cellulose, or a polymer such as a poly(ethylene glycol). In certain variants, the polysaccharide carrier molecule includes dextran, agarose or FICOLL.

The carrier molecules used in the methods and composition described herein can also include a lipid including, but not limited to, glycolipids, phospholipids, and sphingolipids. In certain varinats, such lipids contain 6-25 carbons. In other embodiments, the carrier molecules include a lipid vesicle, such as a liposome, or is a lipoprotein (see below). Some lipophilic substituents are useful for facilitating transport of the conjugated dye into cells or cellular organelles. In certain variants, the carrier molecule that possess a lipophilic substituent can be used to target lipid assemblies such as biological membranes or liposomes by non-covalent incorporation of the dye compound within the membrane, e.g., for use as probes for membrane structure or for incorporation in liposomes, lipoproteins, films, plastics, lipophilic microspheres or similar materials.

The carrier molecules used in the methods and composition described herein can also be a cell, cellular systems, cellular fragment, or subcellular particles, including virus particles, bacterial particles, virus components, biological cells (such as animal cells, plant cells, bacteria, or yeast), or cellular components. Non-limiting examples of such cellular components that are useful as carrier molecules in the methods and composition described herein include lysosomes, endosomes, cytoplasm, nuclei, histones, mitochondria, Golgi apparatus, endoplasmic reticulum and vacuoles.

The carrier molecules used in the methods and composition described herein can also non-covalently associate with organic or inorganic materials.

The carrier molecules used in the methods and composition described herein can also include a specific binding pair member wherein the proteins described herein can be conjugated to a specific binding pair member and used in the formation of a bound pair. In certain embodiments, the presence of a labeled specific binding pair member indicates the location of the complementary member of that specific binding pair; each specific binding pair member having an area on the surface or in a cavity which specifically binds to, and is complementary with, a particular spatial and polar organization of the other. In certain embodiments, the dye compounds (fluorophores or chromophores) described herein function as a reporter molecule for the specific binding pair. Exemplary binding pairs are set forth in Table 2.

**Table 2. Representative Specific Binding Pairs**

| antigen | antibody |
|---|---|
| biotin | avidin (or streptavidin or anti-biotin) |
| IgG* | protein A or protein G |
| drug | drug receptor |
| folate | folate binding protein |
| toxin | toxin receptor |
| carbohydrate | lectin or carbohydrate receptor |
| peptide | peptide receptor |
| protein | protein receptor |
| enzyme substrate | enzyme |
| DNA (RNA) | cDNA (cRNA)† |
| hormone | hormone receptor |
| ion | chelator |

| | |
|---|---|
| * IgG is an immunoglobulin ^{†} cDNA and cRNA are the complementary strands used for hybridization | |

In a particular varinat the carrier molecule, used in the methods and compositions described herein, is an antibody fragment, such as, but not limited to, anti-Fc, an anti-Fc isotype, anti-J chain, anti-kappa light chain, anti-lambda light chain, or a single-chain fragment variable protein; or a non-antibody peptide or protein, such as, for example but not limited to, soluble Fc receptor, protein G, protein A, protein L, lectins, or a fragment thereof. In one variant the carrier molecule is a Fab fragment specific to the Fc portion of the target-binding antibody or to an isotype of the Fc portion of the target-binding antibody (US Serial No. 10/118,204). The monovalent Fab fragments are typically produced from either murine monoclonal antibodies or polyclonal antibodies generated in a variety of animals, for example but not limited to, rabbit or goat. These fragments can be generated from any isotype such as murine IgM, IgG₁, IgG₂ₐ, IgG_{2b} or IgC₃.

In alternative variants, a non-antibody protein or peptide such as protein G, or other suitable proteins, can be used alone or coupled with albumin. Preferred albumins include human and bovine serum albumins or ovalbumin. Protein A, G and L are defined to include those proteins known to one skilled in the art or derivatives thereof that comprise at least one binding domain for IgG, i.e. proteins that have affinity for IgG. These proteins can be modified but do not need to be and are conjugated to a reactive moiety in the same manner as the other carrier molecules described.

In another variant, the carrier molecules, used in the methods and compositions described herein, can be whole intact antibodies. Antibody is a term of the art denoting the soluble substance or molecule secreted or produced by an animal in response to an antigen, and which has the particular property of combining specifically with the antigen that induced its formation. Antibodies themselves also serve are antigens or immunogens because they are glycoproteins and therefore are used to generate anti-species antibodies. Antibodies, also known as immunoglobulins, are classified into five distinct classes--IgG, IgA, IgM, IgD, and IgE. The basic IgG immunoglobulin structure consists of two identical light polypeptide chains and two identical heavy polypeptide chains (linked together by disulfide bonds).

When IgG is treated with the enzyme papain a monovalent antigen-binding fragment can be isolated, referred herein to as a Fab fragment. When IgG is treated with pepsin (another proteolytic enzyme), a larger fragment is produced, F(ab')₂. This fragment can be split in half by treating with a mild reducing buffer that results in the monovalent Fab' fragment. The Fab' fragment is slightly larger than the Fab and contains one or more free sulfhydryls from the hinge region (which are not found in the smaller Fab fragment). The term "antibody fragment" is used herein to define the Fab', F(ab')₂ and Fab portions of the antibody. It is well known in the art to treat antibody molecules with pepsin and papain in order to produce antibody fragments (Gorevic et al., Methods of Enzyol., 116:3 (1985)).

The monovalent Fab fragments used as carrier molecules in the methods and compositions described herein are produced from either murine monoclonal antibodies or polyclonal antibodies generated in a variety of animals that have been immunized with a foreign antibody or fragment thereof (U.S. Patent No. 4,196,265 discloses a method of producing monoclonal antibodies). Typically, secondary antibodies are derived from a polyclonal antibody that has been produced in a rabbit or goat but any animal known to one skilled in the art to produce polyclonal antibodies can be used to generate anti-species antibodies. The term "primary antibody" describes an antibody that binds directly to the antigen as opposed to a "secondary antibody" that binds to a region of the primary antibody. Monoclonal antibodies are equal, and in some cases, preferred over polyclonal antibodies provided that the ligand-binding antibody is compatible with the monoclonal antibodies that are typically produced from murine hybridoma cell lines using methods well known to one skilled in the art.

In one variant the antibodies used as carrier molecules in the methods and compositions described herein are generated against only the Fc region of a foreign antibody. Essentially, the animal is immunized with only the Fc region fragment of a foreign antibody, such as murine. The polyclonal antibodies are collected from subsequent bleeds, digested with an enzyme, pepsin or papain, to produce monovalent fragments. The fragments are then affinity purified on a column comprising whole immunoglobulin protein that the animal was immunized against or just the Fc fragments.

### Solid Supports: Azide reactive, Alkyne reactive or Phosphine reactive

In the methods and composition described herein, the modified biomolecules can be covalently conjugated to a solid support.

In certain variants provided herein modified biomolecules are covalently conjugated to a solid support. This includes, but is not limited to, any azide modified, alkyne modified, activated alkyne modified, and any phosphine modified biomolecule disclosed herein and any solid support disclosed herein.

In certain embodiments, the modified biomolecules are modified proteins that comprise at least one azido group and are capable of reacting with a solid support comprising at least one alkyne group, including, but not limited to, a terminal alkyne group; at least one activated alkyne group, including, but not limited to, a cyclooctyne group; or at least one phosphine group, including, but not limited to, a triarylphosphine group. In some embodiments, the terminal group is -C≡CH.

In certain embodiments, the modified biomolecule are modified proteins that comprise at least one alkyne group, including, but not limited to, a terminal alkyne group; at least one activated alkyne group, including, but not limited to, a cyclooctyne group; or at least one phosphine group, including, but not limited, to a triarylphosphine group; and are capable of reacting with a solid support comprising at least azido group. In some embodiments, the terminal alkyne group is -C≡CH.

A variety of solid supports can be used in the methods and compositions described herein. Such solid supports are not limited to a specific type of support, and therefore a large number of supports are available and are known to one of ordinary skill in the art. Such solid supports include, but are not limited to, solid and semi-solid matrixes, such as aerogels and hydrogels, resins, beads, biochips (including thin film coated biochips), microfluidic chip, a silicon chip, multi-well plates (also referred to as microtitre plates or microplates), membranes, conducting and nonconducting metals, glass (including microscope slides) and magnetic supports. Other non-limiting examples of solid supports used in the methods and compositions described herein include silica gels, polymeric membranes, particles, derivatized plastic films, derivatized glass, derivatized silica, glass beads, cotton, plastic beads, alumina gels, polysaccharides such as Sepharose, poly(acrylate), polystyrene, poly(acrylamide), polyol, agarose, agar, cellulose, dextran, starch, FICOLL, heparin, glycogen, amylopectin, mannan, inulin, nitrocellulose, diazocellulose, polyvinylchloride, polypropylene, polyethylene (including poly(ethylene glycol)), nylon, latex bead, magnetic bead, paramagnetic bead, superparamagnetic bead, starch and the like. In certain variants, the solid supports used in the methods and compositions described herein are substantially insoluble in liquid phases.

In certain embodiments, the solid support may include a solid support reactive functional group, including, but not limited to, hydroxyl, carboxyl, amino, thiol, aldehyde, halogen, nitro, cyano, amido, urea, carbonate, carbamate, isocyanate, sulfone, sulfonate, sulfonamide, sulfoxide, wherein such functional groups are used to covalently attach the azide-containing glycoproteins described herein. In other embodiments, the solid support may include a solid support reactive functional group, including, but not limited to, hydroxyl, carboxyl, amino, thiol, aldehyde, halogen, nitro, cyano, amido, urea, carbonate, carbamate, isocyanate, sulfone, sulfonate, sulfonamide, sulfoxide, wherein such functional groups are used to covalently attach the alkyne-containing glycoproteins described herein. In still other embodiments, the solid support may include a solid support reactive functional group, including, but not limited to, hydroxyl, carboxyl, amino, thiol, aldehyde, halogen, nitro, cyano, amido, urea, carbonate, carbamate, isocyanate, sulfone, sulfonate, sulfonamide, sulfoxide, wherein such functional groups are used to covalently attach the phosphine-containing glycoproteins described herein. In other embodiments, the solid supports include azide, alkyne or phosphine functional groups to covalently attach such modified glycoproteins.

A suitable solid phase support used in the methods and compositions described herein, can be selected on the basis of desired end use and suitability for various synthetic protocols. By way of example only, where amide bond formation is desirable to attach the modified glycoproteins described herein to the solid support, resins generally useful in peptide synthesis may be employed, such as polystyrene (*e**.**g*., PAM-resin obtained from Bachem Inc., Peninsula Laboratories, etc.), POLYHIPE™ resin (obtained from Aminotech, Canada), polyamide resin (obtained from Peninsula Laboratories), polystyrene resin grafted with polyethylene glycol (TentaGel™, Rapp Polymere, Tubingen, Germany), polydimethyl-acrylamide resin (available from Milligen/Biosearch, California), or PEGA beads (obtained from Polymer Laboratories). In certain embodiments, the modified glycoprotiens described herein are deposited onto a solid support in an array format. In certain embodiments, such deposition is accomplished by direct surface contact between the support surface and a delivery mechanism, such as a pin or a capillary, or by inkjet technologies which utilize piezoelectric and other forms of propulsion to transfer liquids from miniature nozzles to solid surfaces. In the case of contact printing, robotic control systems and multiplexed printheads allow automated microarray fabrication. For contactless deposition by piezoelectric propulsion technologies, robotic systems also allow for automatic microarray fabrication using either continuous and drop-on-demand devices.

The disclosure also provides a method of covalently conjugating a modified biomolecule comprising at least one an alkyne reactive moeity to a solid support, wherein the method comprises the steps of:
a) contacting the modified biomolecule with a solid support comprising at least one azide reactive moiety to form a contacted modified biomolecule; and
b) incubating the contacted modified biomolecule for a sufficient amount of time to form a biomolecule-solid support conjugate.
is the disclosure also provides a method of covalently conjugating a modified biomolecule comprising at least one azide reactive moiety to a solid support, wherein the method comprises the steps of:
a) contacting the modified biomolecule with a solid support comprising at least one alkyne reactive moiety to form a contacted modified biomolecule; and
b) incubating the contacted modified biomolecule for a sufficient amount of time to form a biomolecule-solid support conjugate.

### Compositions

The modified biomolecules, reporter molecules and carrier molecules provided herein can be used to form a first composition that includes a modified biomolecule, a first reporter molecule, and a carrier molecule. In another variant, a second modified biomolecule that includes a first composition in combination with a second conjugate, wherein the second conjugate comprises a carrier molecule or solid support that is covalently bonded to a second reporter molecule. The first and second reporter molecules have different structures and preferably have different emission spectra. In other variants, the first and second reporter molecules are selected so that their fluorescence emissions essentially do not overlap. In other variants, the reporter molecules have different excitation spectra, while in other variants the reporter molecules have similar excitation wavelengths and are excited by the same laser. In such compositions, the carrier molecule (or solid support) of the conjugates in the second composition may be the same or a different molecule. The discussion herein pertaining to the identity of various carrier molecules is generally applicable to this variant as well as other variants.

In certain variants, modified biomolecules, reporter molecules and carrier molecules provided herein can be used to form a first composition that includes a modified biomolecule, a first reporter molecule, and a carrier molecule. In another variant, a second modified biomolecule that includes a first composition in combination with a second conjugate, wherein the second conjugate comprises a carrier molecule or solid support that is covalently bonded to a second reporter molecule. The first and second reporter molecules have different structures and preferably have different emission spectra. In other variants, the first and second reporter molecules are selected so that their fluorescence emissions essentially do not overlap. In other variants, the reporter molecules have different excitation spectra, while in other embodiments the reporter molecules have similar excitation wavelengths and are excited by the same laser. In such compositions, the carrier molecule (or solid support) of the conjugates in the second composition may be the same or a different molecule. The discussion herein pertaining to the identity of various carrier molecules is generally applicable to this variant as well as other variants.

In accordance with the disclosure, the modified biomolecules, reporter molecules and solid supports provided herein can be used to form a first composition that comprises a modified biomolecule, a first reporter molecule, and a solid support. In another variant, a second composition that includes a first composition in combination with a second conjugate. The second conjugate comprises a solid support or carrier molecule (described herein) that is covalently bonded to a second reporter molecule. The first and second reporter molecules have different structures and preferably have different emission spectra. In other variants, the first and second reporter molecules are selected so that their fluorescence emissions essentially do not overlap. In other variants, the reporter molecules have different excitation spectra, while in other variants the reporter molecules have similar excitation wavelengths and are excited by the same laser. In such composition, the solid support (or carrier molecule) of the conjugates in the second composition may be the same or a different molecule. The discussion herein pertaining to the identity of various solid supports is generally applicable to this variant as well as other variants.

In another aspect, the modified proteins, reporter molecules and solid supports provided herein can be used to form a first composition that comprises a modified protein, a first reporter molecule, and a solid support. In another embodiment, a second composition that includes a first composition in combination with a second conjugate. The second conjugate comprises a solid support or carrier molecule (described herein) that is covalently bonded to a second reporter molecule. The first and second reporter molecules have different structures and preferably have different emission spectra. In other embodiments, the first and second reporter molecules are selected so that their fluorescence emissions essentially do not overlap. In other embodiments, the reporter molecules have different excitation spectra, while in other embodiments the reporter molecules have similar excitation wavelengths and are excited by the same laser. In such composition, the solid support (or carrier molecule) of the conjugates in the second composition may be the same or a different molecule. The discussion herein pertaining to the identity of various solid supports is generally applicable to this embodiment of the invention as well as other embodiments.

### Methods for labeling modified biomolecules in a cell or in solution.

The disclosure provides methods for labeling in a cell the modified biomolecules with a reporter molecule to provide a biomolecule-reporter molecule conjugates. If desired, the biomolecule-reporter molecule conjugates which are formed in a cell may then separated from the cell using methods known in the art.

In accordance with the invention, the modified biomolecule to be labeled (and detected) is a modified protein.

In certain variants, the method of labeling (and detecting in a cell) a modified biomolecule generated by in response to oxidative cellular conditions, comprises the steps of contacting a cell in an aqueous solution with a polyunsaturated fatty acid analog of the present invention; contacting the cell in an aqueous solution with a reporter molecule comprising a chemical handle capable of reacting with the alkyne reactive group or azide reactive moiety of the compound; and detecting the presence of the modified biomolecule in the cell. The resulting biomolecule-reporter molecule conjugates are detected by methods known in the art and as described herein.

In certain variants, the modified biomolecule comprises an alkyne reactive group, and the reporter molecule comprises a chemical handle which is an azide reactive group, while in other embodiments, the modified biomolecule comprises an azide reactive group, and the reporter molecule comprises a chemical handle which is an alkyne reactive group. In some variants, the alkyne reactive group is an azido group. In some variants, the azide reactive group is an alkyne group, cyclooctyne group, or phosphine group. In some variants, the alkyne group will be a terminal alkyne group, while in other embodiments, terminal alkyne group will be -C=CH. In some variants, the phosphine group will be a triarylphosphine group.

The disclosure also provides methods for labeling (and detecting in a cell) the modified biomolecules of the present invention using two reporter molecules to provide biomolecule-reporter molecule conjugates. If desired, the biomolecule-reporter molecule conjugates which are formed in a cell may then separated from the cell using methods known in the art.

In certain variants, the method of detecting in a cell modified biomolecule generated in response to oxidative cellular conditions is done by labeling them using two reporter molecules.

In some variants, the method comprises the steps of contacting a cell in an aqueous solution with a first and second polyunsaturated fatty acid analog of the present invention, where the first compound comprises an alkyne reactive moiety and the second compound comprises an azide reactive moiety; contacting the cell with a first reporter molecule comprising a chemical handle capable of reacting with the alkyne reactive moiety; contacting the cell with a second reporter molecule comprising a chemical handle capable of reacting with the azide reactive moiety; and detecting the presence of the modified biomolecules.

In some variants, the method comprises the steps of contacting a cell in an aqueous solution with a first and second polyunsaturated fatty acid analog of the present invention, where the first compound comprises an azide reactive moiety and the second compound comprises an alkyne reactive moiety; contacting the cell with a first reporter molecule comprising a chemical handle capable of reacting with the azide reactive moiety; contacting the cell with a second reporter molecule comprising a chemical handle capable of reacting with the alkyne reactive moiety; and detecting the presence of the modified biomolecules.

The disclosure also provides a method for labeling in solution the modified biomolecules using a reporter molecule, carrier molecule or solid phase. The biomolecule-reporter molecule, biomolecule-carrier biomolecule or biomolecule-solid phase conjugate is formed in solution and then are separated using methods known in the art.

In certain embodiments, a method of labeling in solution a modified protein generated in response to oxidative cellular conditions, comprising the steps of contacting a cell in an aqueous solution with a polyunsaturated fatty acid analog of the present invention; preparing an isolate of the cell; contacting the isolate with a reporter molecule, carrier molecule or solid phase comprising a chemical handle capable of reacting with the alkyne reactive group or azide reactive moiety of the compound to give the labeled modified protein.

In certain variants, the modified biomolecule comprises an alkyne reactive group, and the reporter molecule, carrier molecule, or solid phase comprises a chemical handle which is an azide reactive group, while in other variants, the modified biomolecule comprises an azide reactive group; and the reporter molecule, carrier molecule, or solid phase comprises a chemical handle which is an alkyne reactive group. In some embodiments, the alkyne reactive group is an azido group. In some variants, the azide reactive group is an alkyne group, cyclooctyne group, or phosphine group. In some variants, the alkyne group will be a terminal alkyne group, while in other embodiments, the terminal alkyne group will be -C≡CH. In some variants, the phosphine group will be a triarylphosphine group.

In another variant, the disclosure provides methods for labeling (and detecting in solution) the modified biomolecules using two reporter molecules to provide biomolecule-reporter molecule conjugates. The biomolecule-reporter molecule conjugates are formed in solution and then are separated using methods known in the art.

In accordance with the invention, the modified biomolecules are modified proteins.

In certain variants, the method of detecting in solution modified biomolecule generated in response to oxidative cellular conditions is done by labeling the modified biomolecule with two reporter molecules.

In some embodiments, the method comprises the steps of (a) contacting a cell in an aqueous solution with a polyunsaturated fatty acid analog of the present invention, where the first compound comprises an alkyne reactive moiety and the second compound comprises an azide reactive moiety; (b) preparing an isolate of the cell; (c) contacting the isolate with a first reporter molecule comprising a chemical handle capable of reacting with the alkyne reactive moiety; (d) contacting the isolate from step (c) with a second reporter molecule comprising a chemical handle capable of reacting with the azide reactive moiety; and (e) detecting the presence of the modified proteins.

In some embodiments, the method comprises the steps of (a) contacting a cell in an aqueous solution with a polyunsaturated fatty acid analog of the present invention, where the first compound comprises an azide reactive moiety and the second compound comprises an alkyne reactive moiety; (b) preparing an isolate of the cell; (c) contacting the isolate with a first reporter molecule comprising a chemical handle capable of reacting with the azide reactive moiety; (d) contacting the isolate from step (c) with a second reporter molecule comprising a chemical handle capable of reacting with the alkyne reactive moiety; and (e) detecting the presence of the modified proteins.

Described herein are novel methods for forming conjugates in solution with biomolecules comprising an azido group and a reporter molecule comprising a terminal alkyne under "click" chemistry conditions. In other variants, "click" chemistry is used to form conjugates with a biomolecules comprising a terminal alkyne group and a reporter molecule comprising an azido group.

Also, described herein are novel methods for forming conjugates in solution with biomolecules comprising an azido group and a reporter molecule comprising an activated alkyne group under cycloaddition chemistry conditions. In other variants, cycloaddition chemistry is used to form conjugates with biomolecules comprising an activated alkyne group and a reporter molecule comprising an azido group.

Also, described herein are novel methods for forming conjugates in solution with biomolecules comprising an azido group and a reporter molecule comprising a triaryl phosphine under Staudinger ligation chemistry conditions. In other variants, Staudinger ligation chemistry is used to form conjugates with biomolecules comprising a triarylphosphine group and a reporter molecule comprising an azido group.

The adding of a copper chelator to the "click" chemistry conjugation reaction improves the labeling efficiency and resolution after gel electrophoresis as compared to those reactions without the addition of a copper chelator. In certain variants, the methods of labeling modified biomolecules using "click" chemistry, involve a biomolecule that includes an azido group and a label that includes a terminal alkyne that are reacted in a mixture that includes copper (II), a reducing agent, and at least one copper (I) chelator, thereby producing a labeled biomolecule.

In accordance with the invention, the modified biomolecules used in the labeling methods described herein are modified proteins. The labeling methods used to label modified proteins, include, but are not limited to, "click" chemistry, cycloaddition, or Staudinger ligation.

In certain variants, the labeling of biomolecule occurs by "click" chemistry in which a protein that includes an azido group and a label that comprises a terminal alkyne react in a mixture that includes copper (II), a reducing agent, and at least one copper chelator to produce a labeled biomolecule. In certain variants, the labeling of modified biomolecules occurs by "click" chemistry in which the biomolecule that comprises a terminal alkyne group and a label that comprises an azido group react in a mixture that includes copper (II), a reducing agent, and at least one copper chelator to produce a labeled modified biomolecule.

In other methods of the disclosure provided herein, the methods of labeling modified biomolecules using "click" chemistry, wherein a modified biomolecule comprises an azido group and a label that comprises a terminal alkyne are reacted in a mixture that includes copper (II), a reducing agent, and at least one copper (I) chelator to produce a labeled biomolecule, results in the preservation of the structural integrity of the labeled biomolecule. In the methods of the invention, the modified biomolecule labeled in this way is a modified protein. In such methods, a modified protein that comprises an azido group and a label that comprises a terminal alkyne are reacted in a mixture that includes copper (II), a reducing agent, and at least one copper (I) chelator to produce a labeled modified protein, and results in the preservation of the structural integrity of the labeled protein, wherein the structural integrity of the protein after labeling is not reduced. As described herein, the proteins can be modified, for example, in a cell by lipid peroxidation. In other embodiments, methods of labeling proteins wherein the structural integrity of the protein after labeling is not reduced includes "click" chemistry in which a modified protein that compries a terminal alkyne and a label that comprises an azido group are reacted in a mixture that includes copper (II), a reducing agent, and at least one copper chelator to produce a labeled modified protein.

The methods for labeling modified biomolecules that comprise an azido group using "click" chemistry described herein can also be used for modified biomolecules that comprise a terminal alkyne, wherein the label to be reacted with the modified biomolecule comprises an azido group.

The methods for labeling and detecting biomolecules that comprise an azido group using "click" chemistry described herein can also be used for biomolecules that comprise a terminal alkyne, wherein the label, to be reacted with the biomolecule, comprises an azido group. In one variant, a method using the "click" chemistry reaction described herein to form biomolecule-reporter molecule conjugates in which the reaction mixture includes a reporter molecule comprising an azido group, a modified biomolecule comprising a terminal alkyne group, copper (II) ions, at least one reducing agent and a copper chelator. In certain embodiments of the invention, such modified biomolecules comprising a terminal alkyne are modified proteins and such reporter molecule comprising an azido group is any reporter molecule described herein. In other embodiments, such modified biomolecules are modified proteins comprising a terminal alkyne group and such reporter molecule comprises an azido group is any fluorophore based reporter molecule described herein.

Other methods provided herein, are methods for labeling and detecting separated modified biomolecule using the "click" chemistry reaction described herein. The method includes: combining in a reaction mixture a biomolecule that comprises an azido group, a label that comprise a terminal alkyne group, copper (II), a reducing agent, and a copper chelator; incubating the reaction mixture under conditions that promote chemical conjugation of the label to the biomolecule, separating the modified biomolecule using one or more biochemical or biophysical separation techniques, and detecting the modified biomolecule. In other variants, the method includes: combining in a reaction mixture a biomolecule that comprises an alkyne group, a label that includes an azide group, copper (II), a reducing agent, and a copper chelator; incubating the reaction mixture under conditions that promote chemical conjugation of the label to the biomolecule, separating the modified biomolecule using one or more biochemical or biophysical separation techniques, and detecting the modified biomolecule.

The disclosure also provides a method for detecting modified biomolecules, wherein the method comprises the steps of:
a) forming a reaction mixture comprising a modified biomolecule comprising an azido group, a reporter molecule comprising a terminal alkyne group, copper(II) ions, at least one reducing agent, and a copper chelator;
b) incubating the reaction mixture for a sufficient amount of time to form a biomolecule-reporter molecule conjugate;
c) separating the biomolecule-reporter molecule conjugate by size and/or weight of the biomolecule-reporter molecule conjugate to form a separated biomolecule-reporter molecule conjugate;
d) illuminating the separated biomolecule-reporter molecule conjugate with an appropriate wavelength to form an illuminated biomolecule-reporter molecule conjugate, and
e) observing the illuminated biomolecule-reporter molecule conjugate wherein the biomolecules is detected.

In another variant the disclosure provides a method for detecting modified biomolecules, wherein the method comprises the steps of:
a) forming a reaction mixture comprising a modified biomolecule comprising a terminal alkyne group and a reporter molecule comprising an azido group, copper(II) ions, at least one reducing agent and a copper chelator;
b) incubating the reaction mixture for a sufficient amount of time to form a biomolecule-reporter molecule conjugate;
c) separating the biomolecule-reporter molecule conjugate by size and/or weight of the biomolecule-reporter molecule conjugate to form a separated protein-reporter molecule conjugate;
d) illuminating the separated biomolecule-reporter molecule conjugate with an appropriate wavelength to form an illuminated biomolecule-reporter molecule conjugate, and
e) observing the illuminated biomolecule-reporter molecule conjugate wherein the protein is detected.

In addition such "click" chemistry reaction mixtures can include, without limitation, one or more buffers, polymers, salts, detergents, or solubilizing agents. The reaction can be performed under anaerobic conditions, such as under nitrogen or argon gas, and can be performed for any feasible length of time, such as, for example, from ten minutes to six hours, from about twenty minutes to about three hours, or from about thirty minutes to about two hours. The reaction can be performed at a wide range of temperatures, for example ranging from about 4 degrees Celsius to about 50 degrees Celsius, and is preferably performed at temperatures between about 10 degrees Celsius and about 40 degrees Celsius, and typically between about 15 degrees Celsius and about 30 degrees Celsius.

### Separation and Detection

The disclosure alos provides herein methods directed to detecting modified biomolecules after the modified biomolecules have been labeled, using "click" chemistry reactions, activated alkyne reactions (i.e, cycloadditions), or Saudinger ligation, and separated using, for example, chromatographic methods or electrophoresis methods such as, but not limited to, gel electrophoresis. The modified biomolecules that can be labeled, separated and detected using the methods described herein include, but are not limited to, proteins. In certain variants, such biomolecules have been modified using the methods described herein. The separation methods used to separate such modified biomolecules includes, but are not limited to, thin layer or column chromatography (including, for example, size exclusion, ion exchange, or affinity chromatography) or isoelectric focusing, gel electrophoresis, capillary electrophoresis, capillary gel electrophoresis, and slab gel electrophoresis. Gel electrophoresis can be denaturing or nondenaturing gel electrophoresis, and can include denaturing gel electrophoresis followed by nondenaturing gel electrophoresis (e.g., "2D" gels). In certain variants, the modified biomolecules are used to form conjugates with a reporter molecule, a carrier molecule and/or a solid support prior to separation using the methods described herein. In other variants, the modified biomolecules are used to form conjugates with a reporter molecule, a carrier molecule and/or a solid support after separation using the methods described herein.

In other variants, the separation methods used in such separation and detection methods can be any separation methods used for biomolecules, such as, for example, chromatography, capture to solid supports, and electrophoresis. In certain variants, gel electrophoresis is used to separate biomolecules, such as but not limited to proteins. Gel electrophoresis is well known in the art, and in the context of the present invention can be denaturing or nondenaturing gel electrophoresis and can be 1D or 2D gel electrophoresis.

In certain embodiments of such separation and detection methods, gel electrophoresis is used to separate proteins and the separated proteins are detected in the gel by the attached labels. By way of example only, proteins that have incorporated azido sugars can be labeled in a solution reaction with a terminal alkyne-containing fluorophore, and the proteins can be optionally further purified from the reaction mixture and electrophoresed on a 1D or 2D gel. The proteins can be visualized in the gel using light of the appropriate wavelength to stimulate the fluorophore label.

Gel electrophoresis can use any feasible buffer system described herein including, but not limited to, Tris-acetate, Tris-borate, Tris-glycine, BisTris and Bistris-Tricine. In certain variants, the electrophoresis gel used in the methods described herein comprise acrylamide, including by way for example only, acrylamide at a concentration from about 2.5% to about 30%, or from about 5% to about 20%. In certain variants, such polyacrylamide electrophoresis gel comprise 1% to 10% crosslinker, including but not limited to, bisacrylamide. In certain variants, the electrophoresis gel used in the methods described herein comprises agarose, including by way for example only, agarose at concentration from about 0.1% to about 5%, or from about 0.5% to about 4%, or from about 1% to about 3%. In certain variants, the electrophoresis gel used in the methods described herein comprises acrylamide and agarose, including by way for example only, electrophoresis gels comprising from about 2.5% to about 30% acrylamide and from about 0.1 % to about 5% aga_rose, or from about 5% to about 20% acrylamide and from about 0.2% to about 2.5% agarose. In certain variants, such polyacrylamide/agarose, electrophoresis gel comprise 1% to 10% crosslinker, including but not limited to, bisacrylamide. In certain variants, the gels used to separate biomolecules can be gradient gels.

The methods described herein can be used to detect modified biomolecules for "in-gel" detection using slab gel electrophoresis or capillary gel electrophoresis. In the methods of the invention such modified biomolecules are proteins. In one method of the disclosure, the method includes combining a modified biomolecule comprising an azido group, a label (e.g., a reporter molecule) comprising a terminal alkyne group, copper (II), a reducing agent, and a copper (I) chelator in a reaction mixture; incubating the reaction mixture under conditions that promote chemical conjugation of the label to the biomolecule; separating the biomolecule using one or more biochemical separation techniques; and detecting the biomolecule. The label used in such methods can be any label described herein. The copper (I) chelator used in such methods can be any chelator described herein. In certain variants, the copper (I) chelator use in such methods is a 1,10 phenanthroline-containing copper (I) chelator. In other variants, the copper(I) chelator is bathocuproine disulfonic acid (BCS; 2,9-dimethyl-4,7-diphenyl-1,10-phenanthroline disulfonate. In other variants, the copper(I) chelator is TBTA or THPTA as described in Jentzsch et al., Inorganic Chemistry, 48(2): 9593-9595 (2009). In other variants, the copper(I) chelator are those described in Finn et al., U.S. Patent Publication No. US2010/0197871. In other variants, the copper (I) chelator used in such methods can be used to chelate copper(II).

Without limitation to any specific mechanism, it is known that copper can promote the cleavage of biomolecules such as proteins and nucleic acids. The addition of a copper chelator in such methods reduces the detrimental effects of copper used in the "click" chemistry reactions, and thereby preserves the structural integrity of the biomolecules. Thus, the methods described herein preserve the structural integrity of labeled and detected modified biomolecules, and thereby provide improved methods of separating and detecting modified biomolecules labeled using "click" chemistry. In addition, the methods of detecting separated modified biomolecules using click chemistry, in which the structural integrity of the separated molecules is preserved, improves the detection of such biomolecules.

In another variant of "in-gel" detection, the method includes combining an modified biomolecule comprising a terminal alkyne group, a label comprising an azido group, copper (II), a reducing agent, and a copper (I) chelator in a reaction mixture; incubating the reaction mixture under conditions that promote chemical conjugation of the label to the biomolecule; separating the labeled biomolecule using one or more biochemical separation techniques; and detecting the biomolecule.

In these methods, the structural integrity of labeled and detected biomolecules is preserved. The label used in such methods can be any label described herein. The copper (I) chelator used in such methods can be any chelator described herein. In certain variants, the copper (I) chelator use in such methods is a 1,10 phenanthroline-containing copper (I) chelator. In other variants, the copper(I) chelator is bathocuproine disulfonic acid (BCS; 2,9-dimethyl-4,7-diphenyl-1,10-phenanthroline disulfonate. In other variants, the copper(I) chelator is TBTA or THPTA as described in Jentzsch et al., Inorganic Chemistry, 48(2): 9593-9595 (2009). In other embodiments, the copper(I) chelator are those described in Finn et al., U.S. Patent Publication No. US2010/0197871. In other variants, the copper (I) chelator used in such methods can be used to chelate copper(II).

In-gel fluorescence detection allows for quantitative differential analysis of protein glycosylation between different biological samples and is amenable to multiplexing with other protein gel stains. In certain embodiments of the methods described herein, utilizing fluorescent- and/or UV-excitable alkyne containing probes, or fluorescent- and/or UV-excitable azide containing probes, allow for the multiplexed detection of glycoproteins, phosphoproteins, and total proteins in the same 1-D or 2-D gels.

In certain variants, the labels used in such separation and detection methods are any fluorophores described herein which has been derivatized to contain an alkyne, an azide or a phosphine. In certain variants, such fluorphores include, but are not limited to, fluorescein, rhodamine, TAMRA, an Alexa dye, a SYPRO dye, or a BODIPY dye.

The method described herein can be used for multiplexed detection of modified biomolecules, such as proteins by labeling the proteins with labels of different specificities. For example, a total proteins stain, such as SYPRO Ruby can be used to stain a gel that includes proteins labeled using a fluorophore with distinct spectral emission using the methods of the present invention. Proteins having other characteristics, such as oxidized proteins or phosphorylated proteins, can be detected in the same gel by use of phosphoprotein specific labels used to stain the gel.

In accordance with the disclosure, proteins can be labeled to comprise an azido group, electrophoresed on gels, and the resulting gels can be incubated with an reporter molecule comprising a terminal alkyne group, such as a fluorescent alkyne tag in the presence of copper (I). Copper (I) can be added in its natural form (e.g. CuBr) or can be produced *in situ* from copper (II) compounds with the addition of a reducing agent. The reducing agent usd in such methods can be any reducing agent described herein, including but not limited to, ascorbate or TCEP. Addition of a chelator that stabilizes copper (I) can enhance the chemical ligation. The fluorescent label used in such methods can be any fluorophore described herein. The copper (I) chelator used in such methods can be any chelator described herein. In certain variants, the copper (I) chelator use in such methods is a 1,10 phenanthroline-containing copper (I) chelator. In other variants, the copper(I) chelator is bathocuproine disulfonic acid (BCS; 2,9-dimethyl-4,7-diphenyl-1,10-phenanthroline disulfonate. In other variants, the copper (I) chelator used in such methods can be used to chelate copper(II). After the ligation step, the gel is washed and the tagged proteins are visualized using standard fluorescence scanning devices. In embodiments, proteins can be labeled to comprise a terminal alkyne group, electrophoresed on gels, and the resulting gels can be incubated with a reporter molecule comprising an azido group, such as a fluorescent azide tag in the presence of copper (I). Copper (I) can be added in its natural form (e.g. CuBr) or can be produced *in situ* from copper (II) compounds with the addition of a reducing agent. The reducing agent used in such methods can be any reducing agent described herein, including but not limited to, ascorbate or TCEP. Addition of a chelator that stabilizes copper (I) can enhance the chemical ligation. The fluorescent label used in such methods can be any fluorophore described herein. The copper (I) chelator used in such methods can be any chelator described herein. In certain embodiments, the copper (I) chelator use in such methods is a 1,10 phenanthroline-containing copper (I) chelator. In other embodiments, the copper(I) chelator is bathocuproine disulfonic acid (BCS; 2,9-dimethyl-4,7-diphenyl-1,10-phenanthroline disulfonate. In other embodiments, the copper(I) chelator is TBTA or THPTA as described in Jentzsch et al., Inorganic Chemistry, 48(2): 9593-9595 (2009). In other embodiments, the copper(I) chelator are those described in Finn et al., U.S. Patent Publication No. US2010/0197871.

In other embodiments, the copper (I) chelator used in such methods can be used to chelate copper(II). After the ligation step, the gel is washed and the tagged proteins are visualized using standard fluorescence scanning devices.

In further embodiments, proteins can be labeled to comprise an azido group, electrophoresed on gels, and the resulting gels can be incubated with a reporter molecule comprising an activated alkyne group (e.g., cyclooctyne), such as a fluorescent activated alkyne containing tag, using cycloaddition. After the cycloadditon step, the gel is washed and the tagged proteins are visualized using standard fluorescence scanning devices. In such methods the use of copper, which contributes to the degradation of biomolecules such as proteins, can also be avoided.

In further embodiments, proteins can be labeled to comprise an azido group, electrophoresed on gels, and the resulting gels can be incubated with a reporter molecule comprising a triarylphosphine, such as a fluorescent phosphine containing tag, using Staudinger ligation. After the ligation step, the gel is washed and the tagged proteins are visualized using standard fluorescence scanning devices. In such methods the use of copper, which contributes to the degradation of biomolecules such as proteins, can be avoided.

The detection of proteins labeled using the methods described herein can be by Western blot, in which biomolecules comprising an azido group are labeled with a detectable label prior to gel electrophoresis and transfered to a blotting membrane. Modified biomolecules comprising an azido group can be labeled, for example, with a biotin molecule comprising a terminal alkyne group, an activated alkyne group, or a triarylphosphine group; and after electrophoretic separation and transfer to a blotting membrane, can be detected using streptavidin linked to an enzyme that converts a chromogenic substrate. Those skilled in the art will appreciate that any feasible label that is directly detectable or indirectly detectable and can be derivatized to comprise a terminal alkyne, activated alkyne, triarylphosphine, or azido group can be attached to a biomolecule comprising an azido group, a terminal alkyne, activated alkyne, or triarylphosphine group, and used to detect separated biomolecules, including separated biomolecules transferred to a membrane.

In other embodiments, Western blotting analyses reveal protein detection sensitivities in the low femtomole range and allow for multiplexing with protein-specific antibodies. In certain embodiments, biotin-alkyne probes, or biotin-azide probes, allow for multiplexed Western blot detection of proteins and targeted proteins of interest using monoclonal or polyclonal antibodies. The results achieved with the combined protein detection strategy described herein, provide excellent selectivity and sensitivity.

The methods described herein utilizing copper catalyzed cycloaddition chemistry can result in highly sensitive detection of proteins modified with an azido group or a terminal alkyne group, as shown by 1-D and 2-D fluorescent gel sensitivities on gel electrophoresis and Western blots. In certain variants, the detection sensitivity is in the low picomole range, while in either variants the detection sensitivity is in the mid-to-low femtomole range.

In certain variants, a label attached to a modified biomolecule, such as a protein, using a "click" chemisty reaction with a copper (I) chelator as disclosed herein, can also be used for the separation of biomolecules. By way of example only, affinity chromatography or bead capture techniques can be used to separate biomolecules labeled with biotin or other affinity tags using the methods described herein. The captured molecules can be detected using the affinity tags or by other means, and/or further analyzed for structure or function.

Another aspect of "in gel" detection is the total detection of proteins in electrophoresis gels or Western blot membranes using a "universal click" chemistry in which phenylboronic acid-containing molecules are tethered via a linker to an azide moiety or an alkyne moiety. The phenylboronic acid associates with the cis-diol moieties on glycoproteins which is stable, except uner acidic conditions. Such labels can be used to modify glycoproteins after electrophoretic separation with either azide or alkyne moieties which can then be used to add a label via "click" chemistry, activated alkyne chemistry, or Staudinger ligation. The gel is then visualized to detect the labeled glycoproteins. In certain embodiments, glycoproteins of interest can be isolated by excising bands of interest after such labeling and treating the gel peices under acidic conditions to reverse the association of the phenylboronic acid with the cis-diol moieties on glycoprotiens, thereby releasing the glycoproteins. The released glycoproteins can then be identified using mass spectrometry.

### Methods for labeling immobilized modified biomolecules

The disclosure also provides a method for labeling modified biomolecules that have been immobilized on a solid support. Solid supports used in such methods have been described herein, and can be solid or semi-solid matrix. Such solid supports include, but are not limited to, glass, slides, arrays, silica particles, polymeric particles, microtiter plates and polymeric gels.

In certain variants, it is advantageous to first immobilize the modified biomolecules and then to subsequently form a biomolecule conjugate comprising the biomolecule and a reporter molecule, carrier molecule and the solid support, wherein the reporter molecule, carrier molecule or solid support comprise a reactive group used to form the conjugate. In this variant of the disclosure, the biomolecules are modified using the methods described herein.

In certain variants, the reactive groups on the reporter molecule, carrier molecule or solid support are alkyne reactive groups or azide reactive groups. In some variants, the alkyne reactive group is an azido group. In some variants, the azide reactive group is an alkyne, activated alkyne or phosphine group. In some variants, the alkyne group is a terminal alkyne group, while other embodiments, the terminal alkyne group is -C=CH. In some variants, the activated alkyne group is a cyclooctyne group. In some variants, the phosphine group is a triaryl phosphine group.

In certain variants, the conjugate is formed under "click" chemistry conditions wherein the reporter molecule, carrier molecule or solid support comprises at least one terminal alkyne group or at least one azido group.

In certain variants, the conjugate is formed using activated alkynes wherein the reporter molecule, carrier molecule or solid support comprises an activated alkyne group, such as, for example, a cyclooctyne group; or an azido group.

In certain variants, the conjugate is formed under Staudinger ligation conditions wherein the reporter molecule, carrier molecule or solid support comprises phosphine group, such as, for example, a triarylphosphine; or an azido group.

In certain variants, it is advantageous to first immobilize a modified biomolecule comprising an azido group and then to subsequently form the biomolecule conjugate comprising a reporter molecule, carrier molecule or solid support, wherein the reporter molecule, carrier molecule or solid support comprise an azide reactive group, a terminal alkyne group, an activated alkyne group, or a triarylphosphine group. In certain variants, the conjugate is formed under "click" chemistry conditions wherein the reporter molecule, carrier molecule or solid support comprises a terminal alkyne group. In another variant of the disclosure, the conjugate is formed under cycloaddition chemistry conditions, wherein the reporter molecule, carrier molecule or solid support comprises an activated alkyne group, such as, for example, a cyclooctyne. In another variant of the disclosure, the conjugate is formed under Staudinger ligation conditions, wherein the reporter molecule, carrier molecule or solid support comprises a triarylphosphine group.

In another variant of the disclosure, the modified biomolecule is attached to a solid support using functional groups other than functional groups used in "click" chemistry, cycloaddition chemistry, or Staudinger ligation, whereupon the attached modified biomolecule is used to form a conjugate under "click" chemistry conditions, cycloaddition chemistry conditions, or Staudinger ligation conditions with reporter molecules, carrier molecule or another solid support that have functional groups used in "click" chemistry, cycloaddtion chemistry or Staudinger ligation, respectively. By way of example only, the modified biomolecule can be immobilized to a solid support using hydroxyl, carboxyl, amino, thiol, aldehyde, halogen, nitro, cyano, amido, urea, carbonate, carbamate, isocyanate, sulfone, sulfonate, sulfonamide or sulfoxide functional groups.

In this variant of the disclosure, the biomolecules are modified to comprise an alkyne reactive group or an azide reactive group.

In some embodiments, the modified biomolecule is a modified protein comprising an azide reactive group. In some embodiments, the modified protein comprises an azide reactive group which is an alkyne, an activated alkyne, or a phosphine group. In some embodiments, the modified protein comprises an alkyne group. In some embodiments, the modified protein comprises an alkyne group which is a terminal alkyne group. In some embodiments, the the modified protein comprises a terminal alkyne group which is -C≡CH. In some embodiments, the the modified protein comprises an activated alkyne group. In some embodiments, the modified protein comprises an activated alkyne group which is a cyclooctyne group. In some embodiments, the modified protein comprises a phosphine group. In some embodiments, the modified protein comprises a phosphine group which is a triarylphosphine group.

In some embodiments, the modified biomolecule is a protein comprising an azido group is attached to a solid support using functional groups other than azide reactive functional groups, whereupon the attached azido modified biomolecule is used to form a conjugate under Click chemistry conditions wherein the reporter molecule, carrier molecule or another solid support comprises a terminal alkyne. In another embodiment, the azido modified biomolecule is attached to a solid support using functional groups other than azide reactive functional groups, whereupon the attached azido modified biomolecule is used to form a conjugate under cycloaddition conditions wherein the reporter molecule, carrier molecule or other solid support comprises an activated alkyne. In another embodiment, the azido modified biomolecule is attached to a solid support using functional groups other than azide reactive functional groups, whereupon the attached azido modified biomolecule is used to form a conjugate under Staudinger ligation conditions wherein the reporter molecule, carrier molecule or other solid support comprises a triarylphosphine.

The disclosure also provides a method for detecting an immobilized modified biomolecule comprising an azido group, wherein the method comprises the steps of:
a) immobilizing the modified biomolecule on a solid or semi-solid matrix to form an immobilized modified biomolecule;
b) contacting the immobilized modified biomolecule with a reporter molecule that comprises a terminal alkyne group, an activated alkyne group or a triarylphosphine group to form a contacted biomolecule;
c) incubating the contacted biomolecule for a sufficient amount of time to form a reporter molecule-biomolecule conjugate;
d) illuminating the reporter molecule-biomolecule conjugate with an appropriate wavelength to form an illuminated reporter molecule-biomolecule conjugate, and
e) observing the illuminated reporter molecule-biomolecule conjugate whereby the immobilized biomolecule is detected.

The disclosure also provides a method for detecting an immobilized modified biomolecule comprising a terminal alkyne group, an activated alkyne group or a triarylphosphine group, wherein the method comprises the steps of:
a) immobilizing the modified biomolecule on a solid or semi-solid matrix to form an immobilized biomolecule;
b) contacting the immobilized modified biomolecule with a reporter molecule that comprises an azido group to form a contacted biomolecule;
c) incubating the contacted biomolecule for a sufficient amount of time to form a reporter molecule-biomolecule conjugate;
d) illuminating the reporter molecule-biomolecule conjugate with an appropriate wavelength to form an illuminated reporter molecule-biomolecule conjugate, and
e) observing the illuminated reporter molecule-biomolecule conjugate whereby the immobilized biomolecule is detected.

### Samples and Sample Preparation

The end user will determine the choice of the sample and the way in which the sample is prepared. Samples that can be used with the methods and compositions described herein include, but are not limited to, any biological derived material or aqueous solution that contains a modified biomolecule. In certain variants, a sample also includes material in which a modified biomolecule has been added. The sample that can be used with the methods and compositions described herein can be a biological fluid including, but not limited to, whole blood, plasma, serum, nasal secretions, sputum, saliva, urine, sweat, transdermal exudates, cerebrospinal fluid, or the like. In other variants, the samples are biological fluids that include tissue and cell culture medium wherein modified biomolecule of interest has been secreted into the medium. Cells used in such cultures include, but are not limited to, prokaryotic cells and eukaryotic cells that include primary cultures and immortalized cell lines. Such eukaryotic cells include, without limitation, ovary cells, epithelial cells, circulating immune cells, β cells, hepatocytes, and neurons. In certain variants, the sample may be whole organs, tissue or cells from an animal, including but not limited to, muscle, eye, skin, gonads, lymph nodes, heart, brain, lung, liver, kidney, spleen, thymus, pancreas, solid tumors, macrophages, mammary glands, mesothelium, and the like.

Various buffers can be used in the methods described herein, including inorganic and organic buffers. In certain variants the organic buffer is a zwitterionic buffer. By way of example only, buffers that can be used in the methods described herein include phosphate buffered saline (PBS), phosphate, succinate, citrate, borate, maleate, cacodylate, *N*-(2-Acetamido)iminodiacetic acid (ADA), 2-(N-morpholino)-ethanesulfonic acid (MES), N-(2-acetamido)-2-aminoethanesulfonic acid (ACES), piperazine-N,N'-2-ethanesulfonic acid (PIPES), 2-(N-morpholino)-2-hydroxypropanesulfonic acid (MOPSO), N,N-bis-(hydroxyethyl)-2-aminoethanesulfonic acid (BES), 3-(N-morpholino)-propanesulfonic acid (MOPS), N-tris-(hydroxymethyl)-2-ethanesulfonic acid (TES), N-2-hydroxyethyl-piperazine-N-2-ethanesulfonic acid (HEPES), 3-(N-tris-(hydroxymethyl) methylamino)-2-hydroxypropanesulfonic acid (TAPSO), 3-(*N,N-*Bis[2-hydroxyethyl]amino)-2-hydroxypropanesulfonic acid (DIPSO), N-(2-Hydroxyethyl)piperazine-N'-(2-hydroxypropanesulfonic acid) (HEPPSO), 4-(2-Hydroxyethyl)-1-piperazinepropanesulfonic acid (EPPS), *N-*[Tris(hydroxymethyl)methyl]glycine (Tricine), *N,N-*Bis(2-hydroxyethyl)glycine (Bicine), (2-Hydroxy-1,1-bis(hydroxymethyl)ethyl)amino]-1-propanesulfonic acid (TAPS), N-(1,1-Dimethyl-2-hydroxyethyl)-3-amino-2-hydroxypropanesulfonic acid (AMPSO), tris (hydroxy methyl) amino-methane (Tris), TRIS-Acetate-EDTA (TAE), glycine, bis[2-hydroxyethyl]iminotris[hydroxymethyl]methane (BisTris), or combinations thereof. In certain variants, wherein such buffers are used in gel electrophoresis separations the buffer can also include ethylene diamine tetraacetic acid (EDTA).

The concentration of such buffers used in the methods described herein is from about 0.1 mM to 1 M. In certain variants the concentration is between 10 mM to about 1. M. In certain variants the concentration is between about 20 mM and about 500 mM, and in other variants the concentration is between about 50 mM and about 300 mM. In certain variants, the buffer concentration is from about 0.1 mM to about 50 mM, while in other variants the buffer concentration if from about 0.5 mM to about 20 mM.

The pH will vary depending upon the particular assay system, generally within a readily determinable range wherein one or more of the sulfonic acid moieties is deprotonated.

In certain embodiments, buffers used in the methods described herein have a pH between 5 and 9 at ambient temperature. In certain variants the buffer has a pH between 6 and 8.5 at ambient temperature. In certain variants the buffer has a pH between 6 and 8 at ambient temperature. In certain variants the buffer has a pH between 6 and 7 at ambient temperature. In certain variants the buffer has a pH between 5 and 9 at 25 °C. In certain variants the buffer has a pH between 6 and 8.5 at 25 °C. In certain embodiments the buffer has a pH between 6 and 8 at 25 °C. In certain variants the buffer has a pH between 6 and 7 at 25 °C.

In certain variants, the sample used in the methods described herein have a non-ionic detergent to the sample. Non-limiting examples of such non-ionic detergents added to the samples used in the methods described herein are polyoxyalkylene diols, ethers of fatty alcohols including alcohol ethoxylates (Neodol from Shell Chemical Company and Tergitol from Union Carbide Corporation), alkyl phenol ethoxylates (Igepal surfactants from General Aniline and Film Corporation), ethylene oxide/propylene oxide block copolymers (PLURONIC™ Series from BASF Wyandotte Corporation), polyoxyethylene ester of a fatty acids (Stearox CD from Monsanto Company), alkyl phenol surfactants (Triton series, including Triton X-100from Rohm and Haas Company), polyoxyethylene mercaptan analogs of alcohol ethoxylates (Nonic 218 and Stearox SK from Monsanto Company), polyoxyethylene adducts of alkyl amines (Ethoduomeen and Ethomeen surfactants from Armak Company), polyoxyethylene alkyl amides, sorbitan esters (such as sorbitan monolaurate) and alcohol phenol ethoxylate (Surfonic from Jefferson Chemical Company, Inc.). Non-limiting examples of sorbitan esters include polyoxyethylene(20) sorbitan monolaurate (TWEEN20), polyoxyethylene(20) sorbitan monopalmitate (TWEEN40), polyoxyethylene(20) sorbitan monostearate (TWEEN60) and polyoxyethylene(20) sorbitan monooleate (TWEEN 80). In certain variants, the concentration of such non-ionic detergents added to a sample is from 0.01 to 0.5%. In other variants the concentration is from about 0.01 to 0.4 vol. %. In other variants the concentration is from about 0.01 to 0.3 vol. %. In other variants the concentration is from about 0.01 to 0.2 vol. %. In other variants the concentration is from about 0.01 to 0.1 vol. %.

### Illumination

The compounds and compositions described herein may, at any time before, after or during an assay, be illuminated with a wavelength of light that results in a detectable optical response, and observed with a means for detecting the optical response. In certain embodiments, such illumination can be by a violet or visible wavelength emission lamp, an arc lamp, a laser, or even sunlight or ordinary room light, wherein the wavelength of such sources overlap the absortion spectrum of a fluorpohore or chromaphore of the compounds or compositions decribed herein. In certain embodiments, such illumination can be by a violet or visible wavelength emission lamp, an arc lamp, a laser, or even sunlight or ordinary room light, wherein the fluorescent compounds, including those bound to the complementary specific binding pair member, display intense visible absorption as well as fluorescence emission.

In certain embodiments, the sources used for illuminating the fluorpohore or chromaphore of the compounds or compositions decribed herein include, but are not limited to, hand-held ultraviolet lamps, mercury arc lamps, xenon lamps, argon lasers, laser diodes, blue laser diodes, and YAG lasers. These illumination sources are optionally integrated into laser scanners, flow cytometer, fluorescence microplate readers, standard or mini fluorometers, or chromatographic detectors. These fluorescence emission of such fluorophores is optionally detected by visual inspection, or by use of any of the following devices: CCD cameras, video cameras, photographic film, laser scanning devices, fluorometers, photodiodes, photodiode arrays, quantum counters, epifluorescence microscopes, scanning microscopes, flow cytometers, fluorescence microplate readers, or by means for amplifying the signal such as photomultiplier tubes. Where the sample is examined using a flow cytometer, a fluorescence microscope or a fluorometer, the instrument is optionally used to distinguish and discriminate between the fluorescent compounds of the invention and a second fluorophore with detectably different optical properties, typically by distinguishing the fluorescence response of the fluorescent compounds of the invention from that of the second fluorophore. Where a sample is examined using a flow cytometer, examination of the sample optionally includes isolation of particles within the sample based on the fluorescence response by using a sorting device.

In certain embodiments, fluorescence is optionally quenched using either physical or chemical quenching agents.

### Kits of the Invention

In another aspect, the present invention provides a kit that comprises a compound of formula [I] as described above, and further comprises at least one of
(a) a solution comprising Cu(I) ions; Cu(I) ions and a copper chelator; Cu(II) ions; at least one reducing agent; a copper chelator; at least one reducing and a copper chelator; Cu(II) ions and at least one reducing agent; Cu(II) ions and a copper chelator; or, Cu(II) ions, at least one reducing agent and a copper chelator; or
(b) a reporter molecule, carrier molecule, or solid support comprising a chemical handle capable of reacting with the alkyne reactive group or azide reactive moiety of the compound.

In another variant, the disclosure includes a kit for labeling a modified biomolecule that comprises at least one label that comprises an azido group, and a solution comprising copper ions, a solution that comprises a copper (I) chelator. The kit can further comprise a solution that comprises a reducing agent, one or more buffers, or one or more detergents.

In one form of this variant, a label comprising an azido group provided in a kit is a fluorophore, such as, but not limited to, a xanthene, coumarin, borapolyazaindacene, pyrene, cyanine, carbopyranine, or semiconductor nanocrystal. In other variants, a label comprising an azido group provided in a kit is a tag, such as but not limited to a peptide or a hapten, such as biotin. In one variant, a kit provides two or more different labels each comprising an azido group, one or more of which is a fluorophore. In some variants, a copper (I) chelator provided in the kit is a 1,10 phenanthroline, bathocuproine disulfonic acid, or THPTA. In some variants, copper is provided in the form of a copper sulfate or copper acetate solution. In some variants, a reducing agent is provided in the form of ascorbate.

In one variant, the disclosure includes a kit for labeling a modified biomolecule that includes at least one label that comprises a terminal alkyne group, a solution comprising copper, and a solution that comprises a copper (I) chelator. The kit can further comprise a solution that comprises a reducing agent, one or more buffers, or one or more detergents.

In one variant, a label comprising a terminal alkyne provided in a kit is a fluorophore, such as, but not limited to, a xanthene, coumarin, borapolyazaindacene, pyrene, cyanine, carbopyranine, or semiconductor nanocrystal. In one variant, a kit provides two or more different terminal labels each comprising a terminal alkyne group, where each label is different a fluorophore. In other variants, the label comprising a terminal alkyne group provided in a kit is a tag, such as but not limited to a peptide or a hapten, such as biotin. In certain variants, a copper (I) chelator provided in the kit is a 1,10 phenanthroline, bathocuproine disulfonic acid, or THPTA. In some variants, copper is provided in the form of a copper sulfate or copper acetate solution. In some variants, a reducing agent is provided in the form of ascorbate.

In another variant, the dislcosure includes a kit for labeling a modified biomolecule that includes at least one label that comprises an azido group. In one variant, an azido-containing label provided in a kit is a fluorophore, such as, but not limited to, a xanthene, coumarin, borapolyazaindacene, pyrene, cyanine, carbopyranine, or semiconductor nanocrystal. In other variants, a label comprising an azido group provided in a kit is a tag, such as but not limited to a peptide or a hapten, such as biotin. In one variant, a kit provides two or more different a label comprising an azido group, one or more of which is a fluorophore.

In another variant, the disclosure includes a kit for labeling a modified biomolecule that includes at least one label that comprises activated alkyne group.

In one varint, a label comprises an activated alkyne provided in a kit is a fluorophore, such as, but not limited to, a xanthene, coumarin, borapolyazaindacene, pyrene, cyanine, carbopyranine, or semiconductor nanocrystal. In one variant, a kit provides two or more different terminal labels each comprising an activated alkyne group, where each label is different a fluorophore. In other variants, the label comprising an activated alkyne group provided in a kit is a tag, such as but not limited to a peptide or a hapten, such as biotin.

In another variant, the disclosure includes a kit for labeling a modified biomolecule that includes at least one label that comprises a triarylphosphine group.

In one form of this variant, label comprising an triarylphosphine group provided in a kit is a fluorophore, such as, but not limited to, a xanthene, coumarin, borapolyazaindacene, pyrene, cyanine, carbopyranine, or semiconductor nanocrystal. In other variants, label comprising a triarylphosphine group provided in a kit is a tag, such as but not limited to a peptide or a hapten, such as biotin. In one variant, a kit provides two or more different labels each comprising an triarylphosphine group, one or more of which is a fluorophore.

In other variants, a kit can further comprise one or more reagents and solutions for chromogenic detection on Western blots.

A detailed description of the invention having been provided above, the following examples are given for the purpose of illustrating the invention and shall not be construed as being a limitation on the scope of the invention or claims.

The following examples are intended to illustrate but not limit the invention.

### Example 1

The linoleic acid analog containing an azido group 3 and the linoleic acid analog containing an terminal acetylene group **4** were synthesized as shown in FIG. 1.

The synthesis of the acid chloride **1** was done under anhydrous conditions, using DMF as a catalyst. The reaction was fairly quick (∼5 min) and observed on TLC by conversion to the methyl ester (quench with MeOH). The acid chloride **2** was then readily converted to either compound **3** or **4** by low temperature addition (-78°C) in the presence of DIEA.

### Example 2

The linoleic acid analogs **11, 12,** and **14** are made as shown in FIG. 2.

### Example 3

Bovine pulmonary artery endothelial (BPAE) cells were cultured on coverslips in DMEM +10% FBS. The media was replaced with with DMEM + 0.5% FBS 19 hours prior to treatment for 3 hours with 40 µM linoleic acid azide analog **3** with or without cotreatment with 40 µM menadione. Cells were fixed 15 minutes with 4% methanol-free paraformaldehyce and permeablized 15 minutes with 0.25% Triton X-100 in dPBS. Cells were then labeled for 30 minutes with 2µM Alexa Fluor® 594 alkyne (Invitrogen, San Diego, California, catalog # A10275) under the click conditions (2mM CuSO₄, 10mM sodium ascorbate in Tris-buffered saline) followed by staining with 2 µg/mL Hoechst nuclear stain for 15 min. Coverslips were mounted in ProLong® Gold anti-fade reagent. Images shown are are 40X.

FIG. 3 shows the image of BPAE cells which were treated with the linoleic acid azide analog **3,** then were treated with and without menadione to induce oxidative stress. FIG. 4 shows the image of BPAE cells which were not treated with the linoleic acid azide analog **3,** but were treated with and without menadione to induce oxidative stress.

Cells treated with the linoleic acid azide analog 3 showed increased Alexa Fluor® 594 click staining over control untreated cells, demonstrating a background level of linoleic acid-induced protein modification. A significant increase in Alexa Fluor® 594 staining was seen in cells treated with the linoleic acid azide analog 3 plus menadione, indicating that menadione treatment increased the formation of oxidatively-induced linoleic acid protein modification.

To confirm these results, proteins from lysed cells were click-labeled with TAMRA alkyne dye (Invitrogen, San Diego, California, catalog # T10183) and separated by SDS-PAGE. The gels were imaged and results showed a significant increase in fluorescence from menadione treated cells over cells treated with the linoleic acid azide analog **3** only. Modified proteins from control and treated cells were enriched using click chemistry-based alkyne resins. After stringent washing with denaturing agents, the proteins were reduced and alkylated. The bound proteins were serially digested off the resin with Lys-C and trypsin, and the resulting peptide pools were separated by 2-D chromatography (SCX and RP) and analyzed by tandem mass spectrometry. Greater than fifty modified proteins were identified in cells treated with the linoleic acid azide analog **3,** while only 2 proteins were identified in the control untreated samples demonstrating the high-selectivity and purity of the sample digests. The results also show a significant increase in protein modification in response to menadione treatment.

### Example 4

BPAE cells were cultured on coverslips in DMEM +10% FBS. The media was replaced with with DMEM + 0.5% FBS 19 hours prior to treatment for 3 hours with 40 uM linoleic acid alkyne analog **4** with or without co-treatment with 40 µM menadione. Cells were fixed 15 minutes with 4% methanol-free paraformaldehyce and permeablized 15 minutes with 0.25% Triton X-100 in dPBS. Cells were then labeled for 30 minutes with 2µM Alexa Fluor® 594 azide (Invitrogen, San Diego, California, catalog # A10270) under the click conditions (2mM CuSO₄, 10mM sodium ascorbate in Tris-buffered saline) followed by staining with 2 µg/mL Hoechst nuclear stain for 15 min. Coverslips were mounted in ProLong® Gold anti-fade reagent. Images shown are are 40X.

FIG. 3 shows the image of BPAE cells which were treated with the linoleic acid alkyne analog **4,** then were treated with and without meriadione to induce oxidative stress. FIG. 4 shows the image of BPAE cells which were not treated with the linoleci acid alkyne analog **4,** but were treated with and without menadione to induce oxidative stress.

No significant difference in staining was seen between the specificity of the linoleic acid analogs **3** and **4,** although the alkyne detection dye had a slightly higher background.

Cells treated with the linoleic acid alkyne analog **4** showed increased Alexa Fluor® 594 click staining over control untreated cells, demonstrating a background level of linoleic acid-induced protein modification. A significant increase in Alexa Fluor® 594 staining was seen in cells treated with the linoleic acid alkyne analog **4** plus menadione, indicating that menadione treatment increased the formation of oxidatively-induced linoleic acid protein modification. No significant difference in staining was seen between the specificity of the linoleic acid analogs **3** and **4,** although the alkyne detection dye had a slightly higher background.

### Example 5

Macrophages were metabolically labeled with linoleic acid -azide or -alkyne analogs **3** and **4** and treated with hemin to induce oxidative stress. Fixed macrophages, or isolated macrophage proteins, were click-labeled with fluorescent dyes and analyzed by fluorescence microscopy, or SDS-PAGE, respectively. In each case, there was a dramatic increase in fluorescence signal upon hemin treatment. Azide modified proteins were also enriched on alkyne resin. After stringent washing, bound proteins were digested off the resin and analyzed by mass spectrometry. The resulting peptide pools demonstrate unprecedented selectivity and purity of labeled samples versus controls.

### Imaging of cells after treatment with linoleic acid alkyne analog 4 and hemin.

FIG. 7 shows the image of RAW 264.7 cells after treatment with linoleic acid alkyne analog **4** using hemin to induce oxidative stress. RAW 264.7 cells were grown in a 96 well plate in DMEM, 10% FBS at 37°C/5% CO₂ to 40-70% confluence. Medium was replaced for two hours with serum free DMEM and cells were then treated for two hours with 20 µM linoleic acid alkyne analog **4** in the absence (middle) or presence (right) of 10 µM hemin-Cl. As control, cells were exposed to an equal volume of vehicle DMSO (left).

After removal of the medium, cells were fixed for 15 minutes with 4% formaldehyde in DPBS at room temperature, washed 2 x with DPBS, permeabilized with 0.25% TX-100 in DPBS and washed for 10 minutes with 3% BSA in DPBS. Cu click staining was carried out with 1 µM Alexa Fluor® 488 azide (Invitrogen, San Diego, California, catalog # A10266) in TBS with 2 mM CuSO₄ and 10 mM ascorbate for 30 minutes at room temperature in 100 µL per well. Cells were washed for 10 minutes with 3% BSA in DPBS, counterstained with Hoechst 33342 (Invitrogen, San Diego, California, catalog # H3570, 1:3000 dilution), washed 3 x with DPBS and imaged with Thermo Scientific Cellomics® ArrayScan® VTI platform at 10X for quantitation of signal increase due to hemin induced oxidation. After that, imaging was carried out on an Axiovert (Zeiss) at 40X to obtain the presented images. A dramatic increase of signal was observed in the hemin treated, linoleic acid alkyne analog **4** containing sample compared to the linoleic acid alkyne analog **4** only and the DMSO sample.

### SDS-PAGE of cells treated with linoleic acid alkyne 4 and hemin.

Cells were grown on 6 well plates to 95-100% confluence in DMEM, 10% FBS at 37°C/5% CO₂. After replacing the medium with serum free DMEM and incubation for 2 hours at 37°C/5% CO₂, 20 µM linoleic acid alkyne analog 4 with and without 10 µM hemin was added for 30, 60 and 90 minutes. Controls including hemin (10 µM) or DMSO were incubated for the same time in a time course experiment. After incubation, cells were washed 3 x with DPBS. Cells were collected by adding 200 µL 50 mM Tris, pH 8.0, supplemented with 0.5 % SDS, 2 µL Protease Inhibitor Cocktail and 300 U Benzonase/mL per well and mixing for 15 minutes. The lysates were transferred in 1.5 mL tubes and precipitated with Chloroform/Methanol. The pellet was dried for 15 minutes and dissolved in 200 µL 50 mM Tris, pH 8.0. For Copper click reaction, 50 µL were used per condition. In a total volume of 200 µL click reaction was carried out with 20 µM TAMRA-azide (Invitrogen, San Diego, California, catalog # T10182) as described in MP33370 (Click-iT Protein Analysis Detection Kits). After Chloroform/Methanol precipitation SDS PAGE was carried out with 4-12% Bis-Tris gels in MOPS. TAMRA fluorescence was detected with a Fuji imager at 532 nm (excitation). Gels were then stained with the SYPRO®-Ruby Protein Stain and imaged. The dramatic increase of signal, observed in FIG. 8A of the hemin treated linoleic acid alkyne 4 containing sample, compared to the linoleic acid alkyne 4 only and the DMSO sample, was quantified in FIG. 8B.

LC/MS analysis of lysates from cells treated with linoleic acid-azide **3** and hemin.

RAW 264.7 cells were grown to confluence on T225 plates, placed in DMEM and treated with 20 µM linoleic acid azide 3 and 10 µM hemin for two hours and collected cells were lysed in 2 mL tubes. Modified proteins were bound by click chemistry to alkyne beads. Depletion efficiency was determined by click reacting with TAMRA alkyne equal amounts before and after binding to the alyne resin with the EZQ kit by dividing the TAMRA fluorescence intensity by SYPRO Ruby intensity on membranes.

Chromatography and mass spectrometry analysis. All mass spectrometry analysis was done using Waters SYNAPT™ mass spectrometry system with electrospray ionization. All separations were done on a Waters AcQuity UPLC^{®} system. Peptides were fractionated by strong cation exchange chromatography using a PolySULFOETHYL ATM 5 µm, 2.1 x 100mm column (PolyLC, Inc). Peptide fractions were separated by reverse phase chromatography using a Waters UPLC^{®} BEH C18 column, 1.7 µm, 1.0 x 150 mm and analyzed by tandem mass spectrometry.

Database searching for protein identification and quantification. Tandem mass spectra were extracted by Mascot Distiller version 2.3.2.0. All MS/MS samples were analyzed using Mascot (Matrix Science) and X! Tandem (The GPM; version 2007.01.01.1). Mascot and X! Tandem was set up to search a subset of the SwissProt database also assuming trypsin. Mascot and X! Tandem were searched with a fragment ion mass tolerance of 0.60 Da and a parent ion tolerance of 1.2 Da. Oxidation of methionine was specified as a variable modification. Scaffold Q+ (Proteome Software Inc.) was used to quantify isobaric tag (iTRAQ®) identifications. Peptides were quantified using the centroided reporter ion peak intensity. Multiple isobaric tag samples were normalized by comparing the median protein ratios for the reference channel. Protein quantitative values were derived from only uniquely assigned peptides. The minimum quantitative value for each spectrum was calculated as the 5.0% percent of the highest peak. Protein quantitative ratios were calculated as the median of all peptide ratios.

FIG. 9 shows in a Venn diagram, that the majority of the proteins identified by mass spectrometry were from cells treated with linoleic acid azide analog 3 while under hemin induced oxidative stress. Almost no proteins were identified in the control cells (DMSO treated).

### Example 6

Bovine pulmonary artery endothelial (BPAE) cells were metabolically labeled with linoleic acid alkyne analog 4 and treated with hemin to induce oxidative stress. Fixed BPAE cells were click-labeled with fluorescent dyes and analyzed by fluorescence microscopy. There was a dramatic increase in fluorescence signal upon hemin treatment.

FIG. 10 shows the image of BPAE cells after treatment with linoleic acid alkyne analog 4 treated with hemin to induce oxidative stress. Cells were grown in a 96 well plate in DMEM, 10% FBS at 37°C/5% CO₂ to 40-70% confluence. Medium was replaced for two hours with serum free DMEM and cells were then treated for two hours with 20 µM linoleic acid alkyne analog 4 in the absence (middle) or presence (right) of 10 µM hemin-Cl. As control, cells were exposed to an equal volume of vehicle DMSO (left).

After removal of the medium, cells were fixed for 15 minutes with 4% formaldehyde in DPBS at room temperature, washed 2 x with DPBS, permeabilized with 0.25% TX-100 in DPBS and washed for 10 minutes with 3% BSA in DPBS. Copper click staining was carried out with 1 µM Alexa Fluor® 488 azide (Invitrogen, San Diego, California, catalog # A10266) in TBS with 2 mM CuSO₄ and 10 mM ascorbate for 30 minutes at room temperature in 100 µL per well. Cells were washed for 10 minutes with 3% BSA in DPBS, counterstained with Hoechst 33342 (Invitrogen, San Diego, California, catalog # H3570, 1:3000 dilution), washed 3 x with DPBS and imaged with Thermo Scientific Cellomics® ArrayScan® VTI platform at 10X for quantitation of signal increase due to hemin induced oxidation. After that, imaging was carried out on an Axiovert (Zeiss) at 40X to obtain the presented images. A dramatic increase of signal was observed in the hemin treated linoleic acid alkyne **4** containing sample compared to the linoleic acid alkyne **4** only and the DMSO sample.

### Example 7

Osteosarcoma cells (U-2 OS) were metabolically labeled with linoleic acid alkyne analog **4** and treated with hemin to induce oxidative stress: Fixed U-2 OS cells were click-labeled with fluorescent dyes and analyzed by fluorescence microscopy. There was a dramatic increase in fluorescence signal upon hemin treatment.

FIG. 11 shows the image of U-2 OS cells after treatment with linoleic acid alkyne analog 4 treated with hemin to induce oxidative stress. Cells were grown in a 96 well plate in McCoy's 5a medium, 10% FBS at 37°C/5% CO₂ to 40-70% confluence. Medium was replaced for two hours with serum free McCoy's 5a medium and cells were then treated for two hours with 20 µM linoleic acid alkyne analog 4 in the absence (middle) or presence (right) of 10 µM hemin-Cl. As control, cells were exposed to an equal volume of vehicle DMSO (left).

After removal of the medium, cells were fixed for 15 minutes with 4% formaldehyde in DPBS at room temperature, washed 2 x with DPBS, permeabilized with 0.25% TX-100 in DPBS and washed for 10 minutes with 3% BSA in DPBS. Copper click staining was carried out with 1 µM Alexa Fluor® 488 azide (Invitrogen, San Diego, California, catalog # A10266) in TBS with 2 mM CuSO₄ and 10 mM ascorbate for 30 minutes at room temperature in 100 µL per well. Cells were washed for 10 minutes with 3% BSA in DPBS, counterstained with Hoechst 33342 (Invitrogen, San Diego, California, catalog # H3570,1:3000 dilution), washed 3 x with DPBS and imaged with Thermo Scientific Cellomics® ArrayScan® VTI platform at 10X for quantitation of signal increase due to hemin induced oxidation. After that, imaging was carried out on an Axiovert (Zeiss) at 40X to obtain the presented images. A dramatic increase of signal was observed in the hemin treated linoleic acid alkyne analog **4** containing sample compared to the linoleic acid alkyne analog **4** only and the DMSO sample.

## Claims

1. A compound of the formula: 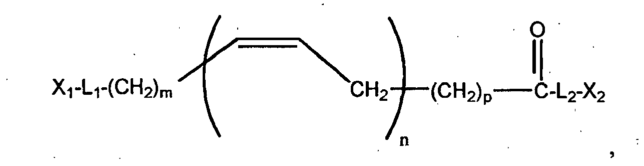 wherein
m is 1-4;
n is 2-3 or 5-6;
p is 1-12;
at least one of X₁ or X₂ is selected from the group consisting of alkyne reactive moiety and azide reactive moiety, and the other is selected from the group consisting of H, alkyl, alkenyl, cycloalkyl, cycloalkenyl, alkoxy, aryl, aralkyl, heteroaryl, and heteroaralkyl; and
L₁ and L₂ are independently selected from the group consisting of O, NH, alkyl linker group comprising 1-10 carbon atoms, and alkyl linker group comprising 1-10 carbon atoms any of which may be substituted with one or more heteroatoms independently selected from the group consisting of O, N and S; wherein the compound is not:

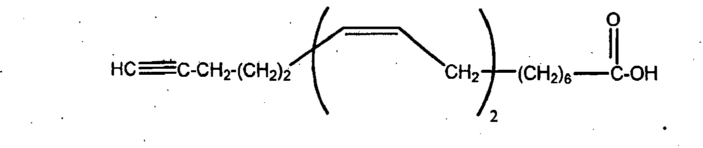

2. The compound of claim 1, wherein X₁ is an alkyne reactive moiety; and X₂ is selected from the group consisting of an H, alkyl, alkenyl, cycloalkyl, cycloalkenyl, alkoxy, aryl, aralkyl, heteroaryl, and heteroaralkyl.

3. The compound of claim 1, wherein X₁ is azide reactive moiety; and X₂ is selected from the group consisting of an H, alkyl, alkenyl, cycloalkyl, cycloalkenyl, alkoxy, aryl, aralkyl, heteroaryl, and heteroaralkyl.

4. The compound of claim 1, wherein X₂ is an alkyne reactive moiety; and X₁ is selected from the group consisting of H, alkyl, alkenyl, cycloalkyl, cycloalkenyl, alkoxy, aryl, aralkyl, heteroaryl, and heteroaralkyl.

5. The compound of claim 1, wherein X₂ is an azide reactive moiety; and X₁ selected from the group consisting of H, alkyl, alkenyl, cycloalkyl, cycloalkenyl, alkoxy, aryl, aralkyl, heteroaryl, and heteroaralkyl.

6. The compound of claim 1, wherein X₁ and X₂ are independently selected from the group consisting of an alkyne reactive moiety, and azide reactive moiety.

7. The compound of claim 1 selected from the group consisting of: 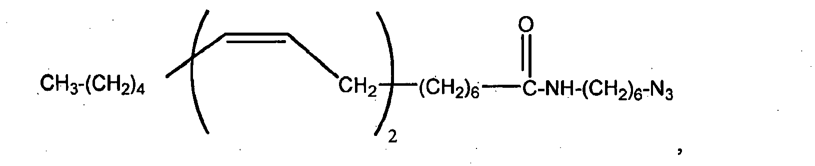 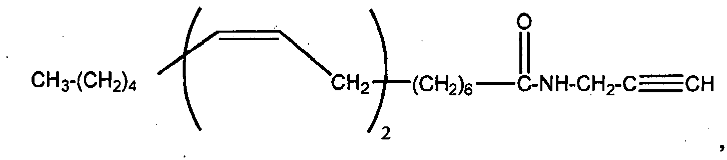 and

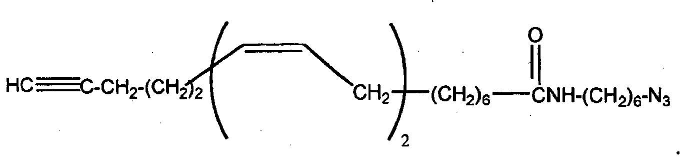

8. A method of detecting in a cell a modified protein generated in response to oxidative cellular conditions, comprising the steps of:
(a) contacting a cell in an aqueous solution with a compound having the formula: 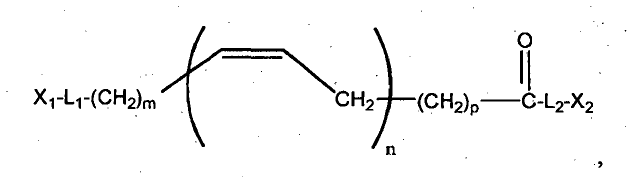 wherein
m is 1-4;
n is 2-6;
pis 1-12;
at least one of X₁ or X₂ is selected from the group consisting of alkyne reactive moiety and azide reactive moiety, and the other is selected from the group consisting of H, alkyl, alkenyl, cycloalkyl, cycloalkenyl, alkoxy, aryl, aralkyl, heteroaryl, and heteroaralkyl; and
L₁ and L₂ are independently selected from the group consisting of O, NH, alkyl linker group comprising 1-10 carbon atoms, and alkyl linker group comprising 1-10 carbon atoms any of which may be substituted with one or more heteroatoms independently selected from the group consisting of O, N and S;
(b) contacting the cell in the aqueous solution with a reporter molecule comprising a chemical handle capable of reacting with the alkyne reactive moiety or azide reactive moiety of the compound; and
(c) detecting the presence of the modified protein in the cell.

9. The method of claim 8, wherein the aqueous solution in step (b) further comprises Cu(I) ions; Cu(I) ions and a copper chelator; Cu(II) ions and at least one reducing agent; or, Cu(II) ions, at least one reducing agent and a copper chelator.

10. A method of detecting in solution a modified protein generated in response to oxidative cellular conditions, comprising the steps of:
(a) contacting a cell in an aqueous solution with a compound having the formula: 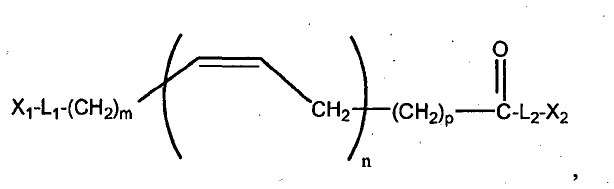 wherein
m is 1-4;
n is 2-6;
p is 1-12;
at least one of X₁ or X₂ is selected from the group consisting of alkyne reactive moiety and azide reactive moiety, and the other is selected from the group consisting of H, alkyl, alkenyl, cycloalkyl, cycloalkenyl, alkoxy, aryl, aralkyl, heteroaryl, and heteroaralkyl; and
L₁ and L₂ are independently selected from the group consisting of O, NH, alkyl linker group comprising 1-10 carbon atoms, and alkyl linker group comprising 1-10 carbon atoms any of which may be substituted with one or more heteroatoms independently selected from the group consisting of O, N and S;
(b) preparing an isolate of the cell;
(c) contacting the isolate with a reporter molecule, carrier molecule or solid support comprising a chemical handle capable of reacting with the alkyne reactive moiety or azide reactive moiety of the compound; and
(d) detecting the presence of the modified protein.

11. The method of claim 10, wherein the isolate in step (c) further comprises Cu(I) ions; Cu(I) ions and a copper chelator; Cu(II) ions and at least one reducing agent; or, Cu(II) ions, at least one reducing agent and a copper chelator.

12. The method of any of claims 8 to 11, wherein X₁ and X₂ are as defined in any of claims 2 to 6.

13. The method of any of claims 8 to 11, wherein the compound is as defined in claim 7.

14. A kit comprising a compound of any of claims 1 to 7:
and further comprising at least one of:
(a) a solution comprising Cu(I) ions; Cu(I) ions and a copper chelator; Cu(II) ions; at least one reducing agent; a copper chelator; at least one reducing and a copper chelator; Cu(II) ions and at least one reducing agent; Cu(II) ions and a copper chelator; or, Cu(II) ions, at least one reducing agent and a copper chelator; or,
(b) a reporter molecule, carrier molecule, or solid support comprising a chemical handle capable of reacting with the alkyne reactive moiety or azide reactive moiety of the compound.

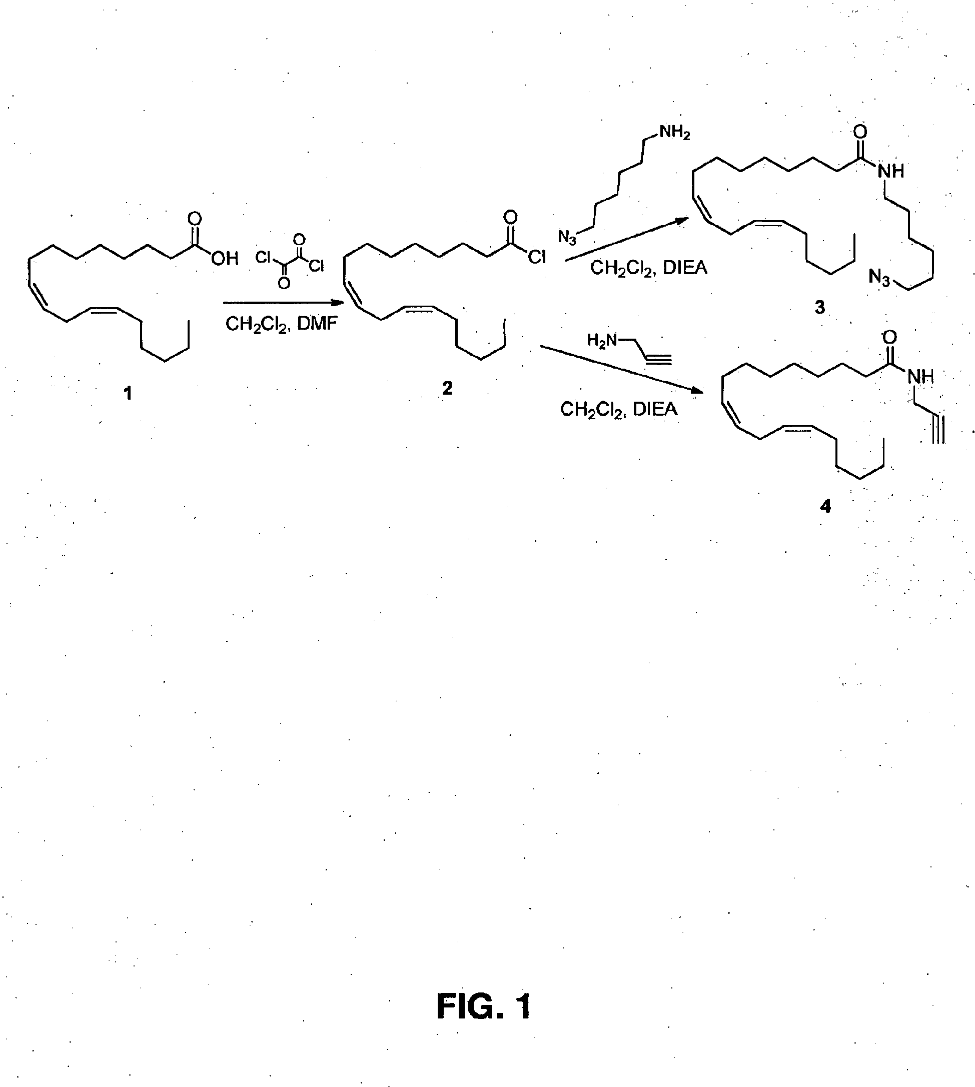

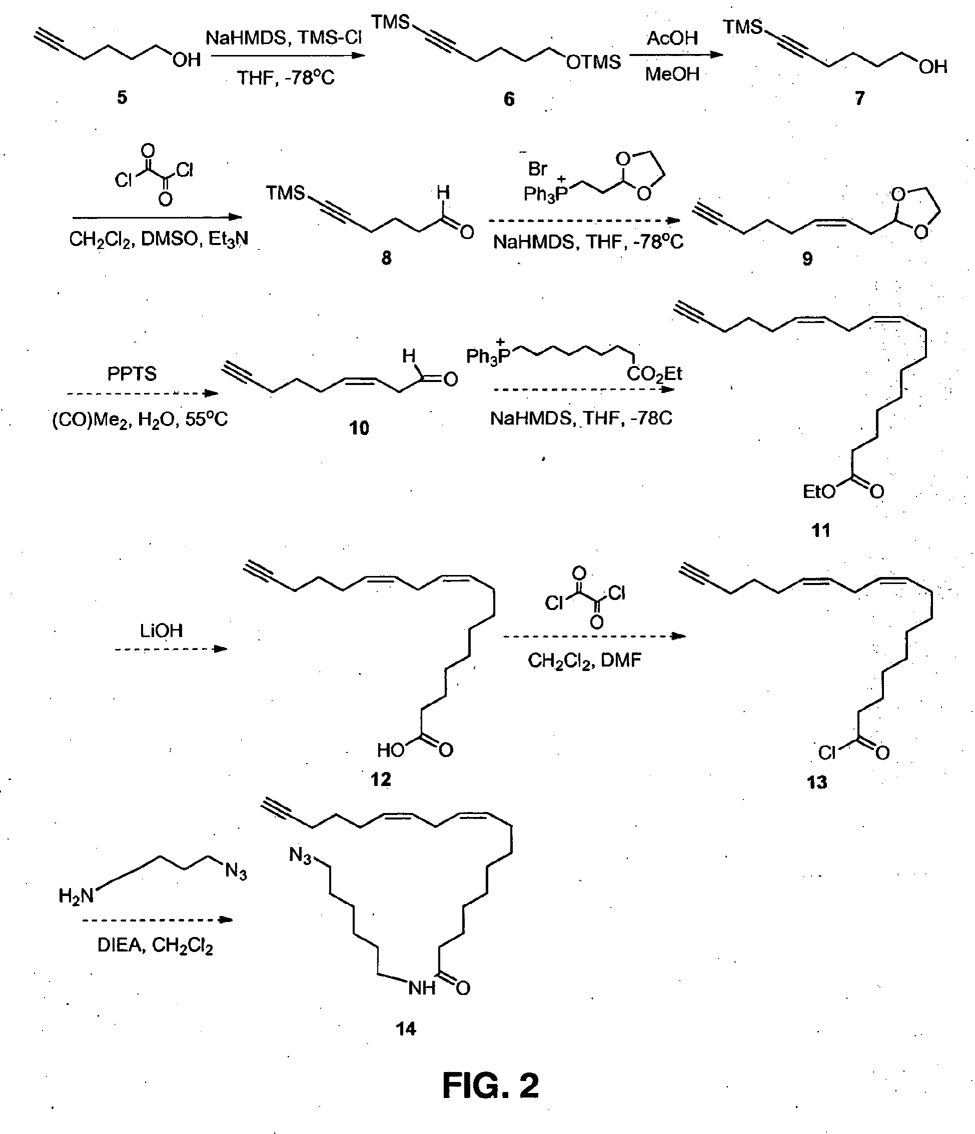

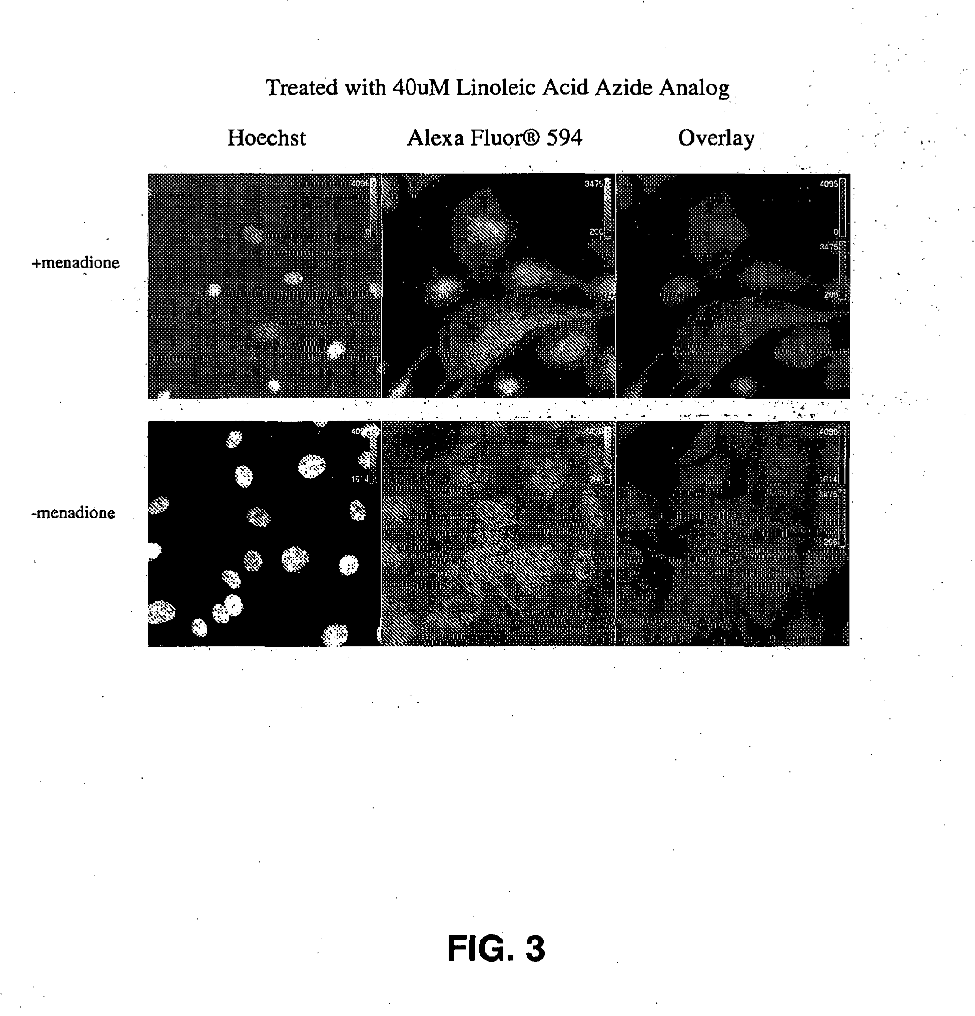

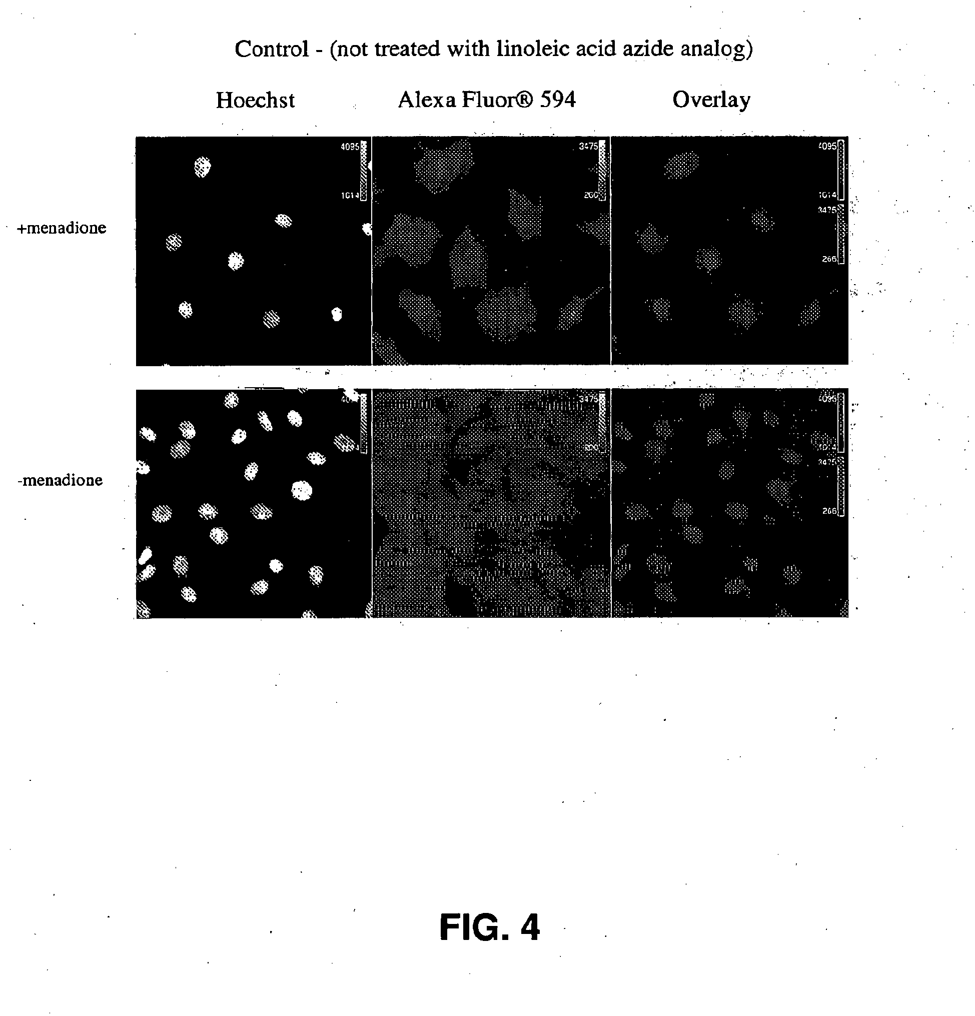

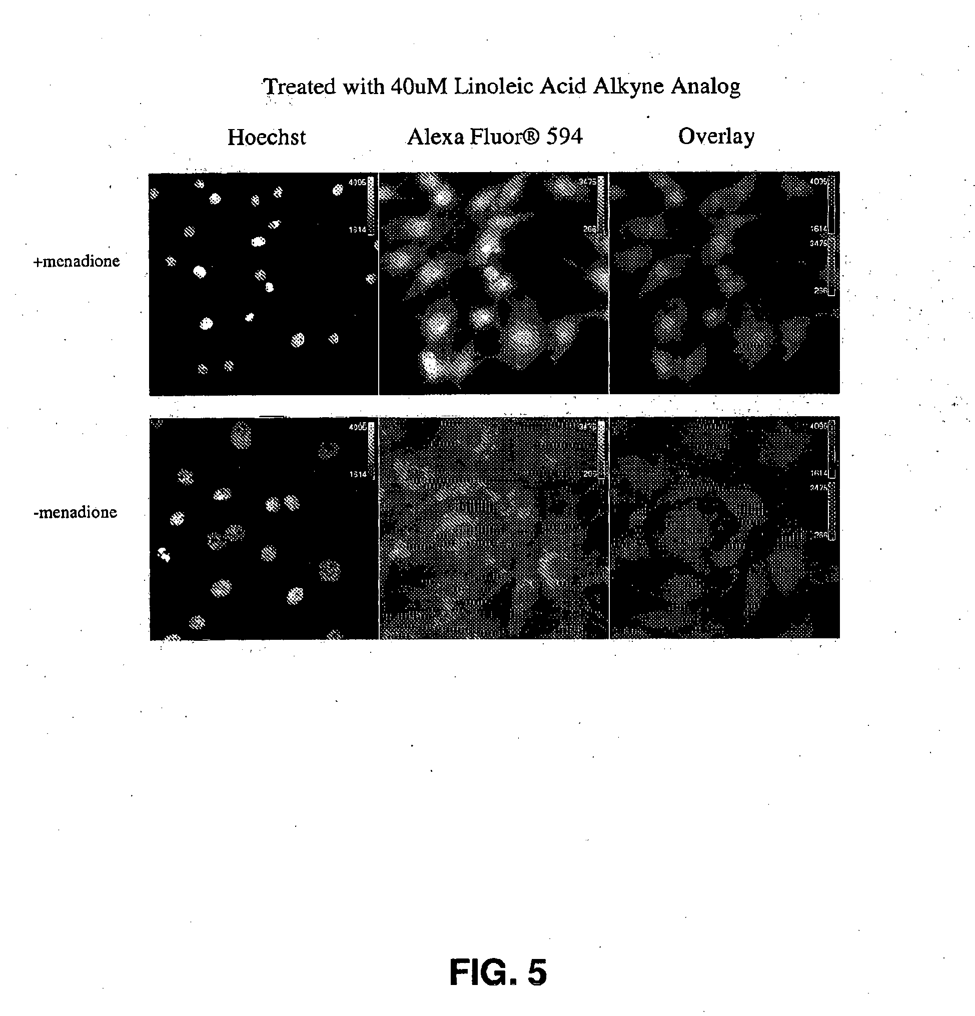

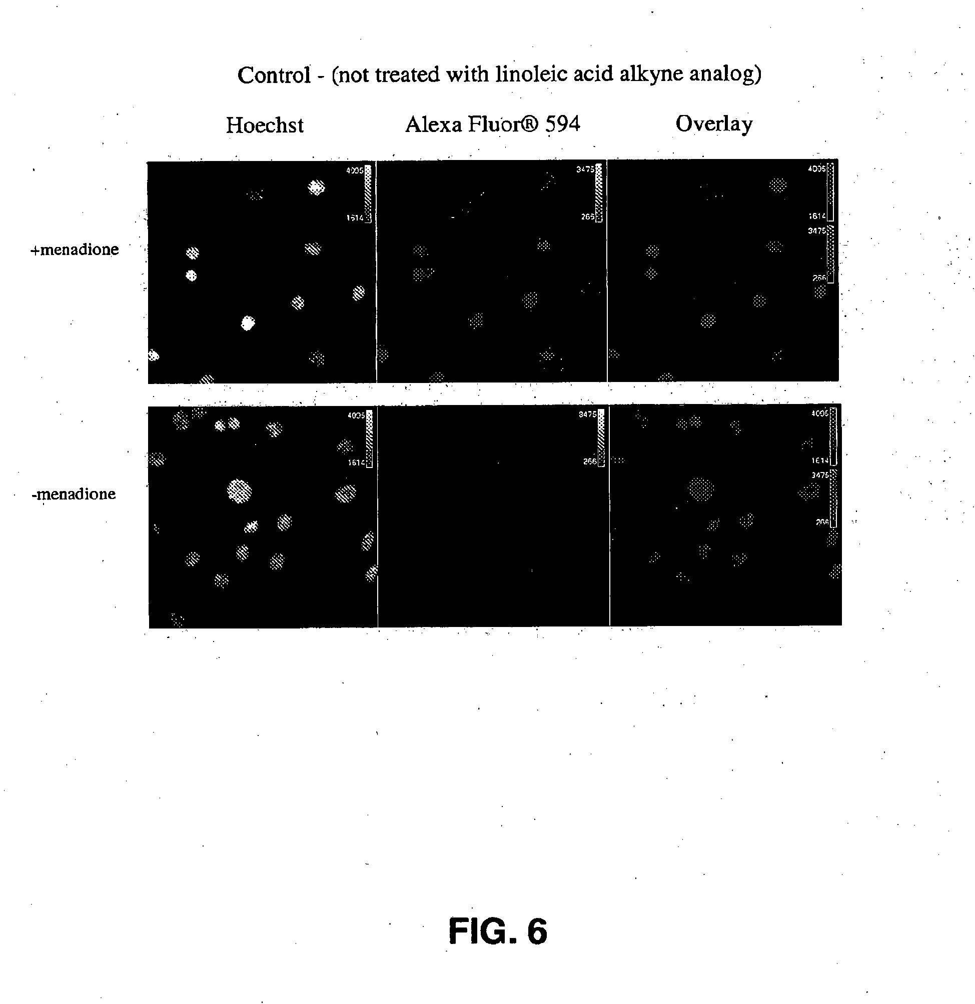

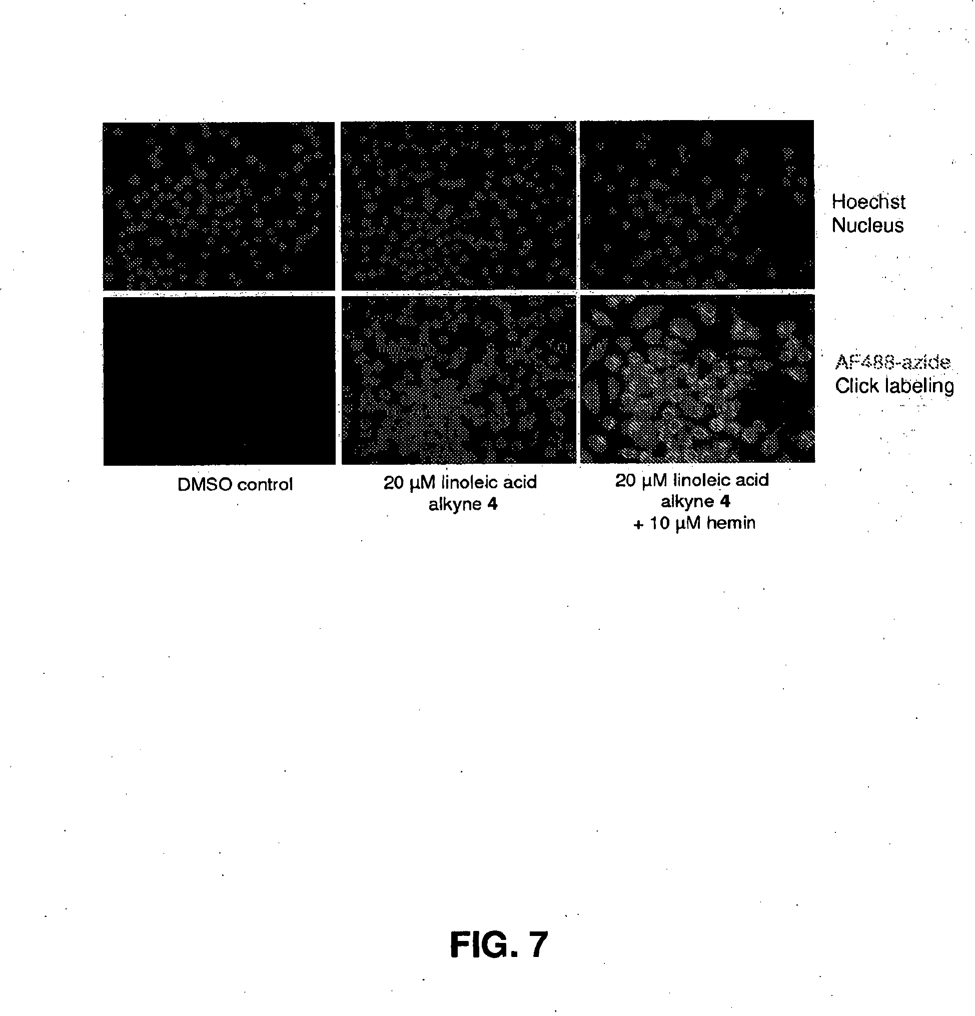

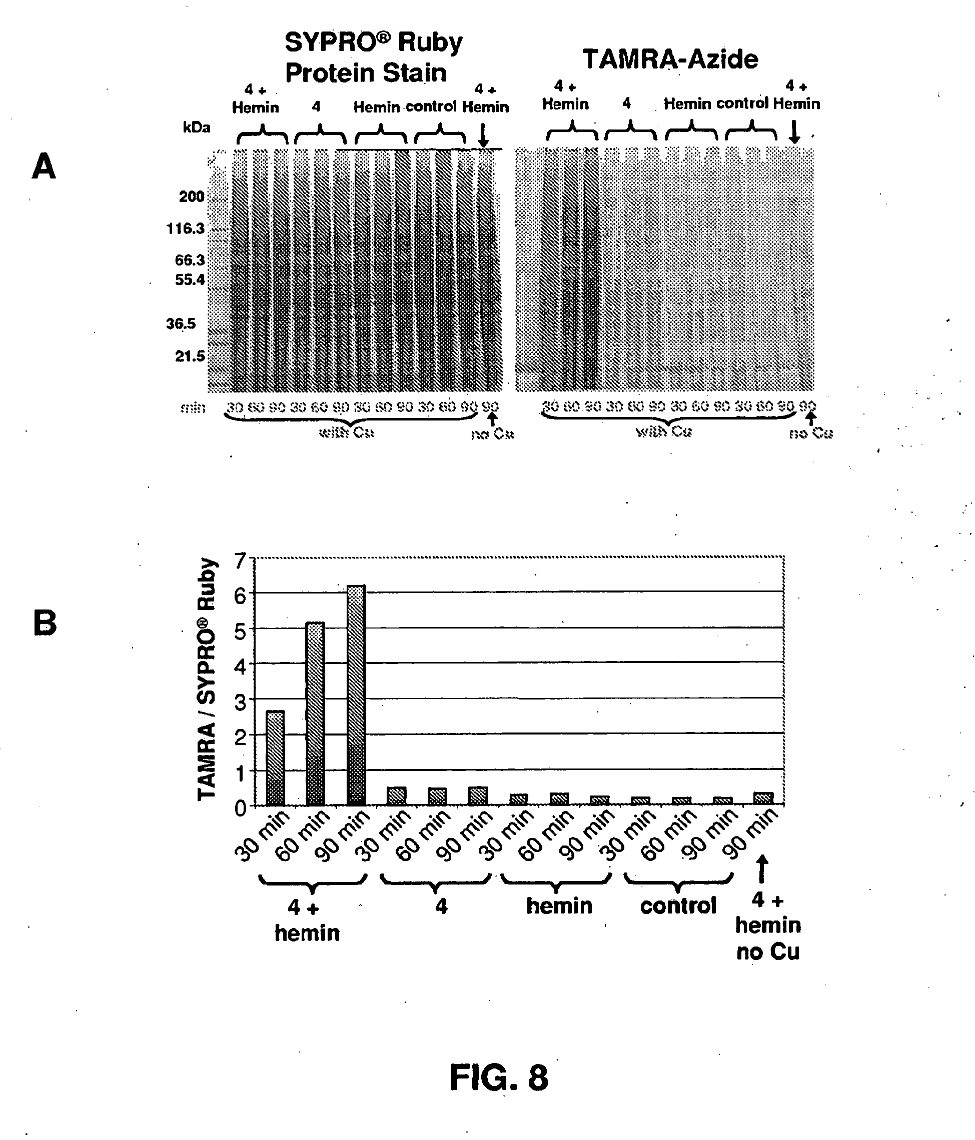

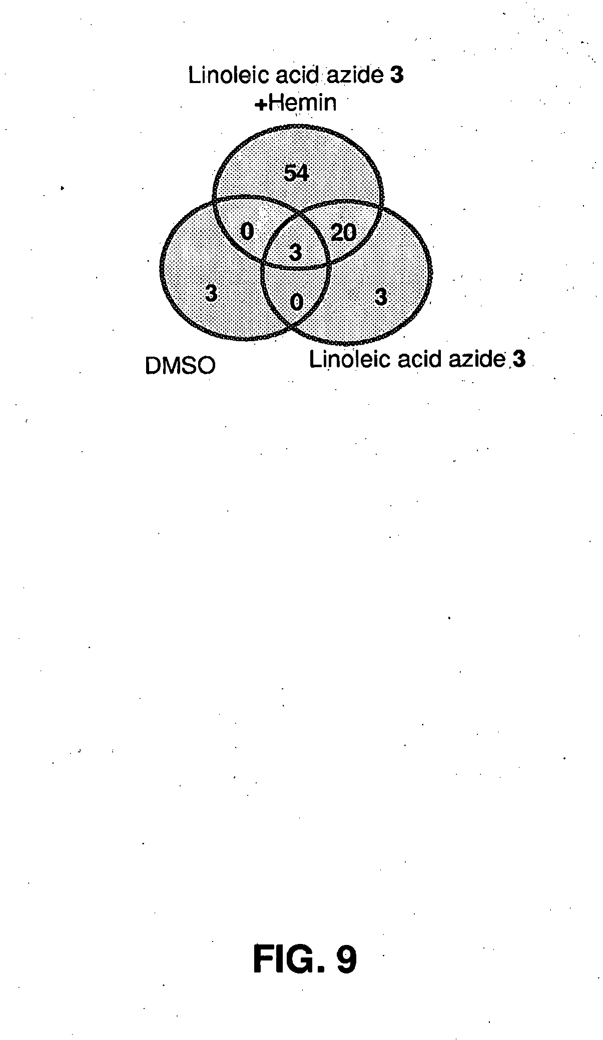

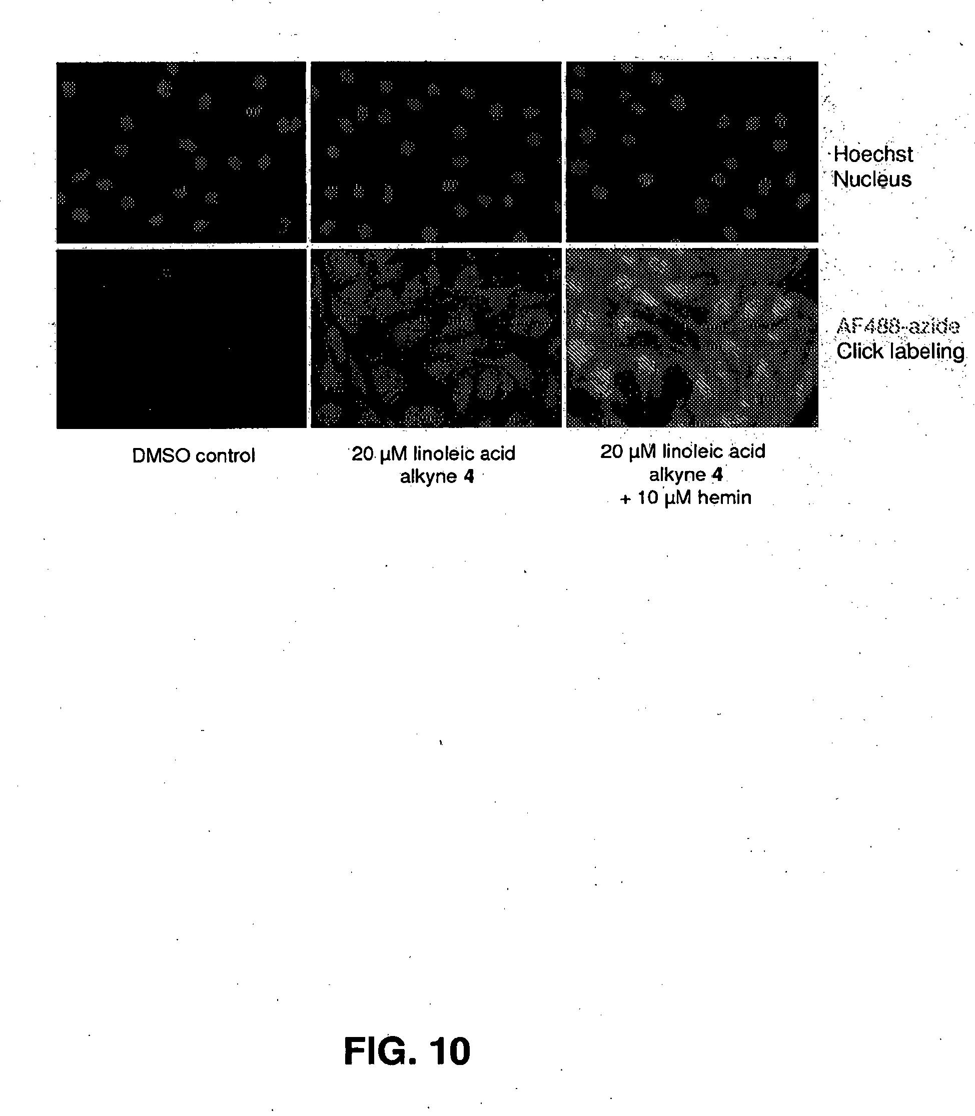

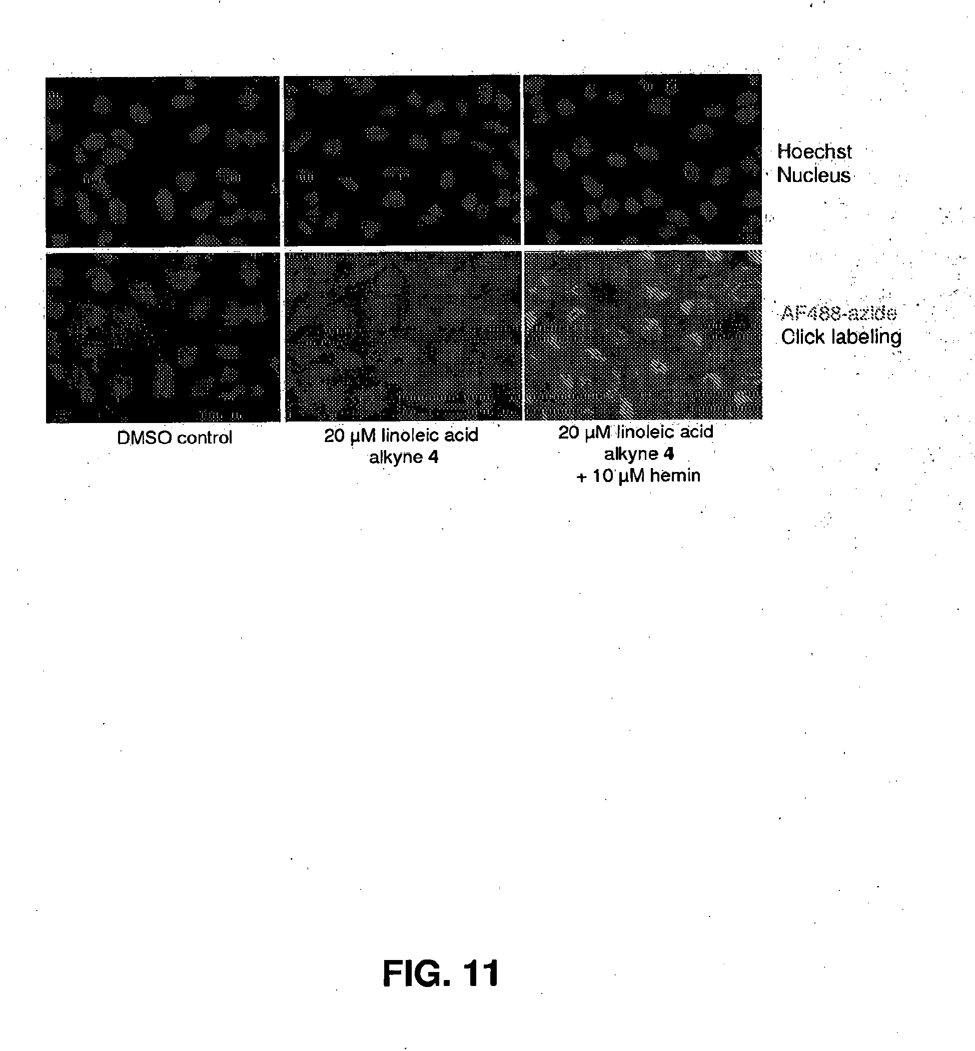

## Patentansprüche

1. Verbindung der Formel: 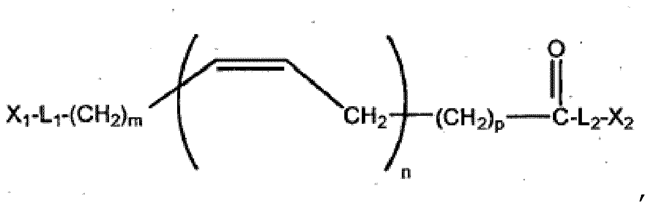 worin
m 1 bis 4 ist;
n 2 bis 3, oder 5 bis 6 ist;
p 1 bis 12 ist;
mindestens eines von X₁ oder X₂ ausgewählt ist aus der Gruppe, bestehend aus einer reaktiven Alkineinheit und einer reaktiven Azideinheit und das andere ausgewählt ist aus der Gruppe, bestehend aus H, Alkyl, Alkenyl, Cycloalkyl, Cycloalkenyl, Alkoxy, Aryl, Aralkyl, Heteroaryl und Heteroaralkyl; und
L₁ und L₂ unabhängig ausgewählt sind aus der Gruppe, bestehend aus 0, NH, einer Alkyl-Linkergruppe, umfassend 1 bis 10 Kohlenstoffatome, und einer Alkyl-Linkergruppe, umfassend 1 bis 10 Kohlenstoffatome, von denen ein beliebiges mit einem oder mehreren Heteroatomen substituiert sein kann, die unabhängig ausgewählt sind aus der Gruppe, bestehend 0, N und S; wobei die Verbindung nicht wie folgt ist:

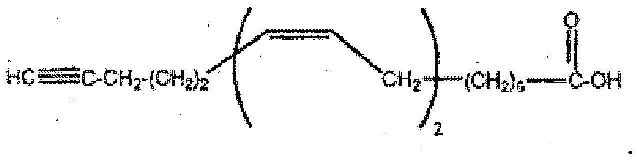

2. Verbindung nach Anspruch 1, wobei X₁ eine reaktive Alkineinheit ist; und X₂ ausgewählt ist aus der Gruppe, bestehend aus einem H, Alkyl, Alkenyl, Cycloalkyl, Cycloalkenyl, Alkoxy, Aryl, Aralkyl, Heteroaryl und Heteroaralkyl.

3. Verbindung nach Anspruch 1, wobei X₁ eine reaktive Azideinheit ist; und X₂ ausgewählt ist aus der Gruppe, bestehend aus einem H, Alkyl, Alkenyl, Cycloalkyl, Cycloalkenyl, Alkoxy, Aryl, Aralkyl, Heteroaryl und Heteroaralkyl.

4. Verbindung nach Anspruch 1, wobei X₂ eine reaktive Alkineinheit ist; und X₁ ausgewählt ist aus der Gruppe, bestehend aus einem H, Alkyl, Alkenyl, Cycloalkyl, Cycloalkenyl, Alkoxy, Aryl, Aralkyl, Heteroaryl und Heteroaralkyl.

5. Verbindung nach Anspruch 1, wobei X₂ eine reaktive Azideinheit ist; und X₁ ausgewählt ist aus der Gruppe, bestehend aus H, Alkyl, Alkenyl, Cycloalkyl, Cycloalkenyl, Alkoxy, Aryl, Aralkyl, Heteroaryl und Heteroaralkyl.

6. Verbindung nach Anspruch 1, wobei X₁ und X₂ unabhängig ausgewählt sind aus der Gruppe, bestehend aus einer reaktiven Alkineinheit und einer reaktiven Azideinheit.

7. Verbindung nach Anspruch 1, die ausgewählt ist aus der Gruppe, bestehend aus: 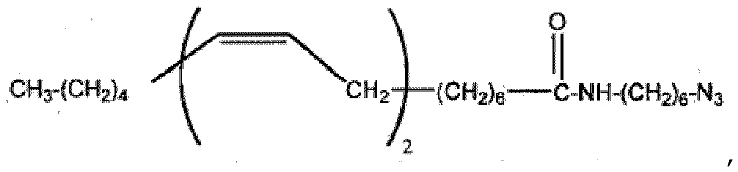 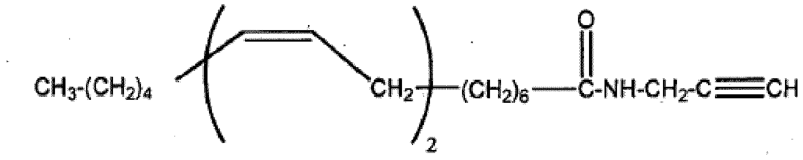 un d

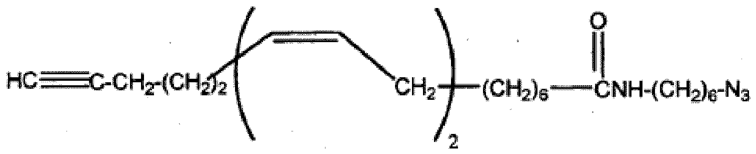

8. Verfahren zum Detektieren eines modifizierten Proteins, das als Reaktion auf oxidative zelluläre Bedingungen erzeugt wird, in einer Zelle, umfassend die folgenden Schritte:
(a) Inkontaktbringen einer Zelle in einer wässrigen Lösung mit einer Verbindung mit der folgenden Formel: 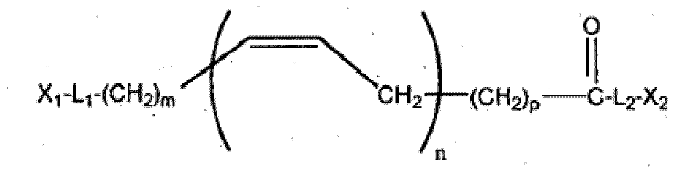 worin m 1 bis 4 ist;
n 2 bis 6 ist;
p 1 bis 12 ist;
mindestens eines von X₁ oder X₂ ausgewählt ist aus der Gruppe, bestehend aus einer reaktiven Alkineinheit und einer reaktiven Azideinheit und das andere ausgewählt ist aus der Gruppe, bestehend aus H, Alkyl, Alkenyl, Cycloalkyl, Cycloalkenyl, Alkoxy, Aryl, Aralkyl, Heteroaryl und Heteroaralkyl; und
L₁ und L₂ unabhängig ausgewählt sind aus der Gruppe, bestehend aus 0, NH, einer Alkyl-Linkergruppe, umfassend 1 bis 10 Kohlenstoffatome, und einer Alkyl-Linkergruppe, umfassend 1 bis 10 Kohlenstoffatome, von denen ein beliebiges mit einem oder mehreren Heteroatomen substituiert sein kann, die unabhängig ausgewählt sind aus der Gruppe, bestehend 0, N und S;
(b) Inkontaktbringen der Zelle in der wässrigen Lösung mit einem Reportermolekül, das einen chemischen Griff umfasst, der mit der reaktiven Alkineinheit oder reaktiven Azideinheit der Verbindung reagieren kann;
und
(c) Detektieren der Gegenwart des modifizierten Proteins in der Zelle.

9. Verfahren nach Anspruch 8, wobei die wässrige Lösung in Schritt (b) ferner Cu(I)-Ionen; Cu(I)-Ionen und einen Kupferchelatbildner; Cu(II)-Ionen und mindestens ein Reduktionsmittel; oder Cu(II)-Ionen, mindestens ein Reduktionsmittel und einen Kupferchelatbildner umfasst.

10. Verfahren zum Detektieren, in Lösung, eines modifizierten Proteins, das als Reaktion auf oxidative zelluläre Bedingungen erzeugt wird, umfassend die folgenden Schritte:
(a) Inkontaktbringen einer Zelle in einer wässrigen Lösung mit einer Verbindung mit der folgenden Formel: 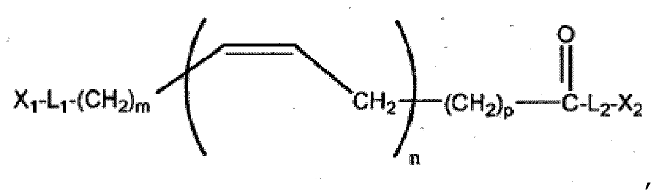 worin m 1 bis 4 ist;
n 2 bis 6 ist;
p 1 bis 12 ist;
mindestens eines von X₁ oder X₂ ausgewählt ist aus der Gruppe, bestehend aus einer reaktiven Alkineinheit und einer reaktiven Azideinheit und das andere ausgewählt ist aus der Gruppe, bestehend aus H, Alkyl, Alkenyl, Cycloalkyl, Cycloalkenyl, Alkoxy, Aryl, Aralkyl, Heteroaryl und Heteroaralkyl; und
L₁ und L₂ unabhängig ausgewählt sind aus der Gruppe, bestehend aus 0, NH, einer Alkyl-Linkergruppe, umfassend 1 bis 10 Kohlenstoffatome, und einer Alkyl-Linkergruppe, umfassend 1 bis 10 Kohlenstoffatome, von denen ein beliebiges mit einem oder mehreren Heteroatomen substituiert sein kann, die unabhängig ausgewählt sind aus der Gruppe, bestehend 0, N und S;
(b) Herstellen eines Isolats der Zelle;
(c) Inkontaktbringen des Isolats mit einem Reportermolekül, einem Trägermolekül oder einem festen Trägermedium, das einen chemischen Griff umfasst, der mit der reaktiven Alkineinheit oder reaktiven Azideinheit der Verbindung reagieren kann; und
(d) Detektieren der Gegenwart des modifizierten Proteins.

11. Verfahren nach Anspruch 10, wobei das Isolat in Schritt (c) ferner Cu(I)-Ionen; Cu(I)-Ionen und einen Kupferchelatbildner; Cu(II)-Ionen und mindestens ein Reduktionsmittel; oder Cu(II)-Ionen, mindestens ein Reduktionsmittel und einen Kupferchelatbildner umfasst.

12. Verfahren nach einem der Ansprüche 8 bis 11, wobei X₁ und X₂ wie in einem der Ansprüche 2 bis 6 definiert sind.

13. Verfahren nach einem der Ansprüche 8 bis 11, wobei die Verbindung wie in Anspruch 7 definiert ist.

14. Kit, umfassend eine Verbindung nach einem der Ansprüche 1 bis 7; und ferner umfassend mindestens einen von:
(a) einer Lösung, die Cu(I)-Ionen; Cu(I)-Ionen und einen Kupferchelatbildner; Cu(II)-Ionen; mindestens ein Reduktionsmittel; einen Kupferchelatbildner; mindestens ein Reduktionsmittel und einen Kupferchelatbildner; Cu(II)-Ionen und mindestens ein Reduktionsmittel; Cu(II)-Ionen und einen Kupferchelatbildner; oder Cu(II)-Ionen, mindestens ein Reduktionsmittel und einen Kupferchelatbildner umfasst; oder
(b) einem Reportermolekül, einem Trägermolekül oder einem festen Trägermedium, das einen chemischen Griff umfasst, der mit der reaktiven Alkineinheit oder reaktiven Azideinheit der Verbindung reagieren kann.

## Revendications

1. Composé de la formule : 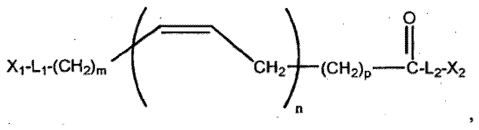 où
m est un nombre de 1 à 4 ;
n est un nombre de 2 à 3 ou de 5 à 6 ;
p est un nombre de 1 à 12 ;
l'un au moins parmi X₁ et X₂ est choisi dans le groupe constitué par le groupement réactif alcyne et le groupement réactif azoture, et l'autre est choisi dans le groupe constitué par H, un alkyle, un alcényle, un cycloalkyle, un cycloalcényle, un alcoxy, un aryle, un aralkyle, un hétéroaryle, et un hétéroaralkyle ; et
L₁ et L₂ sont choisis indépendamment dans le groupe constitué par 0, NH, un groupe de liaison alkyle comprenant 1 à 10 atomes de carbone, et un groupe de liaison alkyle comprenant de 1 à 10 atomes de carbone dont l'un quelconque peut être substitué avec un ou plusieurs hétéroatomes choisis indépendamment dans le groupe constitué par 0, N et S ; le composé n'étant pas :

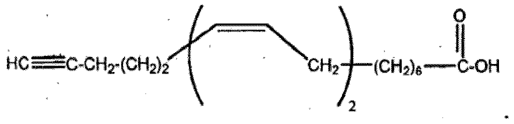

2. Composé selon la revendication 1, dans lequel X₁ est un groupement réactif alcyne ; et X₂ est choisi dans le groupe constitué par l'hydrogène, un alkyle, un alcényle, un cycloalkyle, un cycloalcényle, un alcoxy, un aryle, un aralkyle, un hétéroaryle, et un hétéroaralkyle.

3. Composé selon la revendication 1, dans lequel X₁ est un groupement réactif azoture ; et X₂ est choisi dans le groupe constitué par l'hydrogène, un alkyle, un alcényle, un cycloalkyle, un cycloalcényle, un alcoxy, un aryle, un aralkyle, un hétéroaryle, et un hétéroaralkyle.

4. Composé selon la revendication 1, dans lequel X₂ est un groupement réactif alcyne ; et X₁ est choisi dans le groupe constitué par H, un alkyle, un alcényle, un cycloalkyle, un cycloalcényle, un alcoxy, un aryle, un aralkyle, un hétéroaryle, et un hétéroaralkyle.

5. Composé selon la revendication 1, dans lequel X₂ est un groupe réactif azoture ; X₁ est choisi dans le groupe constitué par H, un alkyle, un alcényle, un cycloalkyle, un cycloalcényle, un alcoxy, un aryle, un aralkyle, un hétéroaryle, et un hétéroaralkyle.

6. Composé selon la revendication 1, dans lequel X₁ et X₂ sont choisis indépendamment dans le groupe constitué par un groupement réactif alcyne et un groupement réactif azoture.

7. Composé selon la revendication 1 choisi dans le groupe constitué par : 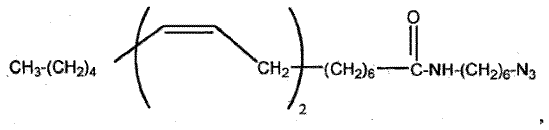 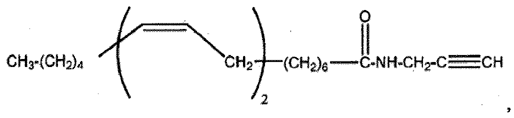 et

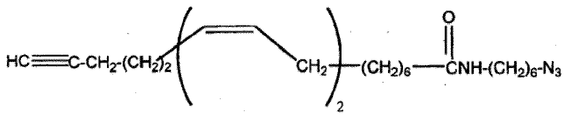

8. Procédé de détection, dans une cellule, d'une protéine modifiée générée en réponse à des conditions cellulaires oxydantes, comprenant les étapes consistant à :
(a) mettre en contact une cellule dans une solution aqueuse avec un composé ayant la formule : 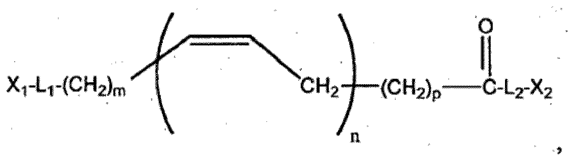 où
m est un nombre de 1 à 4 ;
n est un nombre de 2 à 3 ou de 5 à 6 ;
p est un nombre de 1 à 12 ;
l'un au moins parmi X₁ et X₂ est choisi dans le groupe constitué par le groupement réactif alcyne et le groupement réactif azoture, et l'autre est choisi dans le groupe constitué par H, un alkyle, un alcényle, un cycloalkyle, un cycloalcényle, un alcoxy, un aryle, un aralkyle, un hétéroaryle, et un hétéroaralkyle ; et
L₁ et L₂ sont choisis indépendamment dans le groupe constitué par 0, NH, un groupe de liaison alkyle comprenant 1 à 10 atomes de carbone, et un groupe de liaison alkyle comprenant de 1 à 10 atomes de carbone dont l'un quelconque peut être substitué avec un ou plusieurs hétéroatomes choisis indépendamment dans le groupe constitué par 0, N et S ;
(b) mettre en contact la cellule dans la solution aqueuse avec une molécule rapporteuse comprenant une poignée chimique apte à réagir avec le groupement réactif alcyne ou le groupe réactif azoture du composé ; et
(c) détecter la présence de la protéine modifiée dans la cellule.

9. Procédé selon la revendication 8, dans lequel la solution aqueuse à l'étape (b) comprend en outre des ions Cu(I) ; des ions Cu(I) et un chélateur du cuivre ; des ions Cu(II) et au moins un agent réducteur ; ou, des ions Cu(II), au moins un agent réducteur et un chélateur du cuivre.

10. Procédé de détection, dans une solution, d'une protéine modifiée générée en réponse à des conditions cellulaires oxydantes, comprenant les étapes consistant à :
(a) mettre en contact une cellule dans une solution aqueuse avec un composé ayant la formule : 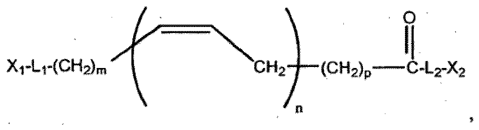 où
m est un nombre de 1 à 4 ;
n est un nombre de 2 à 3 ou de 5 à 6 ;
p est un nombre de 1 à 12 ;
l'un au moins parmi X₁ et X₂ est choisi dans le groupe constitué par le groupement réactif alcyne et le groupement réactif azoture, et l'autre est choisi dans le groupe constitué par H, un alkyle, un alcényle, un cycloalkyle, un cycloalcényle, un alcoxy, un aryle, un aralkyle, un hétéroaryle, et un hétéroaralkyle ; et
L₁ et L₂ sont choisis indépendamment dans le groupe constitué par 0, NH, un groupe de liaison alkyle comprenant 1 à 10 atomes de carbone, et un groupe de liaison alkyle comprenant de 1 à 10 atomes de carbone dont l'un quelconque peut être substitué avec un ou plusieurs hétéroatomes choisis indépendamment dans le groupe constitué par 0, N et S ;
(b) préparer un isolat de la cellule ;
(c) mettre l'isolat en contact avec une molécule rapporteuse, une molécule porteuse ou un support solide comprenant une poignée chimique apte à réagir avec le groupement réactif alcyne ou le groupement réactif azoture du composé ; et
(d) détecter la présence de la protéine modifiée.

11. Procédé selon la revendication 10, dans lequel l'isolat de l'étape (c) comprend en outre des ions Cu(I) ; des ions Cu(I) et un chélateur du cuivre ; des ions Cu(II) et au moins un agent réducteur ; ou, des ions Cu(II), au moins un agent réducteur et un chélateur du cuivre.

12. Procédé selon l'une quelconque des revendications 8 à 11, dans lequel X₁ et X₂ sont tels que définis dans l'une quelconque des revendications 2 à 6.

13. Procédé selon l'une quelconque des revendications 8 à 11, dans lequel le composé est tel que défini dans la revendication 7.

14. Kit comprenant un composé selon l'une quelconque des revendications 1 à 7 :
et comprenant en outre au moins un élément parmi :
(a) une solution comprenant des ions Cu(I) ; des ions Cu(I) et un chélateur du cuivre ; des ions Cu(II) ; au moins un agent réducteur ; un chélateur du cuivre ; des ions Cu(II) et au moins un agent réducteur ; des ions Cu(II) et un chélateur du cuivre ; ou, des ions Cu(II), au moins un agent réducteur et un chélateur du cuivre ; ou,
(b) une molécule rapporteuse, une molécule porteuse, ou un support solide comprenant une poignée chimique apte à réagir avec le groupement réactif alcyne ou le groupement réactif azoture du composé.
